# EUROPEAN PATENT APPLICATION

(11) **EP 4 729 068 A1**
(43) Date of publication of application: **22.04.2026**
(21) Application number: 24306709.7
(22) Date of filing: 16.10.2024
(51) Int. Cl.: A61K 39/12, A61P 35/00

(54) **LENTIVIRAL VECTOR-BASED IMMUNOTHERAPY OF PROSTATE TUMORS**

(71) Applicant: Theravectys, 75015 Paris (FR); Institut Pasteur, 75015 Paris (FR)
(72) Inventor: CHARNEAU, Pierre, 75724 PARIS (FR); MAJLESSI, Laleh, 75724 PARIS (FR); FERT, Ingrid, 75724 PARIS (FR); DOUGUET, Laëtitia, 75724 PARIS (FR); VESIN, Benjamin, 75724 PARIS (FR); MONCOQ, Fanny, 75724 PARIS (FR); AUTHIE, Pierre, 75724 PARIS (FR); LE CHEVALIER, Fabien, 75724 PARIS (FR)
(74) Representative: Ipsilon

(57) **Abstract**

The present invention relates to a lentiviral vector comprising a nucleic acid sequence encoding at least one antigen sequence selected from the group consisting of human prostatic acid phosphatase, human prostate specific antigen, human prostate-specific membrane antigen, human prostate stem cell antigen, human six-transmembrane epithelial antigen of the prostate-1 and fragments thereof.

It further relates to lentiviral vector particles, isolated cells or pharmaceutical compositions comprising a lentiviral vector according to the invention.

The invention further provides a combinatory treatment comprising at least one lentiviral vector, lentiviral vector particle, isolated cell or pharmaceutical composition according to the invention, and at least one anticancer agent or treatment and/or at least one immunotherapy agent or treatment.

Finally, the invention provides any of the above for use in the treatment and/or prevention of prostate cancers, in particular of metastatic prostate cancers, more particularly of metastatic castration-resistant prostate cancers.

## Description

### FIELD OF THE INVENTION

The present invention relates to the field of recombinant vaccine technology and relates to generation of lentiviral vectors, which can be used as therapeutic and prophylactic vaccines. In particular, the present invention relates to lentiviral vectors expressing human prostatic acid phosphatase (hPAP), human prostate specific antigen (hPSA), human prostate-specific membrane antigen (hPSMA), human prostate stem cell antigen (hPSCA), human six-transmembrane epithelial antigen of the prostate-1 (hSTEAP1), human trophoblast cell surface antigen 2 (hTROP2), and/or human TCR gamma alternate reading frame protein (hTARP) antigens, and fragments thereof, and to their implementation in the prevention and/or treatment of prostate cancers, in particular of metastatic prostate cancers, more particularly of metastatic castration-resistant prostate cancers.

### BACKGROUND OF THE INVENTION

Prostate cancer is the second most frequently diagnosed cancer in men after lung cancer. It accounts for 15 % of newly diagnosed cancer in high-income countries and causes the death of approximately 350,000 men annually (Bray et al., CA Cancer J Clin 68:394-424 (2018)). At the time of initial diagnosis, approximately 80 % of cancers are confined to the prostate organ, approximately 15 % exhibit locoregional metastases and 5 % present distant metastases (Siegel et al., CA Cancer J Clin 68:7-30 (2018)). Since treatment approaches can be curative, localized prostate cancers have favorable outcome and 5-year-survival usually reaches in 99 % of cases (Mazzone et al., Eur Urol Oncol 2:541-548 (2019)). However, the currently available surgery, chemo-/radiotherapies or hormone therapies can alter the life quality. When the prostate cancer has metastasized, usually to the lymph nodes, bone of the axial skeleton or visceral sites, treatment options become limited and often palliative. Prostate cancer can progress toward metastatic castration-sensitive prostate cancer (mCSPC), in which the androgen-deprivation standard of care limits the growth of prostate malignant cells which require high concentrations of androgens (Hahn et al., Am Soc Clin Oncol Educ Book 38:363-371 (2018)). The 5-year survival rate for mCSPC declines compared to that of localized prostate cancer and only reaches ≈ 30 % (Crawford et al., JU open plus, 2(4):e00029 (2024)). Progressively, tumor cells may develop resistance to androgen deprivation and the disease reaches its final stage, i.e. metastatic castration-resistant prostate cancer (mCRPC) (Rebello et al., Nat Rev Dis Primers 7:9 (2021)). At this stage, treatment options remain extremely limited and include the microtubule inhibitor (cabazitaxel), poly ADP-ribose polymerase (PARP) inhibitors, and PSMA radioligand therapy (von Amsberg et al., Int J Mol Sci 23(5):2569 (2022)).

The present invention aims at providing an effective immunotherapy for prostate cancers, with robust anti-tumor effects. In particular, the present invention aims at providing a lentiviral vector-based immunotherapy that induces highly specific immune responses and long-lasting immune memory against prostate cancer tumor cells.

Antagonistic antibodies targeting cytotoxic T-lymphocyte antigen 4 (CTLA-4), and/or programmed death-1 (PD1) or PD1 ligand (PDL1) have been effective in tumor types with high mutation burdens such as melanoma or lung cancers. These immune checkpoint blockers are available for selected mCRPC patients. However, these treatments provided limited benefits in prostate tumors, which are usually "cold" and poor in tumor infiltrating lymphocytes (TILs) because of their immunosuppressive microenvironment (Shiao et al., Cancer Lett 380:340-348 (2016)). The American Food and Drug Administration (FDA) has licensed a therapeutic T-cell vaccine, Sipuleucel-T (Provenge; Dendreon Corporation, Seattle, USA), which is based on leukapheresis of autologous activated dendritic cells (DC), and which provided more encouraging results than immune checkpoint inhibitors (Kantoff et al., N Engl J Med 363:411-422 (2010), Anassi et al., P T 36:197-202 (2011) and Thara et al., Maturitas 69:296-303 (2011)), motivating development of more efficient and better adapted T cell-based immunotherapies of prostate cancers (Sridaran et al., Cell Rep Med 4:101199 (2023)).

Non-integrative lentiviral vectors are outperforming inducers of high-quality T-cell responses, characterized by their long-lasting immune memory (Ku et al., Commun Biol 4:713 (2021), Ku et al., Expert Rev Vaccines 20:1571-1586 (2021) and Nemirov et al., Pharmaceutics 15(3):846 (2023)). The inventors and other expert laboratories have established proofs of concept of the efficacy of lentiviral vectors in numerous pre-clinical infectious and immuno-oncotherapy models (Ku et al., Expert Rev Vaccines 20:1571-1586 (2021), Nemirov et al., Pharmaceutics 15(3):846 (2023), Anna et al., Mucosal Immunol 15:1389-1404 (2022), Cousin et al., Cell Rep 26:1242-1257 (2019), Demidova et al., Expert Rev Vaccines 23(1):674-687 (2024), Douguet et al., EMBO Mol Med 15:e17723 (2023), Fert et al., NPJ Vaccines 9:102 (2024), Ku et al., EMBO Mol Med 13:e14459 (2021), Ku et al., Cell Host Microbe 29:236-249 (2021), Lopez et al., Cell Rep 40:111142 (2022), Nemirov et al., Front Immunol 14:1208041 (2023), Somaiah et al., Clin Cancer Res 25:5808-5817 (2019), Tada et al., JCI Insight 7(18):e161598 (2022) and Vesin et al., Mol Ther 30:2984-2997 (2022)). The safety, tolerability and immunogenicity of these vectors have been already established in two clinical trials (Somaiah et al., Clin Cancer Res 25:5808-5817 (2019) and 2011-006260-52, C.T.r.-S.f. (2011)). A preclinical lentiviral vector-based immuno-oncotherapy has recently been used with high efficacy against Human Papillomavirus (HPV)-induced cancers (Douguet et al., EMBO Mol Med 15:e17723 (2023) and Fert et al., NPJ Vaccines 9:102 (2024)).

A cross-sectional comparative review of the most relevant immuno-oncotherapy strategies, evaluated in the same HPV-induced murine cancer model, established that certain adjuvanted proteins and viral vectors showed the most robust anti-tumor effects, among which the lentiviral vectors were the only strategy resulting in full tumor eradication in 100% of animals with the longest-lasting immune memory which avoided tumor relapse (Demidova et al., Expert Rev Vaccines 23(1):674-687 (2024)). Based on these preclinical results, a lentiviral vector, called "Lenti-HPV-07" recently reached a Phase I/IIa clinical trial to treat patients with HPV-induced cervix or oropharyngeal cancers (NCT06319963).

Therefore, there remains a need in the art for efficient and better adapted T cell-based immunotherapies of prostate cancers, in particular of metastatic prostate cancers, more particularly of metastatic castration-resistant prostate cancers.

In particular, there remains a need for a lentiviral vector-based immunotherapy eliciting strong CD8⁺ T-cell immunogenicity.

There also remains a need for lentiviral vector-based immunotherapies that induce strong anti-tumor effects.

There also remains a need for lentiviral vector-based immunotherapies that can induce and maintain long-lasting T-cell immune memory and avoid tumor relapse in prostate cancers, in particular in metastatic prostate cancers, more particularly in metastatic castration-resistant prostate cancers.

There also remains a need for lentiviral vector-based immunotherapies that can induce a remodelling of tumor microenvironment including "cold-to-hot" inflammatory switch.

The present invention aims to meet at least one of the above-mentioned needs.

### SUMMARY OF THE INVENTION

The present invention accordingly relates to the following items:
Item 1: A lentiviral vector, in particular an integrative or a non-integrative lentiviral vector, comprising a nucleic acid sequence encoding at least one antigen sequence selected from the group consisting of human prostatic acid phosphatase (hPAP), human prostate specific antigen (hPSA), human prostate-specific membrane antigen (hPSMA), human prostate stem cell antigen (hPSCA), human six-transmembrane epithelial antigen of the prostate-1 (hSTEAP1), human trophoblast cell surface antigen 2 (hTROP2), human TCR gamma alternate reading frame protein (hTARP), and fragments thereof.
Item 2: The lentiviral vector according to item 1, comprising a nucleic acid sequence encoding at least two antigen sequences independently selected from the group consisting of human prostatic acid phosphatase (hPAP), human prostate specific antigen (hPSA), human prostate-specific membrane antigen (hPSMA), human prostate stem cell antigen (hPSCA), human six-transmembrane epithelial antigen of the prostate-1 (hSTEAP1), human trophoblast cell surface antigen 2 (hTROP2), human TCR gamma alternate reading frame protein (hTARP), and fragments thereof.
Item 3: The lentiviral vector according to item 1 or 2, wherein the at least one or at least two antigen sequence(s) comprise:
   (i) human prostatic acid phosphatase (hPAP) or at least a fragment thereof; and/or
   (ii) human prostate specific antigen (hPSA) or at least a fragment thereof; and/or
   (iii) human trophoblast cell surface antigen 2 (hTROP2) or at least a fragment thereof; and/or
   (iv) human TCR gamma alternate reading frame protein (hTARP) or at least a fragment thereof.
Item 4: The lentiviral vector according to any of the preceding items, wherein the at least one or at least two antigen sequence(s) comprise:
   - human prostatic acid phosphatase (hPAP) or at least a fragment thereof, and human prostate specific antigen (hPSA) or at least a fragment thereof; or
   - human trophoblast cell surface antigen 2 (hTROP2) or at least a fragment thereof.
Item 5: The lentiviral vector according to any one of the preceding items, wherein the nucleic acid sequence encodes at least:
   - an amino acid sequence having at least 60% sequence identity, in particular at least 80% sequence identity, more particularly at least 90% sequence identity, with the sequence set forth as SEQ ID NO: 1; and/or
   - an amino acid sequence having at least 60% sequence identity, in particular at least 80% sequence identity, more particularly at least 90% sequence identity, with the sequence set forth as SEQ ID NO: 2.
Item 6: The lentiviral vector according to any one of the preceding items, wherein the nucleic acid sequence encodes at least an amino acid sequence having at least 60% sequence identity, in particular at least 80% sequence identity, more particularly at least 90% sequence identity, with the sequence set forth as SEQ ID NO: 1 and an amino acid sequence having at least 60% sequence identity, in particular at least 80% sequence identity, more particularly at least 90% sequence identity, with the sequence set forth as SEQ ID NO: 2;
   in particular wherein the amino acid sequence having at least 60% sequence identity, in particular at least 80% sequence identity, more particularly at least 90% sequence identity, with the sequence set forth as SEQ ID NO: 1 is fused, directly or by the intermediate of a linker sequence, at the N-terminal end of the amino acid sequence having at least 60% sequence identity, in particular at least 80% sequence identity, more particularly at least 90% sequence identity, with the sequence set forth as SEQ ID NO: 2.
Item 7: The lentiviral vector according to any one of the preceding items, wherein the nucleic acid sequence encodes at least an amino acid sequence having 60% sequence identity, in particular at least 80% sequence identity, more particularly at least 90% sequence identity, with the sequence set forth as SEQ ID NO: 3.
Item 8: The lentiviral vector according to any one of items 1 to 4, wherein the human trophoblast cell surface antigen 2 (hTROP2) antigen sequence or at least a fragment thereof is selected from the group consisting of:
   (a) an amino acid sequence having at least 60% sequence identity, in particular at least 80% sequence identity, more particularly at least 90% sequence identity, with the sequence set forth as SEQ ID NO: 4; and/or
   (b) an amino acid sequence having at least 60% sequence identity, in particular at least 80% sequence identity, more particularly at least 90% sequence identity, with the sequence set forth as SEQ ID NO: 5; and/or
   (c) an amino acid sequence having at least 60% sequence identity, in particular at least 80% sequence identity, more particularly at least 90% sequence identity, with the sequence set forth as SEQ ID NO: 6; and/or
   (d) an amino acid sequence having at least 60% sequence identity, in particular at least 80% sequence identity, more particularly at least 90% sequence identity, with the sequence set forth as SEQ ID NO: 7; and/or
   (e) an amino acid sequence having at least 60% sequence identity, in particular at least 80% sequence identity, more particularly at least 90% sequence identity, with the sequence set forth as SEQ ID NO: 8.
Item 9: The lentiviral vector according to any one of items 1 to 3, wherein the human TCR gamma alternate reading frame protein (hTARP) antigen sequence or at least a fragment thereof is selected from the group consisting of:
   - an amino acid sequence having at least 60% sequence identity, in particular at least 80% sequence identity, more particularly at least 90% sequence identity, with the sequence set forth as SEQ ID NO: 9; and/or
   - an amino acid sequence having at least 60% sequence identity, in particular at least 80% sequence identity, more particularly at least 90% sequence identity, with the sequence set forth as SEQ ID NO: 10.
Item 10: The lentiviral vector according to any one of the preceding items, wherein the lentiviral vector comprises:
   (i) a MHC Class I promoter, and in particular a human β2-microglobulin promoter; and/or
   (ii) a cPPT/CTS sequence, in particular the cPPT/CTS sequence set forth as sequence SEQ ID NO: 34; and/or
   (iii) a 3' long terminal repeat (LTR) which is devoid of its U3 promoter sequence; and/or
   (iv) a mutant form of the woodchuck hepatitis B virus (WHV) post-transcriptional regulatory element (WPRE), and in particular having the sequence set forth as sequence SEQ ID NO: 35.
Item 11: A lentiviral vector particle comprising at least a lentiviral vector as defined in any one of items 1 to 10.
Item 12: The lentiviral vector particle according to item 11, further comprising:
   - a vesicular stomatitis virus glycoprotein (VSV-G), in particular a VSV-G Indiana serotype or a VSV-G New Jersey serotype; and/or
   - HIV-1 subtype D Gag and Pol proteins.
Item 13: A pharmaceutical composition, in particular a vaccine composition, comprising, in a physiologically acceptable medium, at least one lentiviral vector according to any one of items 1 to 10 or a lentiviral vector particle according to any one of items 11 to 12.
Item 14: A combinatory treatment comprising:
   (i) at least one lentiviral vector according to any one of items 1 to 10, or lentiviral vector particle according to any one of items 11 to 12, or pharmaceutical composition according to item 13; and
   (ii) at least one anticancer agent or treatment and/or at least one immunotherapy agent or treatment.
Item 15: The combinatory treatment according to item 14, wherein the at least one anticancer agent or treatment is selected from the group consisting of DNA methyltransferase inhibitors; histone deacetylase inhibitors; and adjuvants, in particular Toll-Like Receptor (TLR) agonists, poly I:C (polyinosinic:polycytidylic acid), the stabilized form of poly I:C (polyICLC), CpG oligodeoxynucleotide, and cGAMP (Cyclic GMP-AMP); and mixtures thereof; and/or
   wherein the at least one immunotherapy agent or treatment is selected from the group consisting of immune checkpoint inhibitors, in particular anti-PD-1, anti-PD-L1 (PD-1 Ligand), anti-CTLA-4 (Cytotoxic T-Lymphocyte-Associated protein 4), anti-TIM-3 (T-cell immunoglobulin and mucin-domain containing-3), anti-LAG3 (Lymphocyte-activation gene 3), anti-TIGIT (T cell immunoreceptor with Ig and ITIM domains), and anti-NKG2A (Natural killer group 2 member ) antibodies; and mixtures thereof.
Item 16: A lentiviral vector according to any one of items 1 to 10, or a lentiviral vector particle according to any one of items 11 to 12, or a pharmaceutical composition according to item 13, or a combinatory treatment according to any one of items 14 to 15, for use in the treatment and/or prevention of prostate cancers.

The details, examples and preferences provided in relation to any particular one or more of the stated aspects of the present invention will be further described herein and apply equally to all aspects of the present invention. Any combination of the embodiments, examples and preferences described herein in all possible variations thereof is encompassed by the present invention unless otherwise indicated herein, or otherwise clearly contradicted by context.

### BRIEF DESCRIPTION OF THE FIGURES

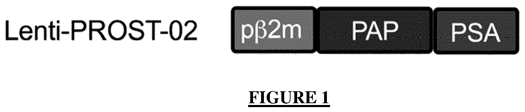

**Figure 1** shows schematic representation of the PROST-02 poly-epitopic hPAP-hPSA fusion protein.
   Figure 1 depicts a schematic representation of the PROST-02 antigen composed of clusters of T-cell epitopes derived from hPAP (PAP) and hPSA (PSA) under the human β2-microglobulin promoter (pβ2m), as encoded by Lenti-PROST-02 non-integrative vector. From left to right, the schematic representation shows: pβ2m, PAP and PSA.

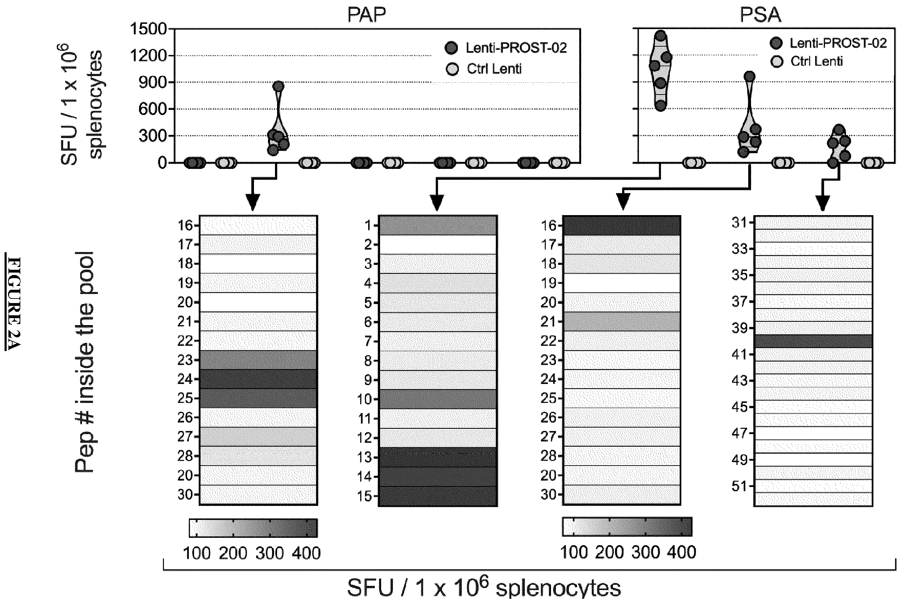

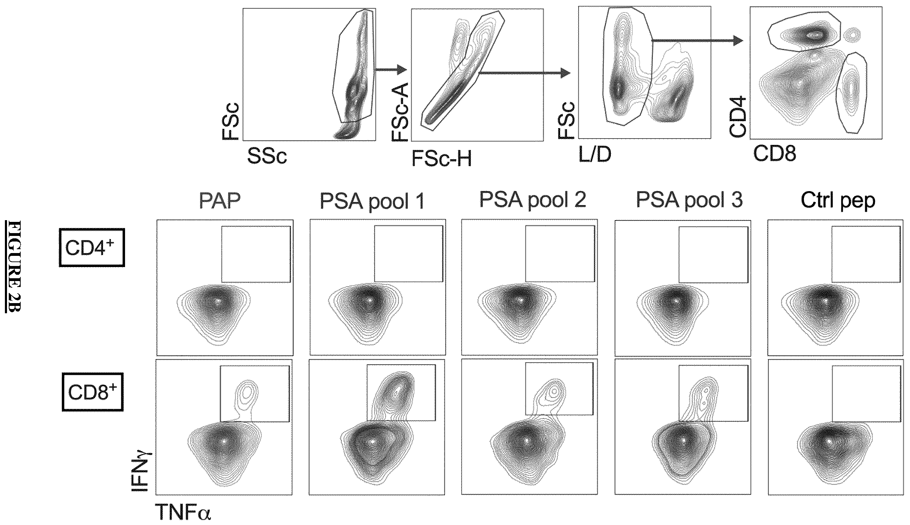

**Figure 2** shows the T-cell immunogenicity of lenti-PROST-02.
   Figure 2A, in the top two graphs, displays T-cell responses of C57BL/6 (H-2^{b}) mice, injected intramuscularly with 1 × 10⁹ Transduction Units (TU) of Lenti-PROST-02 (black points) or Ctrl Lenti (light grey points) (*n* = 5 mice per group). Specifically, the left graph shows the frequencies (in SFU 1 × 10⁶ splenocytes) of T splenocytes specific to hPAP (PAP) Lenti-PROST-02 antigen segments in individual mice, after *in vitro* stimulation with 5 different peptide pools, while the right graph shows the frequencies of T splenocytes specific to hPSA (PSA) antigen segments, after stimulation with 3 different peptide pools. Frequencies were determined by IFN-γ ELISPOT on day 14 post-injection.
   The bottom heatmaps depict deconvolution of positive peptide pools by IFN-γ ELISPOT, applied to splenocytes pooled from 5 Lenti-PROST-02-immunized mice. The heatmap on the left represents hPAP specific peptide pool (1 group), and the three on the right represent hPSA (3 groups) specific peptide pools. Heatmaps show SFU per 1 × 10⁶ splenocytes, with immunogenic 15-mer sequences inside the corresponding cells.
Figure 2B shows the Intracellular cytokine staining (ICS) and cytometric analyses gating strategy (top line) for T-splenocyte cytokine response analysis. The two representative blots (middle and bottom lines) show T-splenocyte cytokine responses of Lenti-PROST-02-immunized mice studied by ICS and cytometric analyses, after stimulation with, from left to right: PAP, PSA 1, PSA 2 and PSA 3 positive peptide pools and an irrelevant control peptide (Ctrl pep). One representative mouse of three/group is showed per peptide pool (middle line = CD4⁺ T cells, bottom line = CD8⁺ T cells).

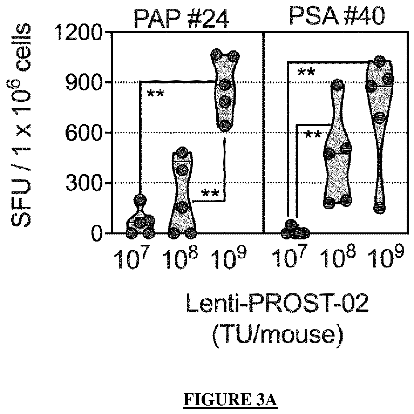

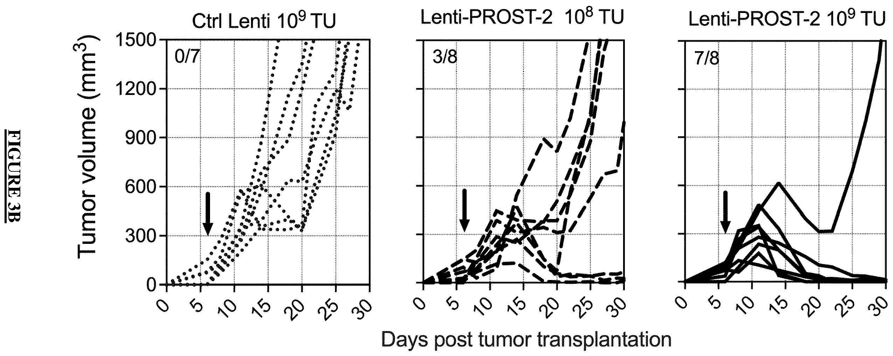

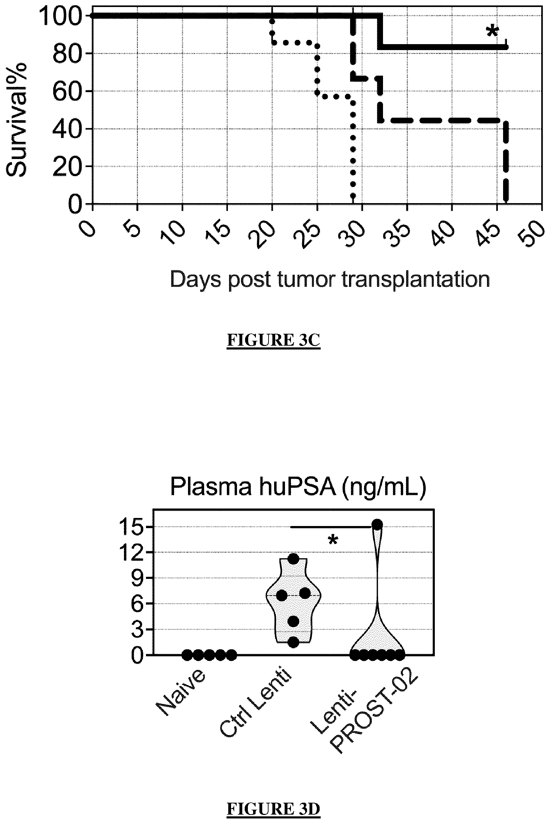

**Figure 3** shows Lenti-PROST-02-based immunotherapy of 3E9 tumors expressing human PSA.
   Figure 3A shows dose dependent T-cell splenocyte response (in SFU 1 × 10⁶ splenocytes) of individual C57BL/6 mice (n = 5 mice per group) immunized with, from left to right in each panel, 1 × 10⁷, 1 × 10⁸ or 1 × 10⁹ TU of Lenti-PROST-02 after *in vitro* stimulation with the peptides "PAP#24" (left panel) or "PSA#40" (right panel). Statistical significance for was determined by two-tailed Mann-Whitney test (**p < 0.01).
   Figure 3B depicts the variation of tumor volume (in mm³) in individual mice (*n* = 7 or 8 mice per group) over time (in days post tumor transplantation) in the Ctrl Lenti group (left graph), the Lenti-PROST-02 group immunized with 1 × 10⁸ TU of Lenti-PROST-02 (middle graph) and the Lenti-PROST-02 group immunized with 1 × 10⁹ TU of Lenti-PROST-02 (right graph). Each curve corresponds to an individual mouse from the corresponding group. Figure 3C shows the survival curve of the animals (% survival) over time (in days post tumor transplantation) for the following groups: Ctrl Lenti group (dots curve), Lenti-PROST-02 10⁸ TU group (dashes curve) and Lenti-PROST-02 10⁹ TU group (solid curve). Statistical significance was determined by Log-rank Mantel-Cox tests (**p* < 0.05). Mice were sacrificed when the tumors reached 1500 mm³, defined as humane endpoints.
   Figure 3D shows the concentration of hPSA (huPSA) in the plasma (in ng/mL) of, from left to right: naive control mice, mice that received the Ctrl Lenti, or mice immunized with 1 × 10⁹ TU of Lenti-PROST-02. The plasma concentration of hPSA (*n* = 5 to 7 mice per group) was determined on day 21 post tumor engraftment, by ELISA (Human kallikrein 3/PSA DuoSet ELISA kit (Cat # DY1344, RD systems, biotechne)). 2 out of the 7 Ctrl Lenti-treated mice were sacrificed days before blood sampling due to tumor size exceeding 1500 mm³.

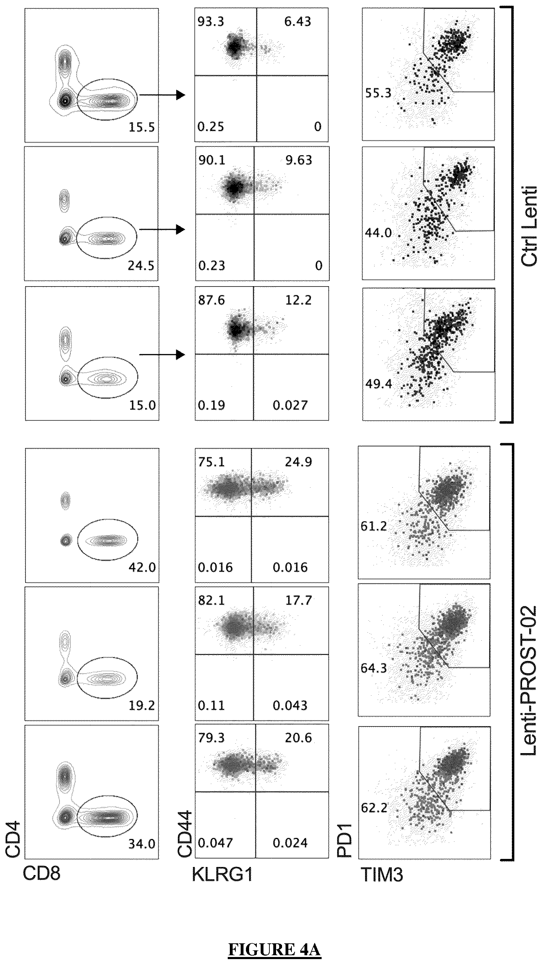

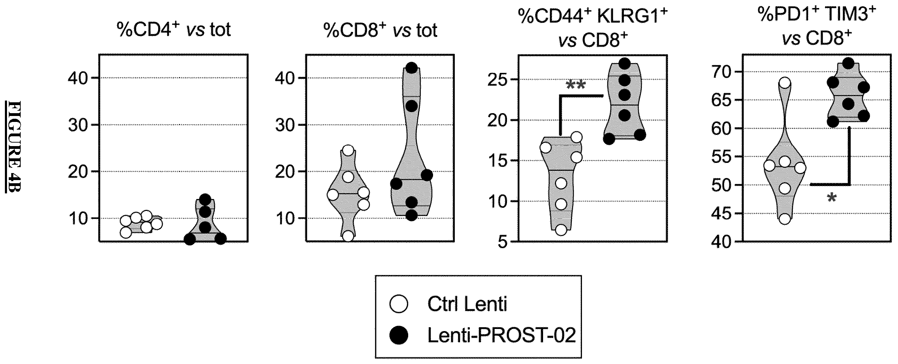

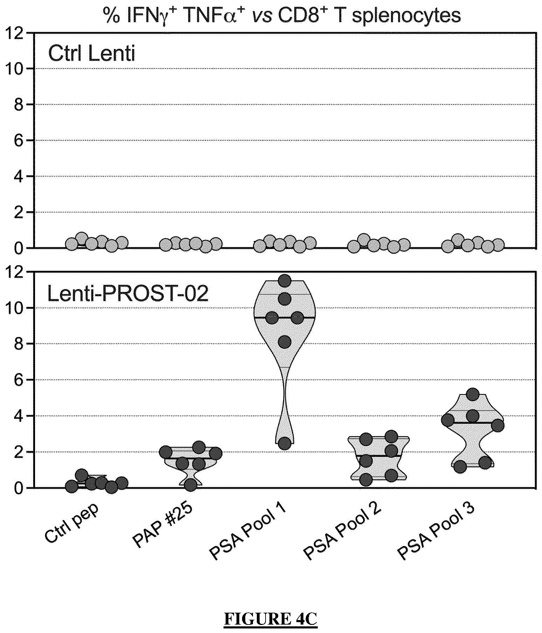

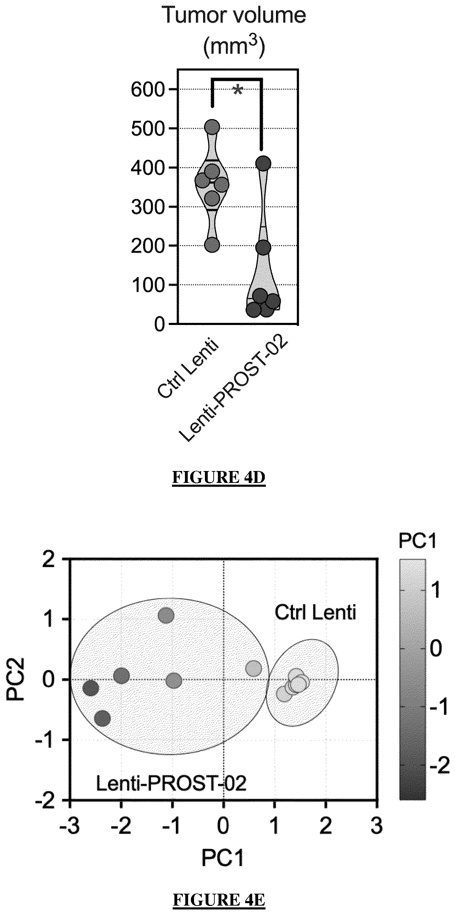

**Figure 4** shows TILs in Lenti-PROST-02-treated mice.
   C57BL/6 mice were engrafted subcutaneously with 8 × 10⁵ 3E9 cells on day 0 and treated with 1 × 10⁹ TU of Lenti Ctrl or Lenti-PROST-02 on day 6 (*n* = 6 mice per group). TILs were studied by cytometry on day 15, i.e. 9 days post-vaccination.
   Figure 4A shows representative cytometric dot blots, from for 3 mice per group, of TILs and their differentiation status in control (Ctrl Lenti, right panels) or vaccinated (Lenti-PROST-02, left panels) mice for the following markers: CD4 + CD8 (top line), CD44 + KLRG1 (middle line) and PD1 + TIM3 (bottom line).
   Figure 4B shows recapitulative percentages of various TIL subsets (from left to right: %CD4⁺ cells in the total alive cells, %CD8⁺ cells in the total alive cells, %CD44⁺ KLRG1⁺ cells within the CD8⁺ compartment, and %PD1⁺ TIM3⁺ cells within the CD8⁺ compartment) studied comparatively in mice treated with Ctrl Lenti (white circles) and Lenti-PROST-02 (black circles). Each circle represents an individual mouse (*n* = 5 or 6 mice per group).
   Figure 4C shows hPAP- and hPSA-specific IFNγ⁺ TNFα⁺ T splenocyte responses within the CD8⁺ compartment of T splenocytes (from left to right: the Ctrl pep irrelevant control peptide, and PAP, PSA 1, PSA 2 and PSA 3 positive peptide pools) in the Ctrl Lenti group (top graph) or the Lenti-PROST-02-treated mice (bottom graph) (*n* = 6 mice per group). Each circle represents an individual mouse.
   Figure 4D depicts the tumor volume (in mm³) in Ctrl Lenti (light grey circles) or Lenti-PROST-02 (dark grey circles) treated mice 9 days post-vaccination (*n* = 6 mice per group). Each circle represents an individual mouse.
   Figure 4E shows a PCA projection of the parameters studied in Figures 4B, 4C and 4D, which splits the Lenti-PROST-02 and Lenti Ctrl groups in two distinct groups. Proportion of variance for PC1 = 87.62%.

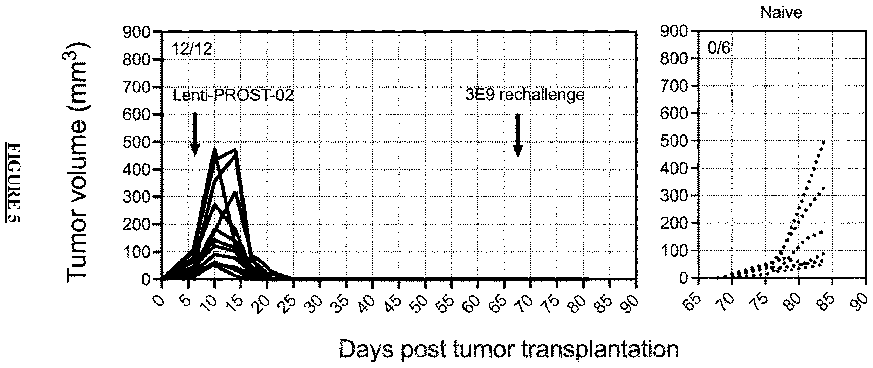

**Figure 5** shows that Lenti-PROST-02 therapy induces a long-term anti-tumor immune memory which prevents tumor relapse.
   Figure 5 shows the tumor volume (in mm³) over time (in days post 3E9 tumor transplantation) in individual mice. The left graph depicts the tumor volume in mice treated on day 6 post-tumor transplantation with 1 × 10⁹ TU of Lenti-PROST-02 and rechallenged on day 68 post tumor transplantation (*n* = 12 mice) (left graph). The right graph shows the tumor volume in mice from the naive group (*n* = 6 mice), transplanted with the same tumor cell suspension used on day 68 post-tumor transplantation.
**Figure 6** shows the inflammatory status of the 3E9 tumors in Ctrl Lenti- or Lenti-PROST-02-treated mice.

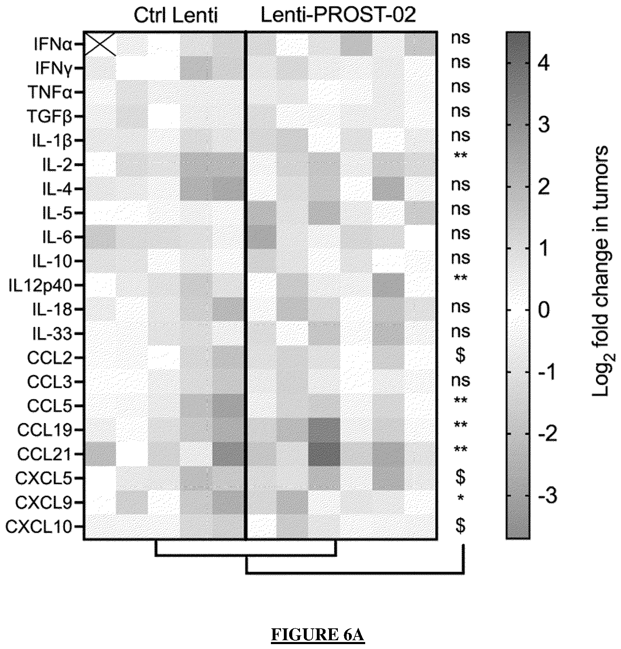

   Figure 6A represents the Log₂ fold change in various cytokines mRNA expression in the tumors of mice (*n* = 5 or 6 mice per group) engrafted with 3E9 tumor cells on day 0, treated with Ctrl Lenti (left side of the heatmap) or Lenti-PROST-02 (right side of the heatmap) on day 6, and analyzed by qRT-PCR on day 16. Each column of the heatmap represents an individual mouse (*n* = 5 or 6 mice per group). Each line of the heatmap represents the mRNA expression of a specific cytokine or chemokine. For each sample, the mRNA abundance (*C*_{T} value) of the target genes was normalized to that of the succinate dehydrogenase complex flavoprotein subunit A (SDHA) reference gene and compared to a calibrator value corresponding to the mean of the *C*_{T} values determined in the tumors from the Ctrl Lenti individuals. The fold change in gene expression was then calculated using 2^{-ΔΔ*C*}_{T}. Statistical significance was evaluated using Mann-Whitney tests (ns: not significant, ^{$}*p*<0.1, **p* < 0.05, ***p* < 0.01). One outlier sample was discarded from the analyses in the group of Ctrl Lenti for IFNα analyte (black cross).
   Figure 6B shows the Pearson correlation coefficient of differentially expressed cytokines or chemokines in the tumors of Lenti-PROST-02-treated mice.
**Figure 7** shows the immunogenicity of Lenti-TROP2-1 and Lenti-TROP2-2.

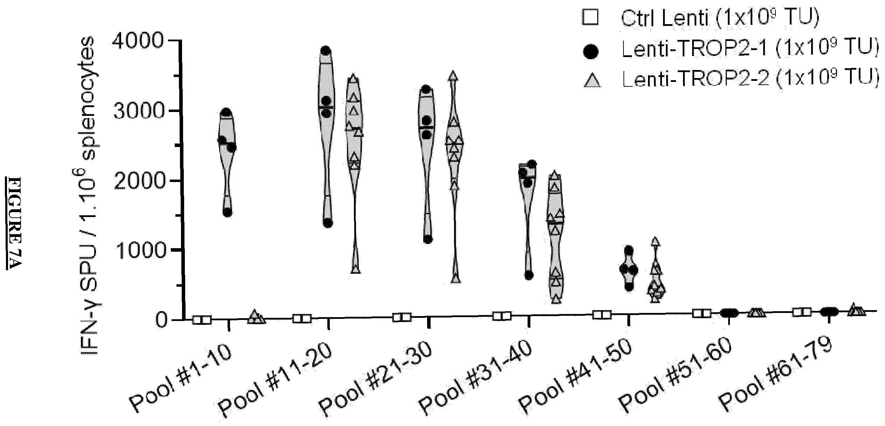

   Figure 7A depicts T-cell splenocyte response (in IFN-γ SPU per 1 × 10⁶ splenocytes) of C57BL/6 mice (*n* = 4 to 8 per group), injected intramuscularly with 1 × 10⁹ TU of Lenti-TROP2-1 (black circles), Lenti TROP2-2 (grey triangles) or Ctrl Lenti (white squares). Each circle represents an individual mouse. Specifically, at day 14 post injection, splenocytes from individual mice were studied by IFN-γ ELISPOT after *in vitro* stimulation with 7 distinct pools of overlapping 15-mer peptides spanning the full-length sequence of human TROP2 (from left to right: Pool #1-10, Pool #11-20, Pool #21-30, Pool #31-40, Pool #41-50, Pool #51-60 and Pool #61-79). Each pool contains ten overlapping 15-mers, except for the last pool (Pool #61-79) which contains fifteen overlapping 15-mers.
Figure 7B represents T-cell splenocyte response (in IFN-γ spot forming unit (SPU) per 1 × 10⁶ splenocytes) from mice immunized with 1 × 10⁹ TU of Lenti-TROP2-1 (black bars), Lenti-TROP2-2 (light grey bars), or Ctrl Lenti (white bars), specific to individual 15-mer peptides numbered 1 to 50 (from left to right) as detailed in Figure 7A.
**Figure 8** shows the effective dose range of Lenti-TROP2-2 in mice.

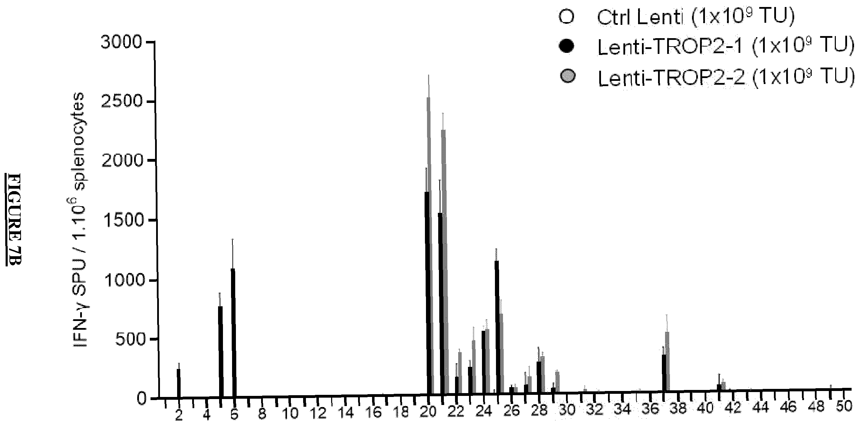

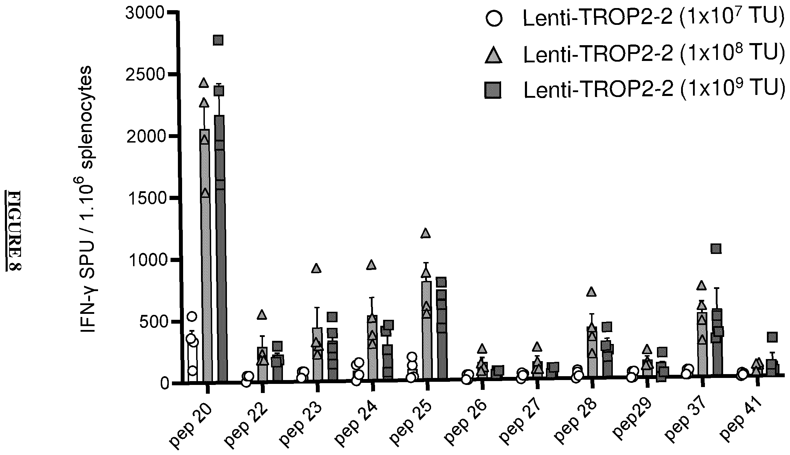

   Figure 8 represents T-cell splenocyte response (in IFN-γ SPU per 1 × 10⁶ splenocytes) specific to individual positive 15-mer peptides of human TROP2 (from left to right: peptides number 20, 22, 23, 24, 25, 26, 27, 28, 29, 37 and 41 as described in Figure 7B), from mice immunized intramuscularly with 1 × 10⁷ (white circles), 1 × 10⁸ (light grey triangles) or 1 × 10⁹ (grey squares) TU of Lenti-TROP2-2. The frequency of IFNγ-producing T cells was studied in ELISPOT at day 14 post injection.

### DETAILED DESCRIPTION OF THE INVENTION

The inventors demonstrated that T-cell immunotherapy implementing lentiviral vectors according to the invention results in the induction of an adaptive immune response, and more particularly highly specific T-cell responses.

The inventors also demonstrated that T-cell immunotherapy implementing lentiviral vectors according to the invention results in robust anti-tumor effect in a virulent tumor model of non-viral prostate cancer. In particular, the Lenti-PROST-02 immunotherapy resulted in a complete prostate tumor regression in the great majority of treated mice. The anti-tumor effect was correlated with a strong and highly specific CD8⁺ T-cell immunogenicity.

It was further demonstrated that this lentiviral vector-based immunotherapy prevented tumor relapse in the cured mice after a late tumor re-challenge, which mimics the prostate tumor relapse. These experiments therefore established the induction and maintenance of a long-term anti-tumor T-cell immune memory.

The Lenti-PROST-02 anti-tumor effect was concomitant with strong CD8⁺ T splenocyte responses to multiple immunogenic regions of human PAP and PSA encoded by the vector. The Lenti-TROP2-1 and Lenti-TROP2-2 vectors according to the invention also elicited strong CD8⁺ T splenocyte responses to multiple immunogenic regions of the hTROP2 antigen sequence encoded by the vector.

Lentiviral vectors according to the invention, as well as lentiviral vector particles comprising them, isolated cells comprising said lentiviral vectors or lentiviral vector particles, pharmaceutical compositions comprising them, and combinatory treatments comprising them are described throughout the present specification.

### Definitions

All scientific and technical terms used in this application have meanings commonly used in the art unless otherwise specified.

As used herein, "transgene" means a polynucleotide sequence that can be expressed, via recombinant techniques, in a non-native environment or heterologous cell under appropriate conditions.

As used herein, the term "recombinant", when used in reference to a cell of the invention, indicates that the cell has been modified by the introduction of an endogenous and/or heterologous nucleic acid or protein into the cell, or the alteration of a native cell, or that the cell is derived from a cell so modified. Thus, for example, recombinant cells express genes or nucleic acid that are not found within the native (non-recombinant) form of the cell, or express native (e.g. endogenous) genes at a different level than their native level, or express additional or supplementary copies of native genes (e.g. endogenous) at a different level than their native level. An isolated cell according to the invention is recombinant in that it comprises at least one lentiviral vector according to the invention and/or at least one lentiviral vector particle according to the invention.

As used herein, the term "recombinant", when used in reference to a vector, refers to sequences formed/obtained by techniques of genetic engineering well known to the man skilled in the art.

As used herein, the term "polypeptide" refers to a molecule comprising amino acid residues linked by peptide bonds and containing more than five amino acid residues. The amino acids are identified by either the single-letter or three-letter designations. The term "protein" as used herein is synonymous with the term "polypeptide" and may also refer to two or more polypeptides. Thus, the terms "protein", "peptide" and "polypeptide" can be used interchangeably. Polypeptides may optionally be modified (e.g. glycosylated, phosphorylated, acylated, famesylated, prenylated, sulfonated, and the like) to add functionality. Polypeptides exhibiting activity may be referred to as enzymes. It will be understood that, as a result of the degeneracy of the genetic code, a multitude of nucleotide sequences encoding a given polypeptide may be produced.

The terms "encoding" or "coding for" refer to the process by which a polynucleotide sequence, through the mechanisms of transcription and translation, produces an amino-acid sequence.

For each or the amino acid sequences of interest, reference sequences are described herein. The present description also encompasses amino acid sequences having specific percentages of amino acid identity with a reference amino acid sequence.

For obvious reasons, in all the present description, a specific nucleic acid sequence or a specific amino acid sequence which complies with, respectively, the considered nucleotide or amino acid identity, should further lead to obtaining a protein (or antigen) which displays the desired biological activity. As used herein, the "percentage of identity" between two nucleic acid sequences or between two amino acid sequences is determined by comparing both optimally aligned sequences through a comparison window.

The portion of the nucleotide or amino-acid sequence in the comparison window may thus include additions or deletions (for example "gaps") as compared to the reference sequence (which does not include these additions or these deletions) so as to obtain an optimal alignment between both sequences.

The terms "sequence homology" or "sequence identity" or "homology" or "identity" are used interchangeably herein. For the purpose of the invention, it is defined here that in order to determine the percentage of sequence homology or sequence identity of two amino acid sequences or of two nucleic acid sequences, the sequences are aligned for optimal comparison purposes. In order to optimize the alignment between the two sequences, gaps may be introduced in any of the two sequences that are compared. Such alignment can be carried out over the full length of the sequences being compared. Alternatively, the alignment may be carried out over a shorter length, for example over about 20, about 50, about 100 or more nucleic acids/based or amino acids. The sequence identity is the percentage of identical matches between the two sequences over the reported aligned region.

A comparison of sequences and determination of percentage of sequence identity between two sequences can be accomplished using a mathematical algorithm. The skilled person will be aware of the fact that several different computer programs are available to align two sequences and determine the identity between two sequences (Kruskal, J. B. (1983) An overview of sequence comparison In D. Sankoff and J. B. Kruskal, (ed.), Time warps, string edits and macromolecules: the theory and practice of sequence comparison, pp. 1-44 Addison Wesley).

The percent sequence identity between two amino acid sequences or between two nucleotide sequences may be determined using the Needleman and Wunsch algorithm for the alignment of two sequences. (Needleman, S. B. and Wunsch, C. D. (1970) J. Mol. Biol. 48, 443-453). Both amino acid sequences and nucleotide sequences can be aligned by the algorithm. The Needleman-Wunsch algorithm has been implemented in the computer program NEEDLE.

For the purpose of the invention, the NEEDLE program from the EMBOSS package was used (version 2.8.0 or higher, EMBOSS: The European Molecular Biology Open Software Suite (2000) Rice, P. LongdenJ. and Bleasby,A. Trends in Genetics 16, (6) pp276- 277, http://emboss.bioinformatics.nl/). For protein sequences EBLOSUM62 is used for the substitution matrix. For nucleotide sequence, EDNAFULL is used. The optional parameters used are a gap opening penalty of 10 and a gap extension penalty of 0.5. No end gap penalty is added. In the Output section, Yes has been indicated in response to the question "Brief identity and similarity" and "SRS pairwise" indicated as Output alignment format.

After alignment by the program NEEDLE as described above the percentage of sequence identity between a query sequence and a sequence of the invention is calculated as follows: Number of corresponding positions in the alignment showing an identical amino acid or identical nucleotide in both sequences divided by the total length of the alignment after subtraction of the total number of gaps in the alignment. The identity defined as herein can be obtained from NEEDLE by using the NOBRIEF option and is labeled in the output of the program as "longest-identity".

The similarity of nucleotide and amino acid sequences, i.e. the percentage of sequence identity, can be determined via sequence alignments using several other art-known algorithms, preferably with the mathematical algorithm of Karlin and Altschul (Karlin & Altschul (1993) Proc. Natl. Acad. Sci. USA 90: 5873-5877), with hmmalign (HMMER package, http://hmmer.wustl.edu/) or with the CLUSTAL algorithm (Thompson, J. D., Higgins, D. G. & Gibson, T. J. (1994) Nucleic Acids Res. 22, 4673-80) available for example on https://www.ebi.ac.uk/Tools/msa/clustalo/ or the GAP program (mathematical algorithm of the University of Iowa) or the mathematical algorithm of Myers and Miller (1989 - Cabios 4: 11-17) or Clone Manager 9. Preferred parameters used are the default parameters as they are set on https://www.ebi.ac.uk/Tools/msa/clustalo/.

The grade of sequence identity (sequence matching) may be calculated using for example BLAST, BLAT or BlastZ (or BlastX). A similar algorithm is incorporated into the BLASTN and BLASTP programs of Altschul et al., J. Mol. Biol. 215:403-410 (1990). BLAST polynucleotide searches are performed with the BLASTN program, score = 100, word length = 12, to obtain polynucleotide sequences that are homologous to those nucleic acids which encode the relevant protein.

BLAST protein searches are performed with the BLASTP program, score = 50, word length = 3, to obtain amino acid sequences homologous to the SHC polypeptide. To obtain gapped alignments for comparative purposes, Gapped BLAST is utilized as described in Altschul et al., Nucleic Acids Res. 25:3389-3402 (1997). When utilizing BLAST and Gapped BLAST programs, the default parameters of the respective programs are used. Sequence matching analysis may be supplemented by established homology mapping techniques like Shuffle-LAGAN (Brudno M., Bioinformatics 2003b, 19 Suppl 1: 154-162) or Markov random fields. When percentages of sequence identity are referred to in the present application, these percentages are calculated in relation to the full length of the longer sequence, if not specifically indicated otherwise.

In particular embodiments, % identity between two sequences is determined using CLUSTAL O (version 1.2.4).

The terms non-oncogenic used herein are used in their traditional meaning, i.e. it relates to an element, in the present case to antigens, unable to cause the formation of tumors. As detailed elsewhere, antigens implemented in the present invention have been genetically amended in order to become non-oncogenic. It means, according to the usual meaning of these terms, that the nucleic acid sequences encoding the antigens implemented herein are not found in nature and are modified either by introduction or by deletion or by modification of their nucleic acid sequences, leading to encoded amino acid sequences that also do not naturally exist in nature.

The man skilled in the art has known for a long time a variety of means to perform a deletion, substitution or introduction in a nucleic acid sequence.

As will be understood by those of skill in the art, it can moreover be advantageous to modify a coding sequence to enhance its expression in a particular host. The genetic code is redundant with 64 possible codons, but most organisms typically use a subset of these codons. The codons that are utilized most often in a species are called optimal codons, and those not utilized very often are classified as rare or low-usage codons. Codons can be substituted to reflect the preferred codon usage of the host, in a process sometimes called "codon optimization" or "controlling for species codon bias." Codon optimization for other host cells can be readily determined using codon usage tables or can be performed using commercially available software, such as CodonOp (www.idtdna.com/CodonOptfrom) from Integrated DNA Technologies. Optimized coding sequences containing codons preferred by a particular prokaryotic or eukaryotic host (Murray et al., Nucl Acids Res. 17:477-508 (1989)) can be prepared, for example, to increase the rate of translation or to produce recombinant RNA transcripts having desirable properties, such as a longer half-life, as compared with transcripts produced from a non-optimized sequence. Translation stop codons can also be modified to reflect host preference. For example, typical stop codon for monocotyledonous plants is UGA, whereas insects and E. coli commonly use UAA as the stop codon (Dalphin et al., Nucl Acids Res. 24:216-8 (1996)).

A "non-integrative" lentiviral vector means that, when this lentiviral vector is in a cell, it does not integrate into the host cell genome. A non-integrative lentiviral vector particle relates to a lentiviral vector particle that comprises a non-integrative lentiviral vector. It can also be termed integration-defective lentiviral vectors or non-integrative lentiviral vectors.

An "integrative " lentiviral vector refers to a lentiviral vector that, upon entering a host cell, integrates its genetic material into the host cell's genome. This integration allows for stable and long-term expression of the introduced genetic material in the host cell and its progeny. An integrative lentiviral vector particle pertains to a lentiviral vector particle that contains an integrative lentiviral vector. It can also be referred to as an integration-competent lentiviral vector or an integrative lentiviral vector.

As used herein, "treating" or "treatment" refers to causing a detectable improvement in one or more symptoms associated with prostate cancers, in particular of metastatic prostate cancers, more particularly of metastatic castration-resistant prostate cancers or causing a biological effect (e.g. a decrease in the level of a particular biomarker) that is correlated with the underlying pathologic mechanism(s) giving rise to the condition or symptom(s).

"Prevention" or "preventing" means any treatment of a disease or condition that causes the clinical symptoms of the disease or condition not to develop. Compounds may, in some embodiments, be administered to a subject (including a human) or an individual in need thereof who is at risk or has a family history of the disease or condition, in particular of the prostate cancer as described elsewhere.

"Subject" or "individual in need thereof' refers to an animal, such as a mammal (including a human), that has been or will be the object of treatment, observation, or experiment. The methods described herein may be useful in human therapy and/or veterinary applications. In some embodiments, the subject or individual is a mammal. In one embodiment, the subject or individual is a human.

As used herein, an "antigen" refers to a substance capable of being recognized by the immune system and inducing an immune response. An antigen may be a molecule, such as a protein, peptide, polysaccharide, or other macromolecule, including but not limited to those derived from pathogenic organisms, or from non-pathogenic sources (e.g. toxins, allergens, transplanted cells, etc.). The immune response to an antigen typically involves the generation of specific antibodies or the activation of immune cells that recognize and target the antigen or the structure on which it is present. Antigens may include naturally occurring, synthetic, or recombinant molecules.

As used herein, a "fragment" generally refers to a fragment of a given sequence, in particular of an amino acid sequence, that has the same biological activity as the original peptide from which it originates that has a given number of consecutive amino acids from the original peptide. In a particular embodiment, the fragment is a biologically active fragment. A biologically active fragment may be an immunologically active fragment, which refers to a fragment that has the same immunological activity as the peptide from which it originates.

As used herein, an "antigen fragment" refers to a portion of an antigen that retains the ability to elicit an immune response. In particular, an antigen fragment comprises a sequence of amino acids derived from the original antigen and is capable of being recognized by the immune system, such as by antibodies or T-cell receptors. In a specific embodiment, the antigen fragment is an immunologically active fragment, meaning it retains the immunological properties of the original antigen from which it is derived and can bind to the same immune receptors or trigger the same immune response pathways.

As used herein, a "fusion protein" refers to a single polypeptide chain that is composed of two or more distinct protein or peptide sequences that are not naturally contiguous. These sequences are joined together through recombinant DNA techniques or chemical linkage, resulting in a single, continuous amino acid sequence. Each component of the fusion protein may retain its individual biological activity, or the fusion may create a new or enhanced functional property compared to the individual proteins.

As used herein, a "fusion sequence" refers to a nucleic acid or amino acid sequence that consists of two or more distinct sequences joined together, typically through recombinant techniques, to form a single, continuous sequence. The fusion sequence may encode a fusion protein or peptide with combined or novel biological activities. The fusion sequence may include a linker sequence to provide flexibility or proper folding between the joined polypeptide domains.

A "linker", "linking sequence", or "linker sequence", is to be understood herein as an amino acid sequence or a nucleic acid sequence that serves to link or fuse two other amino acid or nucleic acid sequences together. In particular, the linker's sole role is to link or fuse two amino acid or nucleic acid sequences together. In particular, the linker does not provide any additional biological activity to the amino acid or nucleic acid sequences it links or fuses.

It is to be noted that the term "a" or "an" entity refers to one or more of that entity; for example, "a segment," is understood to represent one or more segments. As such, the terms "a" (or "an"), "one or more," and "at least one" can be used interchangeably herein.

Throughout this specification and embodiments, the words "have" and "comprise," or variations such as "has," "having," "comprises," or "comprising," will be understood to imply the inclusion of a stated integer or group of integers but not the exclusion of any other integer or group of integers. The words "have" and "comprise," or variations such as "has," "having," "comprises," or "comprising," will be understood to imply the inclusion of the stated element(s) (such as a composition of matter or a method step) but not the exclusion of any other elements. The term "consisting of" implies the inclusion of the stated element(s), to the exclusion of any additional elements. The term "consisting essentially of" implies the inclusion of the stated elements, and possibly other element(s) where the other element(s) do not materially affect the basic characteristic(s) of the disclosure. It is understood that the different embodiments of the disclosure using the term "comprising" or equivalent cover the embodiments where this term is replaced with "comprising only", "consisting of" or "consisting essentially of".

It is understood that wherever aspects are described herein with the language "comprising," otherwise analogous aspects described in terms of "consisting of" and/or "consisting essentially of" are also provided.

### Lentiviral vector according to the invention

The inventors have conceived novel therapeutic and prophylactic lentiviral vector-based immunotherapies against prostate cancers, in particular against metastatic castration-sensitive prostate cancer and metastatic castration-resistant prostate cancer.

In particular, the present invention relates to a lentiviral vector comprising a nucleic acid sequence encoding at least one antigen sequence selected from the group consisting of human prostatic acid phosphatase (hPAP), human prostate specific antigen (hPSA), human prostate-specific membrane antigen (hPSMA), human prostate stem cell antigen (hPSCA), human six-transmembrane epithelial antigen of the prostate-1 (hSTEAP1), human trophoblast cell surface antigen 2 (hTROP2), human TCR gamma alternate reading frame protein (hTARP), and fragments thereof.

These antigens are pertinent targets in prostate cancers due to their overexpression and enrichment in prostate tumors (Sridaran et al., Cell Rep Med 4:101199 (2023) and Oh et al., Cancer research 64(7):2610-2618 (2004)).

In a particular embodiment, the lentiviral vector of the invention comprises a nucleic acid sequence encoding at least two antigen sequences independently selected from the group consisting of human prostatic acid phosphatase (hPAP), human prostate specific antigen (hPSA), human prostate-specific membrane antigen (hPSMA), human prostate stem cell antigen (hPSCA), human six-transmembrane epithelial antigen of the prostate-1 (hSTEAP1), human trophoblast cell surface antigen 2 (hTROP2), human TCR gamma alternate reading frame protein (hTARP), and fragments thereof.

The inclusion of two or more prostate antigens decreases the probability of tumor escape linked to a possible loss of expression of a single targeted antigen.

In another embodiment, the at least two antigen sequences may be derived from the same antigen, or from different antigens.

In a particular embodiment, the at least two antigen sequences may be identical, or differ from each other.

In a further embodiment, the at least two antigen sequences may independently include combinations of both full-length antigens and their fragments.

In a particular embodiment, the lentiviral vector comprises a nucleic acid sequence encoding the antigen sequence of (i) hPAP or at least a fragment thereof; and/or (ii) hPSA or at least a fragment thereof; and/or (iii) hTROP2 or at least a fragment thereof; and/or (iv) hTARP or at least a fragment thereof.

hPAP is a 50-kDa dimeric glycoprotein, whose function has not yet been precisely identified. hPAP is expressed on > 95% of prostate cancer cells and has been already used in the only approved prostate cancer vaccine "Sipuleucel-T" (Kantoff et al., N Engl J Med 363:411-422 (2010), Anassi et al., P T 36:197-202 (2011) and Thara et al., Maturitas 69:296-303 (2011)).

The 34 kDa glycoprotein hPSA is a neutral serine protease, involved in the liquefaction of semen coagulum (Lilja et al., World J Urol 11:188-191 (1993)). hPSA is a serum marker followed in men to screen the risk of developing prostate cancer or to monitor the disease progression or recurrence after initial diagnosis (Cunha et al., Cancer Lett 236:229-238 (2006)).

Due to its overexpression in multiple cancers and its correlation with tumor aggressiveness, poor prognosis, and resistance to treatment, human trophoblast cell surface antigen 2 (hTROP2) is classified as a tumor-associated antigen (TAA) and represents an attractive target for vaccine development (Bardia et al., The New England journal of medicine 380(8):741-751 (2019)). hTROP2 overexpression has been detected in several human cancers, including prostate cancers.

Further, it is now well established that human TCR gamma chain alternate reading frame protein (hTARP) is highly expressed in various human cancers, including androgen sensitive prostate tumors (Yue et al., Oral Surg Oral Med Oral Pathol Oral Radiol 123:468-476 (2017)). The expression of TARP transcripts is correlated with cell proliferation and metastases (Yue et al., Oral Surg Oral Med Oral Pathol Oral Radiol 123:468-476 (2017)). Among HLA-II-restricted TARP immunogenic regions, the native TARP:27-35 and an anchor-modified TARP:29-37 segment harboring the L9V substitution have already been used for immunotherapy in prostate cancer patients, either as adjuvanted peptides or loaded on autologous dendritic cells. These trials resulted in significant reduction in tumor growth rate in a majority of patients (Wood et al., Oncoimmunology 5:e1197459 (2016)).

In a further embodiment, the lentiviral vector comprises a nucleic acid sequence encoding the antigen sequence of (i) hPAP or at least a fragment thereof; or (ii) hPSA or at least a fragment thereof; or (iii) hTROP2 or at least a fragment thereof; or (iv) hTARP or at least a fragment thereof.

In particular, the lentiviral vector may comprise only a nucleic acid sequence encoding the antigen sequence of hPAP or a fragment thereof.

In particular, the lentiviral vector may comprise only a nucleic acid sequence encoding the antigen sequence of hPSA or a fragment thereof.

In particular, the lentiviral vector may comprise only a nucleic acid sequence encoding the antigen sequence of hTROP2 or a fragment thereof.

In particular, the lentiviral vector may comprise only a nucleic acid sequence encoding the antigen sequence of hTARP or a fragment thereof.

In an additional embodiment, the at least one or at least two antigen sequence(s) may be selected from the group consisting of hPAP, hPSA, hTROP2, hTARP and fragments thereof, and may include combinations of both full-length antigens and their fragments.

In another embodiment, the at least one or at least two antigen sequence(s) may be selected from the group consisting of hPAP, hPSA, hTROP2, hTARP and fragments thereof, and may include only full-length antigens or only their fragments.

In a further embodiment, the lentiviral vector of the invention comprises a nucleic acid sequence encoding the antigen sequence of hPAP or at least a fragment thereof, and the antigen sequence of hPSA or at least a fragment thereof; or the antigen sequence of hTROP2 or at least a fragment thereof.

In a particular embodiment, the lentiviral vector of the invention comprises a nucleic acid sequence encoding the antigen sequence of hPAP or at least a fragment thereof and of hPSA or at least a fragment thereof.

In a particular embodiment, the lentiviral vector of the invention comprises a nucleic acid sequence encoding the antigen sequence of hTROP2 or at least a fragment thereof.

According to an embodiment, the lentiviral vector comprises a nucleic acid sequence encoding at least a fragment of the antigen sequence of hPAP; and/or at least a fragment of the antigen sequence of hPSA; and/or at least a fragment of the antigen sequence of hTROP2.

According to an embodiment, the lentiviral vector comprises a nucleic acid sequence encoding at least a fragment of the antigen sequence of hPAP; and/or at least a fragment of the antigen sequence of hPSA.

According to an embodiment, the lentiviral vector comprises a nucleic acid sequence encoding at least a fragment of the antigen sequence of hPAP; or at least a fragment of the antigen sequence of hPSA; or at least a fragment of the antigen sequence of hTROP2.

According to an embodiment, the lentiviral vector comprises a nucleic acid sequence encoding at least a fragment of the antigen sequence of hPAP and at least a fragment of the antigen sequence of hPSA.

According to an embodiment, the lentiviral vector comprises a nucleic acid sequence encoding at least a fragment of the antigen sequence of hTROP2.

In a particular embodiment, the lentiviral vector according to the invention comprises a nucleic acid sequence encoding an amino acid sequence having at least 9 consecutive amino acids chosen from the sequence set forth as SEQ ID NO: 1; and/or an amino acid sequence having at least 9 consecutive amino acids chosen from the sequence set forth as SEQ ID NO: 2.

In a particular embodiment, the lentiviral vector according to the invention comprises at least one, in particular at least two, in particular at least three, in particular at least four, in particular at least five, more particularly from 2 to 5 nucleic acid sequence(s) independently encoding an amino acid sequence having at least 9 consecutive amino acids chosen from the sequence set forth as SEQ ID NO: 1; and/or an amino acid sequence having at least 9 consecutive amino acids chosen from the sequence set forth as SEQ ID NO: 2.

By "at least 9 consecutive amino acids" chosen from a given sequence, it is meant herein at least 9, at least 10, at least 11, at least 12, at least 13, at least 14 and in particular at least 15 consecutive amino acids of said sequence, in particular from 9 to 25 consecutive amino acids of said sequence, more particularly from 9 to 20 consecutive amino acids of said sequence, in particular from 12 to 18 consecutive amino acids of said sequence.

In another embodiment, the lentiviral vector according to the invention comprises a nucleic acid sequence encoding an amino acid sequence having at least 9 consecutive amino acids chosen from the sequence set forth as SEQ ID NO: 1.

The amino acid sequence set forth as SEQ ID NO: 1 represents the hPAP portion of the PROST-02 antigen sequence. This sequence encodes clusters of T-cell epitopes derived from hPAP.

In another embodiment, the lentiviral vector according to the invention comprises a nucleic acid sequence encoding an amino acid sequence having at least 9 consecutive amino acids chosen from the sequence set forth as SEQ ID NO: 1, more particularly comprises at least one, in particular at least two, in particular at least three, in particular at least four, in particular at least five, more particularly from 2 to 5 nucleic acid sequence(s) independently encoding an amino acid sequence having at least 9 consecutive amino acids chosen from the sequence set forth as SEQ ID NO: 1.

In another embodiment, the lentiviral vector according to the invention comprises a nucleic acid sequence encoding an amino acid sequence having at least 9, at least 10, at least 11, at least 12, at least 13, at least 14 and in particular at least 15 consecutive amino acids of said sequence, in particular from 9 to 25 consecutive amino acids of said sequence, more particularly from 9 to 20 consecutive amino acids of said sequence, in particular from 12 to 18 consecutive amino acids chosen from the sequence set forth as SEQ ID NO: 1,

more particularly comprises at least one, in particular at least two, in particular at least three, in particular at least four, in particular at least five, more particularly from 2 to 5 nucleic acid sequence(s) independently encoding an amino acid sequence having at least 9, at least 10, at least 11, at least 12, at least 13, at least 14 and in particular at least 15 consecutive amino acids of said sequence, in particular from 9 to 25 consecutive amino acids of said sequence, more particularly from 9 to 20 consecutive amino acids of said sequence, in particular from 12 to 18 consecutive amino acids chosen from the sequence set forth as SEQ ID NO: 1.

In another embodiment, the lentiviral vector according to the invention comprises a nucleic acid sequence encoding an amino acid sequence having at least 9 consecutive amino acids chosen from the sequence set forth as SEQ ID NO: 2.

The amino acid sequence set forth as SEQ ID NO: 2 represents the hPSA portion of the PROST-02 antigen sequence. This sequence encodes clusters of T-cell epitopes derived from hPSA.

In another embodiment, the lentiviral vector according to the invention comprises a nucleic acid sequence encoding an amino acid sequence having at least 9 consecutive amino acids chosen from the sequence set forth as SEQ ID NO: 2, more particularly comprises at least one, in particular at least two, in particular at least three, in particular at least four, in particular at least five, more particularly from 2 to 5 nucleic acid sequence(s) independently encoding an amino acid sequence having at least 9 consecutive amino acids chosen from the sequence set forth as SEQ ID NO: 2.

In another embodiment, the lentiviral vector according to the invention comprises a nucleic acid sequence encoding an amino acid sequence having at least 9, at least 10, at least 11, at least 12, at least 13, at least 14 and in particular at least 15 consecutive amino acids of said sequence, in particular from 9 to 25 consecutive amino acids of said sequence, more particularly from 9 to 20 consecutive amino acids of said sequence, in particular from 12 to 18 consecutive amino acids chosen from the sequence set forth as SEQ ID NO: 2,

more particularly comprises at least one, in particular at least two, in particular at least three, in particular at least four, in particular at least five, more particularly from 2 to 5 nucleic acid sequence(s) independently encoding an amino acid sequence having at least 9, at least 10, at least 11, at least 12, at least 13, at least 14 and in particular at least 15 consecutive amino acids of said sequence, in particular from 9 to 25 consecutive amino acids of said sequence, more particularly from 9 to 20 consecutive amino acids of said sequence, in particular from 12 to 18 consecutive amino acids chosen from the sequence set forth as SEQ ID NO: 2.

In a particular embodiment, the lentiviral vector according to the invention comprises a nucleic acid sequence encoding an amino acid sequence having at least 60% sequence identity, in particular at least 80% sequence identity, more particularly at least 90% sequence identity, with the sequence set forth as SEQ ID NO: 1; and/or an amino acid sequence having at least 60% sequence identity, in particular at least 80% sequence identity, more particularly at least 90% sequence identity, with the sequence set forth as SEQ ID NO: 2.

In a particular embodiment, the lentiviral vector according to the invention:
- comprises a nucleic acid sequence encoding an amino acid sequence having at least 60% sequence identity, in particular at least 80% sequence identity, more particularly at least 90% sequence identity, with the sequence set forth as SEQ ID NO: 1; and/or an amino acid sequence having at least 60% sequence identity, in particular at least 80% sequence identity, more particularly at least 90% sequence identity, with the sequence set forth as SEQ ID NO: 2; and
- comprises at least one, in particular at least two, in particular at least three, in particular at least four, in particular at least five, more particularly from 2 to 5 nucleic acid sequence(s) independently encoding an amino acid sequence having at least 9 consecutive amino acids chosen from the sequence set forth as SEQ ID NO: 1; and/or an amino acid sequence having at least 9 consecutive amino acids chosen from the sequence set forth as SEQ ID NO: 2.

In a particular embodiment, the lentiviral vector according to the invention:
- comprises a nucleic acid sequence encoding an amino acid sequence having at least 60% sequence identity, in particular at least 80% sequence identity, more particularly at least 90% sequence identity, with the sequence set forth as SEQ ID NO: 1; and/or an amino acid sequence having at least 60% sequence identity, in particular at least 80% sequence identity, more particularly at least 90% sequence identity, with the sequence set forth as SEQ ID NO: 2; and
- comprises at least one, in particular at least two, in particular at least three, in particular at least four, in particular at least five, more particularly from 2 to 5 nucleic acid sequence(s) independently encoding an amino acid sequence having at least 9 consecutive amino acids chosen from the sequence set forth as SEQ ID NO: 1; and/or an amino acid sequence having at least 9 consecutive amino acids chosen from the sequence set forth as SEQ ID NO: 2,
more particularly comprises at least one, in particular at least two, in particular at least three, in particular at least four, in particular at least five, more particularly from 2 to 5 nucleic acid sequence(s) independently encoding an amino acid sequence having at least 9, at least 10, at least 11, at least 12, at least 13, at least 14 and in particular at least 15 consecutive amino acids of said sequence, in particular from 9 to 25 consecutive amino acids of said sequence, more particularly from 9 to 20 consecutive amino acids of said sequence, in particular from 12 to 18 consecutive amino acids chosen from the sequence set forth as SEQ ID NO: 2.

In an embodiment, the lentiviral vector according to the invention comprises a nucleic acid sequence encoding at least an amino acid sequence having at least 60% sequence identity, in particular at least 80% sequence identity, more particularly at least 90% sequence identity, with the sequence set forth as SEQ ID NO: 1.

As described herein, an amino acid sequence or nucleic acid sequence having at least 60% amino acid identity or nucleic acid identity with a reference amino acid sequence or nucleic acid sequence encompasses amino acid sequences or nucleic acid sequences having at least 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99% amino acid identity or nucleic acid identity with the said reference amino acid sequence or nucleic acid sequence.

As described herein, an amino acid sequence or nucleic acid sequence having at least 80% amino acid identity or nucleic acid identity with a reference amino acid sequence or nucleic acid sequence encompasses amino acid sequences or nucleic acid sequences having at least 80%, 81%, 82%, 83%, 84%, 85%, 86%, 87%, 88%, 89%, 90%, 91%, 92%, 93%, 94%, 95%, 96%, 97%, 98% and 99% amino acid identity or nucleic acid identity with the said reference amino acid sequence or nucleic acid sequence.

As described herein, an amino acid sequence or nucleic acid sequence having at least 90% amino acid identity or nucleic acid identity with a reference amino acid sequence or nucleic acid sequence encompasses amino acid sequences or nucleic acid sequences having at least 90%, 91%, 92%, 93%, 94%, 95%, 96%, 97%, 98% and 99% amino acid identity or nucleic acid identity with the said reference amino acid sequence or nucleic acid sequence.

In yet another embodiment, the lentiviral vector comprises a nucleic acid sequence encoding at least an amino acid sequence set forth as SEQ ID NO: 1.

In another embodiment, the lentiviral vector according to the invention comprises a nucleic acid sequence encoding:
- an amino acid sequence having at least 60% sequence identity, in particular at least 80% sequence identity, more particularly at least 90% identity with the sequence set forth as SEQ ID NO: 1; and
- an amino acid sequence having at least 9 consecutive amino acids chosen from the sequence set forth as SEQ ID NO: 1, more particularly comprises at least one, in particular at least two, in particular at least three, in particular at least four, in particular at least five, more particularly from 2 to 5 nucleic acid sequence(s) independently encoding an amino acid sequence having at least 9 consecutive amino acids chosen from the sequence set forth as SEQ ID NO: 1.

In another embodiment, the lentiviral vector according to the invention comprises a nucleic acid sequence encoding:
- an amino acid sequence having at least 60% sequence identity, in particular at least 80% sequence identity, more particularly at least 90% identity with the sequence set forth as SEQ ID NO: 1; and
- an amino acid sequence having at least 9, at least 10, at least 11, at least 12, at least 13, at least 14 and in particular at least 15 consecutive amino acids of said sequence, in particular from 9 to 25 consecutive amino acids of said sequence, more particular from 9 to 20 consecutive amino acids of said sequence, in particular from 12 to 18 consecutive amino acids chosen from the sequence set forth as SEQ ID NO: 1,
more particularly comprises at least one, in particular at least two, in particular at least three, in particular at least four, in particular at least five, more particularly from 2 to 5 nucleic acid sequence(s) independently encoding an amino acid sequence having at least 9, at least 10, at least 11, at least 12, at least 13, at least 14 and in particular at least 15 consecutive amino acids of said sequence, in particular from 9 to 25 consecutive amino acids of said sequence, more particular from 9 to 20 consecutive amino acids of said sequence, in particular from 12 to 18 consecutive amino acids chosen from the sequence set forth as SEQ ID NO: 1.

In a particular embodiment, the lentiviral vector according to the invention comprises a nucleic acid sequence encoding at least an amino acid sequence having at least 60% sequence identity, in particular at least 80% sequence identity, more particularly at least 90% identity with the sequence set forth as SEQ ID NO: 2.

In yet another embodiment, the lentiviral vector comprises a nucleic acid sequence encoding at least an amino acid sequence set forth as SEQ ID NO: 2.

In another embodiment, the lentiviral vector according to the invention comprises a nucleic acid sequence encoding:
- an amino acid sequence having at least 60% sequence identity, in particular at least 80% sequence identity, more particularly at least 90% identity with the sequence set forth as SEQ ID NO: 2; and
- an amino acid sequence having at least 9 consecutive amino acids chosen from the sequence set forth as SEQ ID NO: 2, more particularly comprises at least one, in particular at least two, in particular at least three, in particular at least four, in particular at least five, more particularly from 2 to 5 nucleic acid sequence(s) independently encoding an amino acid sequence having at least 9 consecutive amino acids chosen from the sequence set forth as SEQ ID NO: 2.

In another embodiment, the lentiviral vector according to the invention comprises a nucleic acid sequence encoding:
- an amino acid sequence having at least 60% sequence identity, in particular at least 80% sequence identity, more particularly at least 90% identity with the sequence set forth as SEQ ID NO: 2; and
- an amino acid sequence having at least 9, at least 10, at least 11, at least 12, at least 13, at least 14 and in particular at least 15 consecutive amino acids of said sequence, in particular from 9 to 25 consecutive amino acids of said sequence, more particular from 9 to 20 consecutive amino acids of said sequence, in particular from 12 to 18 consecutive amino acids chosen from the sequence set forth as SEQ ID NO: 2,
more particularly comprises at least one, in particular at least two, in particular at least three, in particular at least four, in particular at least five, more particularly from 2 to 5 nucleic acid sequence(s) independently encoding an amino acid sequence having at least 9, at least 10, at least 11, at least 12, at least 13, at least 14 and in particular at least 15 consecutive amino acids of said sequence, in particular from 9 to 25 consecutive amino acids of said sequence, more particular from 9 to 20 consecutive amino acids of said sequence, in particular from 12 to 18 consecutive amino acids chosen from the sequence set forth as SEQ ID NO: 2.

In a particular embodiment, the lentiviral vector comprises a nucleic acid sequence encoding at least an amino acid sequence having at least 9 consecutive amino acids chosen from the sequence set forth as SEQ ID NO: 1, and an amino acid sequence having at least 9 consecutive amino acids chosen from the sequence set forth as SEQ ID NO: 2.

In particular, the amino acid sequence having at least 9 consecutive amino acids chosen from the sequence set forth as SEQ ID NO: 1 is fused, directly or by the intermediate of a linker sequence, with the amino acid sequence having at least 9 consecutive amino acids chosen from the sequence set forth as SEQ ID NO: 2.

In particular, the amino acid sequence having at least at least 9 consecutive amino acids chosen from the sequence set forth as SEQ ID NO: 1 is fused, directly or by the intermediate of a linker sequence, at the N-terminal end of the amino acid sequence having at least at least 9 consecutive amino acids chosen from the sequence set forth as SEQ ID NO: 2.

In another embodiment, the amino acid sequence having at least at least 9 consecutive amino acids chosen from the sequence set forth as SEQ ID NO: 2 is fused, directly or by the intermediate of a linker sequence, at the N-terminal end of the amino acid sequence having at least at least 9 consecutive amino acids chosen from the sequence set forth as SEQ ID NO: 1.

In a particular embodiment, the lentiviral vector comprises a nucleic acid sequence encoding at least an amino acid sequence having at least 60% sequence identity, in particular at least 80% sequence identity, more particularly at least 90% sequence identity, with the sequence set forth as SEQ ID NO: 1, and an amino acid sequence having at least 60% sequence identity, in particular at least 80% sequence identity, more particularly at least 90% sequence identity, with the sequence set forth as SEQ ID NO: 2.

In another embodiment, the amino acid sequence having at least 60% sequence identity, in particular at least 80% sequence identity, more particularly at least 90% sequence identity, with the sequence set forth as SEQ ID NO: 1 is fused, directly or by the intermediate of a linker sequence, with the amino acid sequence having at least 60% sequence identity, in particular at least 80% sequence identity, more particularly at least 90% sequence identity, with the sequence set forth as SEQ ID NO: 2

In another embodiment, the amino acid sequence having at least 60% sequence identity, in particular at least 80% sequence identity, more particularly at least 90% sequence identity, with the sequence set forth as SEQ ID NO: 1 is fused, directly or by the intermediate of a linker sequence, at the N-terminal end of the amino acid sequence having at least 60% sequence identity, in particular at least 80% sequence identity, more particularly at least 90% sequence identity, with the sequence set forth as SEQ ID NO: 2.

In another embodiment, the amino acid sequence having at least 60% sequence identity, in particular at least 80% sequence identity, more particularly at least 90% sequence identity, with the sequence set forth as SEQ ID NO: 2 is fused, directly or by the intermediate of a linker sequence, at the N-terminal end of the amino acid sequence having at least 60% sequence identity, in particular at least 80% sequence identity, more particularly at least 90% sequence identity, with the sequence set forth as SEQ ID NO: 1.

In a particular embodiment, the lentiviral vector comprises a nucleic acid sequence encoding at least an amino acid sequence set forth as SEQ ID NO: 1 and an amino acid sequence set forth as SEQ ID NO: 2.

In another embodiment, the amino acid sequence set forth as SEQ ID NO: 1 is fused, directly or by the intermediate of a linker sequence, with the amino acid sequence set forth as SEQ ID NO: 2.

In another embodiment, the amino acid sequence set forth as SEQ ID NO: 1 is fused, directly or by the intermediate of a linker sequence, at the N-terminal end of the amino acid sequence set forth as SEQ ID NO: 2.

In another embodiment, the amino acid sequence set forth as SEQ ID NO: 2 is fused, directly or by the intermediate of a linker sequence, at the N-terminal end of the amino acid sequence set forth as SEQ ID NO: 1.

In a particular embodiment, the lentiviral vector may further comprise a nucleic acid sequence encoding at least one linker sequence. In some embodiments, the at least one linker sequence comprises an amino acid sequence set forth as SEQ ID NO: 11. In some embodiments, the at least one linker sequence consists in the amino acid sequence set forth as SEQ ID NO: 11. In a particular embodiment, the amino acid sequence set forth as SEQ ID NO: 11 is encoded by the nucleic acid sequence set forth as SEQ ID NO: 27.

In a particular embodiment, where the lentiviral vector comprises at least two antigen sequences and/or fragments thereof, the antigen sequences and/or fragments thereof may be fused together with or without a linker sequence.

In some embodiments, the antigen sequences and/or fragments thereof are fused without a linker sequence. In some embodiments, the antigen sequences and/or fragments thereof are directly fused to one another.

In a particular embodiment, the linker sequence may be positioned between each antigen sequence and/or fragment thereof, or alternatively, it may be located centrally, or only between selected antigen sequences and/or fragments thereof.

By "positioned between", it is understood that the linker is located at the C-terminal end of a first sequence and at the N-terminal end of a second sequence.

In a particular embodiment, the sequences set forth as SEQ ID NO: 1 and SEQ ID NO: 2 may be fused together with or without a linker sequence.

In a particular embodiment, the sequences set forth as SEQ ID NO: 1 and SEQ ID NO: 2 are fused without a linker sequence. In a particular embodiment, the sequences set forth as SEQ ID NO: 1 and SEQ ID NO: 2 are directly fused to one another.

In a particular embodiment, the sequences set forth as SEQ ID NO: 1 and SEQ ID NO: 2 are linked together by an amino acid sequence comprising the sequence set forth as SEQ ID NO: 11. In an embodiment, the sequences set forth as SEQ ID NO: 1 and SEQ ID NO: 2 are linked together by the amino acid sequence consisting of the amino acid sequence set forth as SEQ ID NO: 11.

In an embodiment, the linker sequence set forth as SEQ ID NO: 11 is positioned between the C-terminal end of the amino acid sequence set forth as SEQ ID NO: 1 and the N-terminal end of the amino acid sequence set forth as SEQ ID NO: 2.

In an embodiment, the linker sequence set forth as SEQ ID NO: 11 is positioned between the C-terminal end of the amino acid sequence set forth as SEQ ID NO: 2 and the N-terminal end of the amino acid sequence set forth as SEQ ID NO: 1.

In a particular embodiment, the amino acid sequence set forth as SEQ ID NO: 1 is encoded by the nucleic acid sequence set forth as SEQ ID NO: 17.

In a particular embodiment, the amino acid sequence set forth as SEQ ID NO: 2 is encoded by the nucleic acid sequence set forth as SEQ ID NO: 18.

In a particular embodiment, the lentiviral vector according to the invention comprises a nucleic acid sequence encoding at least an amino acid sequence having at least 9 consecutive amino acids chosen from the sequence set forth as SEQ ID NO: 3.

The amino acid sequence set forth as SEQ ID NO: 3 may be referred to elsewhere in the specification as the "PROST-02" antigen sequence. This sequence encodes clusters of T-cell epitopes derived from hPAP and hPSA.

In another embodiment, the lentiviral vector according to the invention comprises a nucleic acid sequence encoding an amino acid sequence having at least 9 consecutive amino acids chosen from the sequence set forth as SEQ ID NO: 3, more particularly comprises at least one, in particular at least two, in particular at least three, in particular at least four, in particular at least five, more particularly from 2 to 5 nucleic acid sequence(s) independently encoding an amino acid sequence having at least 9 consecutive amino acids chosen from the sequence set forth as SEQ ID NO: 3.

In another embodiment, the lentiviral vector according to the invention comprises a nucleic acid sequence encoding an amino acid sequence having at least 9, at least 10, at least 11, at least 12, at least 13, at least 14 and in particular at least 15 consecutive amino acids of said sequence, in particular from 9 to 25 consecutive amino acids of said sequence, more particularly from 9 to 20 consecutive amino acids of said sequence, in particular from 12 to 18 consecutive amino acids chosen from the sequence set forth as SEQ ID NO: 3,

more particularly comprises at least one, in particular at least two, in particular at least three, in particular at least four, in particular at least five, more particularly from 2 to 5 nucleic acid sequence(s) independently encoding an amino acid sequence having at least 9, at least 10, at least 11, at least 12, at least 13, at least 14 and in particular at least 15 consecutive amino acids of said sequence, in particular from 9 to 25 consecutive amino acids of said sequence, more particularly from 9 to 20 consecutive amino acids of said sequence, in particular from 12 to 18 consecutive amino acids chosen from the sequence set forth as SEQ ID NO: 3.

In a particular embodiment, the lentiviral vector according to the invention comprises a nucleic acid sequence encoding at least an amino acid sequence having at least 60% sequence identity, in particular at least 80% sequence identity, more particularly at least 90% sequence identity, with the sequence set forth as SEQ ID NO: 3.

In another embodiment, the lentiviral vector according to the invention comprises a nucleic acid sequence encoding:
- an amino acid sequence having at least 60% sequence identity, in particular at least 80% sequence identity, more particularly at least 90% identity with the sequence set forth as SEQ ID NO: 3; and
- an amino acid sequence having at least 9 consecutive amino acids chosen from the sequence set forth as SEQ ID NO: 3, more particularly comprises at least one, in particular at least two, in particular at least three, in particular at least four, in particular at least five, more particularly from 2 to 5 nucleic acid sequence(s) independently encoding an amino acid sequence having at least 9 consecutive amino acids chosen from the sequence set forth as SEQ ID NO: 3.

In another embodiment, the lentiviral vector according to the invention comprises a nucleic acid sequence encoding:
- an amino acid sequence having at least 60% sequence identity, in particular at least 80% sequence identity, more particularly at least 90% identity with the sequence set forth as SEQ ID NO: 3; and
- an amino acid sequence having at least 9, at least 10, at least 11, at least 12, at least 13, at least 14 and in particular at least 15 consecutive amino acids of said sequence, in particular from 9 to 25 consecutive amino acids of said sequence, more particular from 9 to 20 consecutive amino acids of said sequence, in particular from 12 to 18 consecutive amino acids chosen from the sequence set forth as SEQ ID NO: 3,
more particularly comprises at least one, in particular at least two, in particular at least three, in particular at least four, in particular at least five, more particularly from 2 to 5 nucleic acid sequence(s) independently encoding an amino acid sequence having at least 9, at least 10, at least 11, at least 12, at least 13, at least 14 and in particular at least 15 consecutive amino acids of said sequence, in particular from 9 to 25 consecutive amino acids of said sequence, more particular from 9 to 20 consecutive amino acids of said sequence, in particular from 12 to 18 consecutive amino acids chosen from the sequence set forth as SEQ ID NO: 3.

In yet another embodiment, the lentiviral vector comprises a nucleic acid sequence encoding at least an amino acid sequence set forth as SEQ ID NO: 3.

In a particular embodiment, the amino acid sequence set forth as SEQ ID NO: 3 is encoded by the nucleic acid sequence set forth as SEQ ID NO: 19.

In a particular embodiment, the lentiviral vector comprises a nucleic acid sequence encoding at least one hTROP2 antigen or at least a fragment thereof.

In a particular embodiment, the hTROP2 antigen sequence, or at least a fragment thereof, is selected from the group consisting of:
(a) an amino acid sequence having at least 9 consecutive amino acids chosen from the sequence set forth as SEQ ID NO: 4; and/or
(b) an amino acid sequence having at least 9 consecutive amino acids chosen from the sequence set forth as SEQ ID NO: 5; and/or
(c) an amino acid sequence having at least 9 consecutive amino acids chosen from the sequence set forth as SEQ ID NO: 6; and/or
(d) an amino acid sequence having at least 9 consecutive amino acids chosen from the sequence set forth as SEQ ID NO: 7; and/or
(e) an amino acid sequence having at least 9 consecutive amino acids chosen from the sequence set forth as SEQ ID NO: 8.

The amino acid sequences set forth as SEQ ID NO: 4, SEQ ID NO: 5, SEQ ID NO: 6, SEQ ID NO: 7 and SEQ ID NO: 8 correspond to five human hTROP2 antigen designs that may be incorporated into lentiviral vectors according to the invention.

As used herein "Lenti-TROP2-1" is a lentiviral vector of the invention that encodes the full-length human TROP2 sequence (SEQ ID NO: 4). "Lenti-TROP2-2" is a lentiviral vector of the invention that encodes the full-length human TROP2 sequence deleted of the 1-26 and 275-297 segments (SEQ ID NO: 5), corresponding respectively to the signal peptide and the transmembrane domain. "Lenti-TROP2-3" is a lentiviral vector of the invention that encodes the protein sequence as in Lenti-TROP2-2 and a sequence encoding for the mutated human ubiquitin C^{G77V} at N-terminal end of hTROP2 sequence (SEQ ID NO: 6). "Lenti-TROP2-4" is a lentiviral vector of the invention that encodes the full-length human TROP2 sequence deleted of the 1-26 and 275-297 segments, corresponding respectively to the signal peptide and the transmembrane domain, and deletion of S303 and S322 (SEQ ID NO: 7). "Lenti-TROP2-5" is a lentiviral vector that encodes the full-length human TROP2 deleted of the 1-26 and 275-323 segments, corresponding respectively to the signal peptide, the transmembrane domain, and the cytoplasmic tail (SEQ ID NO: 8).

In an embodiment, the hTROP2 antigen sequence, or at least a fragment thereof, consists of:
(a) an amino acid sequence having at least 9 consecutive amino acids chosen from the sequence set forth as SEQ ID NO: 4; or
(b) an amino acid sequence having at least 9 consecutive amino acids chosen from the sequence set forth as SEQ ID NO: 5; or
(c) an amino acid sequence having at least 9 consecutive amino acids chosen from the sequence set forth as SEQ ID NO: 6; or
(d) an amino acid sequence having at least 9 consecutive amino acids chosen from the sequence set forth as SEQ ID NO: 7; or
(e) an amino acid sequence having at least 9 consecutive amino acids chosen from the sequence set forth as SEQ ID NO: 8.

In another embodiment, the lentiviral vector according to the invention comprises a nucleic acid sequence encoding an amino acid sequence having at least 9 consecutive amino acids chosen from the sequence set forth as SEQ ID NO: 4; and/or an amino acid sequence having at least 9 consecutive amino acids chosen from the sequence set forth as SEQ ID NO: 5; and/or an amino acid sequence having at least 9 consecutive amino acids chosen from the sequence set forth as SEQ ID NO: 6; and/or an amino acid sequence having at least 9 consecutive amino acids chosen from the sequence set forth as SEQ ID NO: 7; and/or an amino acid sequence having at least 9 consecutive amino acids chosen from the sequence set forth as SEQ ID NO: 8.

In another embodiment, the lentiviral vector according to the invention comprises a nucleic acid sequence encoding an amino acid sequence having at least 9 consecutive amino acids chosen from the sequence set forth as SEQ ID NO: 4; or an amino acid sequence having at least 9 consecutive amino acids chosen from the sequence set forth as SEQ ID NO: 5; or an amino acid sequence having at least 9 consecutive amino acids chosen from the sequence set forth as SEQ ID NO: 6; or an amino acid sequence having at least 9 consecutive amino acids chosen from the sequence set forth as SEQ ID NO: 7; or an amino acid sequence having at least 9 consecutive amino acids chosen from the sequence set forth as SEQ ID NO: 8.

In a particular embodiment, the hTROP2 antigen sequence, or at least a fragment thereof, is selected from the group consisting of:
(a) an amino acid sequence having at least 60% sequence identity, in particular at least 80% sequence identity, more particularly at least 90% sequence identity, with the sequence set forth as SEQ ID NO: 4; and/or
(b) an amino acid sequence having at least 60% sequence identity, in particular at least 80% sequence identity, more particularly at least 90% sequence identity, with the sequence set forth as SEQ ID NO: 5; and/or
(c) an amino acid sequence having at least 60% sequence identity, in particular at least 80% sequence identity, more particularly at least 90% sequence identity, with the sequence set forth as SEQ ID NO: 6; and/or
(d) an amino acid sequence having at least 60% sequence identity, in particular at least 80% sequence identity, more particularly at least 90% sequence identity, with the sequence set forth as SEQ ID NO: 7; and/or
(e) an amino acid sequence having at least 60% sequence identity, in particular at least 80% sequence identity, more particularly at least 90% sequence identity, with the sequence set forth as SEQ ID NO: 8.

In an embodiment, the hTROP2 antigen sequence, or at least a fragment thereof, consists of:
(a) an amino acid sequence having at least 60% sequence identity, in particular at least 80% sequence identity, more particularly at least 90% sequence identity, with the sequence set forth as SEQ ID NO: 4; or
(b) an amino acid sequence having at least 60% sequence identity, in particular at least 80% sequence identity, more particularly at least 90% sequence identity, with the sequence set forth as SEQ ID NO: 5; or
(c) an amino acid sequence having at least 60% sequence identity, in particular at least 80% sequence identity, more particularly at least 90% sequence identity, with the sequence set forth as SEQ ID NO: 6; or
(d) an amino acid sequence having at 60% sequence identity, in particular at least 80% sequence identity, more particularly at least 90% sequence identity, with the sequence set forth as SEQ ID NO: 7; or
(e) an amino acid sequence having at least 60% sequence identity, in particular at least 80% sequence identity, more particularly at least 90% sequence identity, with the sequence set forth as SEQ ID NO: 8.

In another embodiment, the lentiviral vector according to the invention comprises a nucleic acid sequence encoding an amino acid sequence having at least 60% sequence identity, in particular at least 80% sequence identity, more particularly at least 90% sequence identity, with the sequence set forth as SEQ ID NO: 4; and/or an amino acid sequence having at least 60% sequence identity, in particular at least 80% sequence identity, more particularly at least 90% sequence identity, with the sequence set forth as SEQ ID NO: 5; and/or an amino acid sequence having at least 60% sequence identity, in particular at least 80% sequence identity, more particularly at least 90% sequence identity, with the sequence set forth as SEQ ID NO: 6; and/or an amino acid sequence having at least 60% sequence identity, in particular at least 80% sequence identity, more particularly at least 90% sequence identity, with the sequence set forth as SEQ ID NO: 7; and/or an amino acid sequence having at least 60% sequence identity, in particular at least 80% sequence identity, more particularly at least 90% sequence identity, with the sequence set forth as SEQ ID NO: 8.

In another embodiment, the lentiviral vector according to the invention comprises a nucleic acid sequence encoding an amino acid sequence having at least 60% sequence identity, in particular at least 80% sequence identity, more particularly at least 90% sequence identity, with the sequence set forth as SEQ ID NO: 4; or an amino acid sequence having at least 60% sequence identity, in particular at least 80% sequence identity, more particularly at least 90% sequence identity, with the sequence set forth as SEQ ID NO: 5; or an amino acid sequence having at least 60% sequence identity, in particular at least 80% sequence identity, more particularly at least 90% sequence identity, with the sequence set forth as SEQ ID NO: 6; or an amino acid sequence having at least 60% sequence identity, in particular at least 80% sequence identity, more particularly at least 90% sequence identity, with the sequence set forth as SEQ ID NO: 7; or an amino acid sequence having at least 60% sequence identity, in particular at least 80% sequence identity, more particularly at least 90% sequence identity, with the sequence set forth as SEQ ID NO: 8.

In particular, the lentiviral vector may include a nucleic acid sequence encoding the amino acid sequence set forth as SEQ ID NO: 4 or a fragment thereof.

In particular, the lentiviral vector may include a nucleic acid sequence encoding the amino acid sequence set forth as SEQ ID NO: 5 or a fragment thereof.

In particular, the lentiviral vector may include a nucleic acid sequence encoding the amino acid sequence set forth as SEQ ID NO: 6 or a fragment thereof.

In particular, the lentiviral vector may include a nucleic acid sequence encoding the amino acid sequence set forth as SEQ ID NO: 7 or a fragment thereof.

In particular, the lentiviral vector may include a nucleic acid sequence encoding the amino acid sequence set forth as SEQ ID NO: 8 or a fragment thereof.

In a particular embodiment, the amino acid sequence set forth as SEQ ID NO: 4 is encoded by the nucleic acid sequence set forth as SEQ ID NO: 20.

In a particular embodiment, the amino acid sequence set forth as SEQ ID NO: 5 is encoded by the nucleic acid sequence set forth as SEQ ID NO: 21.

In a particular embodiment, the amino acid sequence set forth as SEQ ID NO: 6 is encoded by the nucleic acid sequence set forth as SEQ ID NO: 22.

In a particular embodiment, the amino acid sequence set forth as SEQ ID NO: 7 is encoded by the nucleic acid sequence set forth as SEQ ID NO: 23.

In a particular embodiment, the amino acid sequence set forth as SEQ ID NO: 8 is encoded by the nucleic acid sequence set forth as SEQ ID NO: 24.

As mentioned previously, where the lentiviral vector comprises at least two antigen sequences and/or fragments thereof, the antigen sequences and/or fragments thereof may be fused together with or without a linker sequence.

In some embodiments, the antigen sequences and/or fragments thereof are fused without a linker sequence. In some embodiments, the antigen sequences and/or fragments thereof are directly fused to one another.

In a particular embodiment, the linker sequence may be positioned between each antigen sequence and/or fragment thereof, or alternatively, it may be located centrally, or only between selected antigen sequences and/or fragments thereof. By "positioned between", it is understood that the linker is located at the C-terminal end of a first sequence and at the N-terminal end of a second sequence.

In a particular embodiment, the hTARP antigen sequence, or at least a fragment thereof, is selected from the group consisting of:
- an amino acid sequence having at least 9 consecutive amino acids chosen from the sequence set forth as SEQ ID NO: 9; and/or
- an amino acid sequence having at least 9 consecutive amino acids chosen from the sequence set forth as SEQ ID NO: 10.

The sequences set forth as SEQ ID NO: 9 and SEQ ID NO: 10 represent two hTARP antigen designs that may be incorporated into lentiviral vectors according to the invention. Specifically, these sequences encode the N-terminal part of the sequence 2-58 of hTARP. The sequences of hTARP include: (i) P8L and R31A (SEQ ID NO: 9) or (ii) P8L and L37V (SEQ ID NO: 10) amino acid substitutions.

In an embodiment, the hTARP antigen sequence, or at least a fragment thereof, consists of:
- an amino acid sequence having at least 9 consecutive amino acids chosen from the sequence set forth as SEQ ID NO: 9; or
- an amino acid sequence having at least 9 consecutive amino acids chosen from the sequence set forth as SEQ ID NO: 10.

In a particular embodiment, the lentiviral vector according to the invention comprises a nucleic acid sequence encoding an amino acid sequence having at least 9 consecutive amino acids chosen from the sequence set forth as SEQ ID NO: 9; and/or an amino acid sequence having at least 9 consecutive amino acids chosen from the sequence set forth as SEQ ID NO: 10.

In another embodiment, the lentiviral vector according to the invention comprises a nucleic acid sequence encoding an amino acid sequence having at least 9 consecutive amino acids chosen from the sequence set forth as SEQ ID NO: 9.

In another embodiment, the lentiviral vector according to the invention comprises a nucleic acid sequence encoding an amino acid sequence having at least 9 consecutive amino acids chosen from the sequence set forth as SEQ ID NO: 10.

In a particular embodiment, the lentiviral vector comprises a nucleic acid sequence encoding at least an amino acid sequence having at least 9 consecutive amino acids chosen from the sequence set forth as SEQ ID NO: 9, and an amino acid sequence having at least 9 consecutive amino acids chosen from the sequence set forth as SEQ ID NO: 10.

In a particular embodiment, the hTARP antigen sequence, or at least a fragment thereof, is selected from the group consisting of:
- an amino acid sequence having at least 60% sequence identity, in particular at least 80% sequence identity, more particularly at least 90% sequence identity, with the sequence set forth as SEQ ID NO: 9; and/or
- an amino acid sequence having at least 60% sequence identity, in particular at least 80% sequence identity, more particularly at least 90% sequence identity, with the sequence set forth as SEQ ID NO: 10.

In an embodiment, the lentiviral vector according to the invention comprises a nucleic acid sequence encoding at least an amino acid sequence having at least 60% sequence identity, in particular at least 80% sequence identity, more particularly at least 90% sequence identity, with the sequence set forth as SEQ ID NO: 9.

In yet another embodiment, the lentiviral vector comprises a nucleic acid sequence encoding at least an amino acid sequence set forth as SEQ ID NO: 9.

In a particular embodiment, the lentiviral vector according to the invention comprises a nucleic acid sequence encoding at least an amino acid sequence having at least 60% sequence identity, in particular at least 80% sequence identity, more particularly at least 90% identity with the sequence set forth as SEQ ID NO: 10.

In yet another embodiment, the lentiviral vector comprises a nucleic acid sequence encoding at least an amino acid sequence set forth as SEQ ID NO: 10.

In a particular embodiment, the lentiviral vector comprises a nucleic acid sequence encoding at least an amino acid sequence having at least 60% sequence identity, in particular at least 80% sequence identity, more particularly at least 90% sequence identity, with the sequence set forth as SEQ ID NO: 9, and an amino acid sequence having at least 60% sequence identity, in particular at least 80% sequence identity, more particularly at least 90% sequence identity, with the sequence set forth as SEQ ID NO: 10.

In a particular embodiment, the amino acid sequence set forth as SEQ ID NO: 9 is encoded by the nucleic acid sequence set forth as SEQ ID NO: 25.

In a particular embodiment, the amino acid sequence set forth as SEQ ID NO: 10 is encoded by the nucleic acid sequence set forth as SEQ ID NO: 26.

In a particulier embodiment, the lentiviral vector further comprises an amino acid segment 6-80 of the mutated human ubiquitin C^{G77V}.

In particular, said amino acid segment 6-80 of the mutated human ubiquitin C^{G77V} is located at the C-terminal and/or at the N-terminal end of the antigen sequence.

In some embodiments, said amino acid segment 6-80 of the mutated human ubiquitin C^{G77V} is fused, directly or by the intermediate of a linker sequence, to the antigen sequence.

In a particular embodiment, the amino acid sequence of segment 6-80 of the mutated human ubiquitin C^{G77V} is set forth as SEQ ID NO: 12. In a particular embodiment, the segment 6-80 of the mutated human ubiquitin C^{G77V} is encoded by the nucleic acid set forth as SEQ ID NO: 28.

The amino acid segment 6-80 of the mutated human ubiquitin C^{G77V} (Liu et al., EMBO J 30:2990-3003 (2011)), may serve to favor the recognition and processing of the protein antigens by the proteasome and MHC-I presentation (Loureiro & Ploegh, Adv Immunol 92:225-305 (2006)).

In a particular embodiment, the lentiviral vector comprises a nucleic acid sequence encoding at least one human TCR gamma alternate reading frame protein (hTARP) or at least a fragment thereof and the amino acid segment 6-80 of the mutated human ubiquitin C^{G77V}.

The at least one human TCR gamma alternate reading frame protein (hTARP) or at least a fragment thereof may be as defined above.

The segment 6-80 of the mutated human ubiquitin C^{G77V} may be as defined above.

In a particular embodiment, the lentiviral vector comprises a nucleic acid sequence encoding at least one human TCR gamma alternate reading frame protein (hTARP) or at least a fragment thereof selected from the amino acid sequences set forth as SEQ ID NO: 9 or SEQ ID NO: 10 and a nucleic acid sequence encoding the segment 6-80 of the mutated human ubiquitin C^{G77V} having the amino acid sequence set forth as SEQ ID NO: 12.

In a particular embodiment, the amino acid sequence set forth as SEQ ID NO: 9 or SEQ ID NO: 10 is fused, directly or by the intermediate of a linker sequence, to the amino acid sequence set forth as SEQ ID NO: 12.

In a particular embodiment, the linker sequence may have the amino acid sequence set forth as SEQ ID NO: 14. In a particular embodiment, the linker sequence may have the nucleic acid sequence set forth as SEQ ID NO: 30.

In a particular embodiment, the amino acid sequence set forth as SEQ ID NO: 9 or SEQ ID NO: 10 is fused at the C-terminal end of the amino acid sequence set forth as SEQ ID NO: 12.

In a particular embodiment, the amino acid sequence set forth as SEQ ID NO: 9 or SEQ ID NO: 10 is fused at the N-terminal end of the amino acid sequence set forth as SEQ ID NO: 12.

In a particular embodiment, the at least one antigen sequence may further be fused, directly or by the intermediate of a linker sequence, to an immunological tag. In a particular embodiment, the at least one antigen sequence is fused by the intermediate of a linker sequence to an immunological tag.

The immunological tag may in particular be used in preclinical conditions.

In a particular embodiment, the immunological tag comprises the amino acid sequence set forth as SEQ ID NO: 13. In a particular embodiment, the immunological tag comprises the nucleic acid sequence set forth as SEQ ID NO: 29.

In a particular embodiment, the linker sequence may comprise the amino acid sequence set forth as SEQ ID NO: 14. In a particular embodiment, the linker sequence may comprise the nucleic acid sequence set forth as SEQ ID NO: 31.

In a particular embodiment, the lentiviral vector comprises a nucleic acid sequence encoding an amino acid sequence having at least 9 consecutive amino acids chosen from the sequence set forth as SEQ ID NO: 15 or SEQ ID NO: 16.

In a particular embodiment, the lentiviral vector comprises a nucleic acid sequence set forth as SEQ ID NO: 32 or SEQ ID NO: 33.

A lentiviral vector according to the invention may be single-stranded or doublestranded. A lentiviral vector according to the invention may be an RNA or DNA molecule.

In the context of the present invention, a "lentiviral vector" means a non-replicating vector for the transduction of a host cell with a transgene comprising cis-acting lentiviral RNA or DNA sequences, and requiring essential lentiviral proteins (e.g. Gag, Pol, and/or Env) and accessory proteins (e.g. Tat, Rev) that are provided in trans. The lentiviral vector lacks expression of all functional HIV proteins. The lentiviral vector genome may be present in the form of an RNA or DNA molecule, depending on the stage of production or development of said retroviral vectors.

In an embodiment, a lentiviral vector of the invention is an integrative lentiviral vector.

In a further embodiment, the integrase of these lentiviral vectors may not be mutated.

In a preferred embodiment, a lentiviral vector of the invention is a non-integrative lentiviral vector.

Non-integrative lentiviral vectors have been designed to mitigate the risks of potential oncogenesis linked to insertional mutagenesis events, particularly for vaccination purposes. Examples of non-integrative lentiviral vectors are provided in Coutant et al., PLOS ONE 7(11):e48644 (2012), Karwacz et al., J. Virol. 83(7):3094-3103 (2009), Negri et al., Molecular Therapy 15(9):1716-1723 (2007) and Hu et al., Vaccine 28:6675-6683 (2010). Consequently, it has been reported that a non-integrative lentiviral vector system can mitigate the potential risk of insertional mutagenesis as compared to an integrative system (Hu et al., Vaccine 28:6675-6683 (2010)). It has been further reported that in some functional analysis, both the magnitude and quality of the immune responses elicited by dendritic cell (DC)-directed integration-defective lentiviral vectors (IDLVs) are comparable to that of its integrative counterpart. Thus, integration-defective lentiviral vectors (IDLVs) have been considered safer vectors than integrative vectors for human administration, with comparable effectiveness.

A lentiviral vector according to the invention may comprise long terminal repeats (LTRs) sequences in cis as known in the art and in particular comprise a 3' long terminal repeat (LTR) which is devoid of its U3 promoter sequence (Miyoshi H et al., J Virol. 72(10):81 50-7 (1998) and Zufferey et al., J V/ro/ 72(12):9873-80 (1998)).

In a particular embodiment, a lentiviral vector may or may not comprise an enhancer sequence.

Enhancers are cis-acting sequences, which can act as transcriptional activators at a distance. They have been widely employed in viral derived vectors because they appear to be the most efficient for obtaining transgene strong expression in a variety of cell types, in particular DCs (Chinnasamy et al., Hum Gene Ther 11(13):1901-9 (2000), Rouas et al., Cancer Gene Ther 9(9):715-24 (2008), Kimura et al., Mol Ther 15(7):1390-9 (2007) and Gruh et al., J Gene Med 10(1):21-32 (2008)). However, given the safety issue of insertional mutagenesis, such transcriptional enhancer sequences should be deleted from the lentiviral vector constructs to abolish the risk of insertional mutagenesis by enhancer proximity effect. This enhancer proximity effect is by far the most frequent mechanism of insertional mutagenesis and is the only effect described in human or animal cases of tumorigenic events after gene transfer.

Accordingly, a lentiviral vector according to the invention may not comprise a constitutive enhancer sequence.

Previous studies have reported on the replacement of viral promoters by DC-specific promoters deriving from major histocompatibility complex class II genes (MHC class II) (Kimura et al., Mol Ther 15(7):1390-9 (2007)) and dectin-2 genes (Lopes et al., J Virol 82(1):86-95 (2008)). The dectin-2 gene promoter used in Lopes *et al.* contains a putative enhancer and an adenoviral conserved sequence (inverted terminal repeats in adenovirus promoter) (Bonkabara et al., J. Immunology, 167:6893-6900 (2001)). The MHC class II gene promoter used by Kimura *et al.* does not contain any known enhancer.

Yet, without an enhancer, the MHC class II promoter was found not to provide sufficient transgene expression in DCs, when administered intravenously. In particular, lentiviral vectors including MHC class II promoters did not provoke an immune reaction in immunocompetent C57BL/6 mice, in contrast to the immune responses observed with CMV promoters/enhancers. Although integration and persistent transgene expression were observed after injection in mice, the lentiviral vectors transcribed through MHC class II promoters failed to stimulate an antigen-specific CD8+ cytotoxic T-lymphocyte response, even after vaccination boost. The authors of these studies therefore concluded that the use of MHC class II promoters was of interest only for applications where persistence of expression is sought as in gene replacement therapy, but not in the context of immunotherapy. Of note, MHC class II promoters are expressed poorly in most cell types.

Thus, the MHC class II promoter is not an adequate promoter for lentiviral vectors for induction of an immune response against an antigen via IV injection. Moreover, the dectin-2 promoter is expressed poorly in most cell types and appears to contain an enhancer. Thus, the dectin-2 promoter is not a good promoter for lentiviral vectors for safety reasons.

Accordingly, a lentiviral vector according to the invention may comprise an MHC Class I promoter, i.e. the nucleic acid sequences encoding antigens of a lentiviral vector according to the invention may be under the control of an MHC Class I promoter.

An appropriate MHC Class I promoter may be selected from the group consisting of a β2-microglobulin promoter, a HLA-A2 promoter, a HLA-B7 promoter, a HLA-Cw5 promoter, a HLA-E promoter or a HLA-F promoter and is more particularly a β2-microglobulin promoter.

In a particular embodiment, the lentiviral vector may comprise a β2-microglobulin promoter.

In a particular embodiment, the lentiviral vector may comprise a human β2-microglobulin promoter (pβ2m).

MHC Class I promoters are dendritic-specific (APCs) in that expression of the promoter in BDCA+ dendritic cells is higher than the expression in kidney, smooth muscle, liver, and heart cells. They also have relatively high expression in other transduced cell types, for example, expression of the promoter in BDCA+ dendritic cells is only 12-100 times the expression of that promoter in skeletal muscle cells, in contrast to 900 times with the MHCII HLA-DRα promoter.

This promoter drives in particular the transcription of a nucleic acid sequence encoding at least one antigen sequence and/or fragment(s) thereof in a lentiviral vector of the invention.

Said promoter can be a naturally occurring or a synthetic MHC Class I promoter, obtained using well known molecular biological techniques.

The inventors generated a non-integrative lentiviral vector which encodes clusters of predicted or previously identified T-cell epitopes of human PAP and PSA, under the transcriptional control of the human β2 microglobulin promoter (Ku et al., Commun Biol 4:713 (2021)).

The inventors also generated non-integrative lentiviral vectors which encode the complete sequence of human TROP2 or fragments thereof under the transcriptional control of the human β2 microglobulin promoter.

In a particular embodiment, the lentiviral vector may comprise a cPPT/CTS sequence, such as described in EP2169073. This cPPT/CTS sequence may in particular be the sequence set forth as sequence SEQ ID NO: 34.

Indeed, efficient integration and replication in non-dividing cells generally requires the presence of two cis-acting sequences at the center of the lentiviral genome, the central polypurine tract (cPPT) and the central termination sequence (CTS). This leads to the formation of a triple-stranded DNA structure called the central DNA "flap", which acts as a signal for uncoating of the pre-integration complex at the nuclear pore and efficient importation of the expression cassette into the nucleus of non-dividing cells, such as dendritic cells.

In a particular embodiment, the lentiviral vector may comprise a Woodchuck hepatitis B virus (WHV) Post-Transcriptional Regulatory Element (WPRE), which allows a more stable expression of the transgene *in vivo*, and in particular a mutant form of the woodchuck hepatitis B virus (WHV) post-transcriptional regulatory element (WPRE).

The mutated Woodchuck Posttranscriptional Regulatory Element (mWPRE) is characterized in that point mutations are introduced to avoid expression of the X protein contained in the WPRE region as said X protein may have oncogenic properties (Kingsman et al., Gene Ther Jan 12(1):3-4 (2005)).

The mutant form of the woodchuck hepatitis B virus (WHV) post-transcriptional regulatory element (WPRE) comprised in a lentiviral vector of the invention may in particular have the nucleic acid sequence set forth as sequence SEQ ID NO: 35.

In a particular embodiment, the lentiviral vector comprises an MHC Class I promoter, and in particular a human β2-microglobulin promoter; and/or a cPPT/CTS sequence, in particular the cPPT/CTS sequence set forth as sequence SEQ ID NO: 34; and/or a 3' long terminal repeat (LTR) which is devoid of its U3 promoter sequence; and/or comprises a mutant form of the woodchuck hepatitis B virus (WHV) post-transcriptional regulatory element (WPRE), and in particular the mWPRE sequence set forth as sequence SEQ ID NO: 35.

In a particular embodiment, a lentiviral vector according to the invention:
(i) comprises a nucleic acid sequence encoding at least one antigen sequence selected from the group consisting of hPAP, hPSA, hPSMA, hPSCA, hSTEAP, hTROP2, hTARP and fragments thereof;
(ii) comprises a 3' long terminal repeat (LTR) which is devoid of its U3 promoter sequence;
(iii) comprises an MHC Class I promoter, and in particular a β2-microglobulin promoter;
(iv) comprises a cPPT/CTS sequence, having in particular the sequence set forth as sequence SEQ ID NO: 34; and
(v) comprises a mutant form of the woodchuck hepatitis B virus (WHV) post-transcriptional regulatory element (WPRE), having in particular the nucleic acid sequence set forth as sequence SEQ ID NO: 35.

### Lentiviral vector particles according to the invention

Another object of the present invention relates to a lentiviral vector particle comprising at least one lentiviral vector according to the invention, and in particular at least one lentiviral vector as defined above.

The lentiviral vector comprised in the lentiviral vector particle may be defined as a lentiviral vector described above.

A lentiviral vector particle according to the invention, which contains a lentiviral vector according to the invention, can be produced by recombinant technology known in the art upon transient transfection of cells, for example HEK 293T human cultured cells, by different DNA plasmids:
(i) a packaging plasmid, which expresses at least the Gag, Pol, Rev, Tat and, in some cases, structural and enzymatic proteins necessary for the packaging of the transfer construct;
(ii) a lentiviral vector according to the invention, containing an expression cassette (antigens) and HIV cis-acting factors necessary for packaging, reverse transcription, and integration; and
(iii) an envelope-encoding plasmid, in most cases the glycoprotein of vesicular stomatitis virus (VSV.G), a protein that allows the formation of mixed particles (pseudotypes) that can target a wide variety of cells, especially major histocompatibility (MHC) antigen-presenting cells (APCs), including DCs.

Such a method allows producing a recombinant vector particle according to the invention, comprising the following steps of:
i) transfecting a suitable host cell with a lentiviral vector according to the invention;
ii) transfecting said host cell with a packaging plasmid vector, containing viral DNA sequences encoding at least structural and polymerase activities of a retrovirus (preferably lentivirus); Such packaging plasmids are for example described in the art (Dull et al., J Virol, 72(11):8463-71 (1998) and Zufferey et al., J Virol 72(12):9873-80 (1998)).
iii) culturing said transfected host cell in order to obtain expression and packaging of said lentiviral vector into lentiviral vector particles; and
iv) harvesting the lentiviral vector particles resulting from the expression and packaging of step iii) in said cultured host cells.

In order to pseudotype the retroviral particles of the invention, the host cell can be further transfected with one or several envelope DNA plasmid(s) encoding viral envelope protein(s), preferably a VSV-G envelope protein.

This procedure allows obtaining transient production of lentiviral particle vectors by the transfected cells. However, the lentiviral particle vectors may also be continuously produced by cells by stably inserting the packaging genes, the proviral coding DNA, and the envelope gene into the cellular genome. This allows the continuous production of lentiviral particle vectors by the cells without the need for transient transfection. Of course, a combination of these procedures can be used, with some of the DNAs/plasmids integrated into the cellular genome and others provided by transient transfection.

A lentiviral vector particle may be a non-integrative lentiviral vector particle. Non-integrative vector particles have one or more mutations that eliminate most or all of the integrating capacity of the lentiviral vector particles. For example, a non-integrative vector particle can contain mutation(s) in the integrase encoded by the lentiviral pol gene that cause a reduction in integrating capacity. For example, the non-integrative property of the lentiviral vectors described herein may be linked to a D64V missense amino acid in the catalytic triad of integrase, which prevents the integration of viral DNA into the host chromosome.

A lentiviral vector particle according to the invention in particular comprises a non-integrative lentiviral vector of the invention.

A lentiviral vector particle may be an integrative lentiviral vector particle. The integrative property of the lentiviral vector used here allows the integration of viral DNA into multiple sites of the host chromosome.

A lentiviral vector particle according to the invention may comprise an integrative lentiviral vector of the invention.

A lentiviral vector particle according to the invention may comprise a vesicular stomatitis virus glycoprotein (VSVG), in particular a VSV-G Indiana serotype or a VSV-G New Jersey serotype.

In matter of vaccination strategy, pseudotyped lentiviral vector particles are more likely to escape the immune system, when this latter already developed immunity against lentiviruses. This is particularly helpful when successive injections of similar particle vectors are required for immunizing a patient against a disease.

### Isolated cells according to the invention

A further object of the present specification relates to an isolated cell comprising (i.e. transformed with) a lentiviral vector according to the invention or a lentiviral vector particle of the invention.

The lentiviral vector or the lentiviral vector particle comprised in the isolated cell may be defined as described above.

The cell is preferably a mammalian cell, particularly a human cell. Particularly preferred are human non-dividing cells.

### Pharmaceutical compositions according to the invention

Another object of the present invention relates to a pharmaceutical composition comprising, in a pharmaceutically acceptable medium, a lentiviral vector according to the invention, a lentiviral vector particle according to the invention or an isolated cell according to the invention.

The lentiviral vector or the lentiviral vector particle or the isolated cell comprised in the pharmaceutical composition may be defined as described above.

In a particular embodiment, the invention relates to a vaccine composition comprising, in a pharmaceutically acceptable medium, a lentiviral vector according to the invention, a lentiviral vector particle according to the invention or an isolated cell according to the invention.

A pharmaceutical composition or a vaccine composition according to the invention comprises a pharmaceutically acceptable medium.

By *"physiologically acceptable medium"* is meant any solution used to solubilize and deliver a lentiviral vector, a lentiviral vector particle or an isolated cell according to the invention to an individual. A desirable physiologically acceptable carrier is saline. In desirable embodiments, a physiologically acceptable medium includes an adjuvant.

Appropriate physiologically acceptable mediums and their formulations are known to one skilled in the art and described, for example, in Remington's Pharmaceutical Sciences, (20th edition), ed. A. Gennaro, 2003, Lippincott Williams & Wilkins.

### Combinatory treatments according to the invention

Another object of the present invention relates to a combinatory treatment.

A combinatory treatment refers to the use of two or more different therapeutic approaches simultaneously or sequentially to treat a disease, such as cancer. The rationale behind combinatory treatment is to enhance the effectiveness of therapy by targeting the disease in multiple ways, which can lead to better outcomes and/or fewer undesirable side effects compared to using a single treatment modality alone. In the case of cancer, cancer cells can develop resistance to single therapies. Using multiple treatments simultaneously can reduce the likelihood of cancer cells developing resistance, as they would need to adapt to several mechanisms of action at once.

The invention therefore relates to a combinatory treatment comprising:
(i) at least one lentiviral vector according to the invention, or lentiviral vector particle according to the invention, or isolated cell according to the invention, or pharmaceutical composition according to the invention, and
(ii) at least one anticancer agent or treatment and/or at least one immunotherapy agent or treatment.

The lentiviral vector or the lentiviral vector particle or the isolated cell or the pharmaceutical composition comprised in the combinatory treatment may be defined as described above.

Anticancer agents and treatments, in particular anticancer agents and treatments against prostate cancers are well known in the art.

In a particular embodiment, the at least one anticancer agent or treatment is selected from the group consisting of DNA methyltransferase inhibitors; histone deacetylase inhibitors; and adjuvants, in particular Toll-Like Receptor (TLR) agonists, poly I:C (polyinosinic: polycytidylic acid), the stabilized form of poly I:C (polyICLC), CpG oligodeoxynucleotide and cGAMP (Cyclic GMP-AMP); and mixtures thereof.

Similarly, immunotherapy agents and treatments against prostate cancers are well known in the art.

In a particular embodiment, the at least one immunotherapy agent or treatment is selected from the group consisting of immune checkpoint inhibitors, in particular anti-PD-1, anti-PD-L1, anti-CTLA-4, anti-TIM-3, and anti-LAG3, anti-TIGIT, and anti-NKG2A antagonist antibodies; and mixtures thereof.

Further immune checkpoints according to the invention are described below.

Further characteristics describing the administration of the combinatory treatment are provided below.

### Implementations according to the invention

An object of the present invention relates to a lentiviral vector of the invention, a lentiviral vector particle of the invention, an isolated cell of the invention, or a pharmaceutical composition of the invention, in particular in the form of a vaccine composition, for use in the treatment and/or prevention of prostate cancers, in particular of metastatic prostate cancers, more particularly of metastatic castration-resistant prostate cancers.

An object of the present specification also relates to a lentiviral vector of the invention, a lentiviral vector particle of the invention, an isolated cell of the invention, or a pharmaceutical composition of the invention for use as a medicament or vaccine.

It is further described the use of a lentiviral vector of the invention, a lentiviral vector particle of the invention, an isolated cell of the invention, or a pharmaceutical composition of the invention in the manufacture of a medicament, in particular a medicament for the treatment and/or prevention of prostate cancers.

In an embodiment, the prostate cancer is a prostate adenocarcinoma that may be (i) localized, (ii) with local progression, or (iii) metastatic, each of them being potentially castration (hormone) resistant.

In a particular embodiment, the cancers are metastatic prostate cancers.

In a particular embodiment, the cancers are metastatic castration-resistant prostate cancers.

Such prevention and/or treatment implies the administration of the lentiviral vector of the invention, lentiviral vector particle of the invention, the isolated cell of the invention, or the pharmaceutical composition of the invention, more particularly in the form of a vaccine composition, as defined above, to an individual in need thereof.

An individual in need thereof or a patient in need thereof is an animal, in particular a mammal, and may more particularly be a human being.

Lentiviral vectors, lentiviral vector particles, isolated cells and pharmaceutical compositions according to the invention are administered to an individual in need thereof by conventional methods, in dosages which are sufficient to elicit an immunological response, which can be easily determined by those skilled in the art.

Lentiviral vectors, lentiviral vector particles, isolated cells and pharmaceutical compositions according to the invention may accordingly be administered intravenously or intramuscularly as indicted below.

Lentiviral vectors, lentiviral vector particles, isolated cells and pharmaceutical compositions according to the invention are administered in a therapeutically effective amount, and may in particular be administered in a dose corresponding to at least 1 × 10⁶, 2 × 10⁶, 5 × 10⁶, 10⁷, 2 × 10⁷, 5 × 10⁷, 1 × 10⁸, 2 × 10⁸, 5 × 10⁸, or at least 1 × 10⁹ TU (Transduction units) of lentiviral vectors according to the invention, in particular in a dose corresponding to at least 1 × 10⁷, 2 × 10⁷, 5 × 10⁷, 1 × 10⁸ TU or at least 1 × 10⁹ TU of lentiviral vectors according to the invention. In a preferred embodiment, the lentiviral vectors, lentiviral vector particles, isolated cells and pharmaceutical compositions according to the invention are administered in a dose corresponding to at least 1 × 10⁷ TU of lentiviral vectors according to the invention, more particularly at least 1 × 10⁸ TU of lentiviral vectors according to the invention and in particular at least 1 × 10⁹ TU of lentiviral vectors according to the invention.

By a "therapeutically effective amount" is for example meant the amount of a lentiviral vector or lentiviral vector particle, isolated cell or pharmaceutical composition according to the invention required to generate in a subject one or more of the following effects: an immune response against prostate tumor(s); a decrease in the size of the prostate tumor(s), i.e. a reduction of at least 5 %, 10 %, 20 %, 30 %, 40 %, 50 %, 60 %, 70 %, 80 %, 90 %, or of 100 % of the tumor size in 15 to 45 days compared to the size of the tumor at the time of administration; an increase in tumor infiltrating lymphocytes with activated-effector phenotype in the prostate tumor(s) in 5 to 45 days following the administration; an increase in CD8⁺ T-cells in the prostate tumor(s) in 5 to 45 days following the administration; an engagement of the CD8⁺ T-cell compartment toward a CD44⁺ CD69⁺ KLRG1 (killer-cell lectin like receptor)⁺ phenotype and/or a PD1⁺ TIM3 (T cell immunoglobulin domain and mucin domain)⁺ phenotype in the prostate tumor(s) in 5 to 45 days following the administration.

Administration can be performed using well known routes including, for example, intravenous, intramuscular, intranasal, intraperitoneal or sub-cutaneous injection, and in particular intravenous, intranasal or intramuscular, and may be intravenous or intramuscular.

The appropriate dose and regimen will obviously vary between species and individuals depending on many factors. For example, higher doses will generally be required for an effective immune response in a human compared with a mouse.

Lentiviral vectors, lentiviral vector particles, isolated cells and pharmaceutical compositions according to the invention may for example be administered in a single dose, as illustrated in the examples, or in two or more administrations. Practitioners will determine, in each case, the appropriate regimen and dosage for the administration of actives according to the invention.

Lentiviral vectors, lentiviral vector particles, isolated cells and pharmaceutical compositions according to the invention may advantageously be administered in combination with at least one anticancer agent or treatment. In a particular embodiment, the lentiviral vectors, lentiviral vector particles, isolated cells and pharmaceutical compositions according to the invention may advantageously be administered in combination with at least one anticancer agent or treatment in the form of a combinatory treatment, in particular as described above.

An anticancer agent or treatment according to the invention may in particular be a DNA methyltransferase inhibitor, a histone deacetylase inhibitor, or an adjuvant, such as those described elsewhere in the text.

Lentiviral vectors, lentiviral vector particles, isolated cells and pharmaceutical compositions according to the invention may advantageously be administered in combination with at least one immune checkpoint inhibitor (ICI). In a particular embodiment, the lentiviral vectors, lentiviral vector particles, cells and pharmaceutical compositions according to the invention may advantageously be administered in combination with at least one ICI in the form of a combinatory treatment, in particular as described above.

An ICI according to the invention may in particular be an antibody, in particular an anti-PD-1, an anti-PD-L1 (PD-1 Ligand), an anti-CTLA-4 (Cytotoxic T-Lymphocyte-Associated protein 4), an anti-NKG2A (Natural killer group 2 member A), an anti-TIM-3 (T-cell immunoglobulin and mucin-domain containing-3), an anti-TIGIT (T cell immunoreceptor with Ig and ITIM domains) or an anti-LAG-3 (Lymphocyte-activation gene 3) antibody. More particularly, the at least one immune checkpoint inhibitor according to the invention may be a monoclonal antibody selected from the group consisting of anti-PD-1, anti-PD-L1, anti-CTLA-4, anti-NKG2A, anti-TIM-3, anti-TIGIT and anti-LAG-3 monoclonal antibodies. Even more particularly, the at least one immune checkpoint inhibitor according to the invention may be a monoclonal antibody selected from the group consisting of anti-PD-1, anti-PD-L1, anti-CTLA-4, anti-NKG2A, anti-TIM-3 and anti-TIGIT monoclonal antibodies.

An ICI according to the invention may more particularly be an antibody, in particular an anti-PD-1, an anti-PD-L1, an anti-CTLA-4, an anti-NKG2A, an anti-TIM-3, an anti-TIGIT or an anti-LAG-3 antibody and even more particularly be an antibody, in particular an anti-PD-1, an anti-PD-L1, an anti-CTLA-4, an anti-NKG2A, an anti-TIM-3 or an anti-TIGIT antibody. More particularly, the at least one immune checkpoint inhibitor according to the invention may be a monoclonal antibody selected from the group consisting of anti-PD-1, anti-PD-L1, anti-CTLA-4, anti-NKG2A, anti-TIM-3, anti-TIGIT and anti-LAG-3 monoclonal antibodies and in particular may be a monoclonal antibody selected from the group consisting of anti-PD-1, anti-PD-L1, anti-CTLA-4, anti-NKG2A, anti-TIM-3 and anti-TIGIT monoclonal antibodies.

An anti-PD-1 monoclonal antibody may for example be selected from the group consisting of Nivolumab, Pembrolizumab and Cemiplimab.

An anti-PD-L1 monoclonal antibody may for example be selected from the group consisting of Atezolizumab, Avelumab and Durvalumab.

An anti-CTLA-4 monoclonal antibody may for example be selected from the group consisting of ipilimumab, tremelimumab and quavonlimab.

NKG2A is an ITIM (intracytoplasmic tyrosine-based inhibitory motifs)-bearing receptor expressed at the surface of 50% of peripheral blood NK cells and 5% of human peripheral blood CD8+ T cells. This cell surface molecule is expressed as a heterodimer with CD94 and interacts with the Major Histocompatibility Complex class I (MHC-I) non-classical molecules, i.e. human leukocyte antigen (HLA)-E in and murine Qa-1b. This interaction inhibits both T and NK effector anti-tumor functions (Andre et al., Cell 175(7):1731-1743 (2018)). It has been described that an anti-NKG2A mAb can be used as a checkpoint inhibitor and can promote anti-tumor cellular immunity by unleashing not only NK, but also CD8⁺ T cells in mice (Andre et al., Cell 175(7):1731-1743 (2018)). An anti-NKG2A monoclonal antibody may for example be monalizumab.

T-cell immunoglobulin and mucin domain-3 (TIM-3) is a negative regulatory immune checkpoint. TIM-3 is expressed by various immune cells, notably T cells. TIM-3 has four ligands, including galectin-9 (Gal-9), carcinoembryonic antigen cell adhesion molecule 1 (CEACAM-1), high-mobility group protein B1 (HMGB1), and phosphatidylserine (PS) (He et al., Onco Targets Ther 11:7005-7009 (2018)). TIM-3/Gal-9 can inhibit cancer immunity by negatively regulating T-cell immunity. TIM-3 displays an important role in T-cell exhaustion. In cancer immunotherapy anti-TIM-3 mAb treatment displays beneficial effects comparable to those of anti-PD-1 mAb therapy. An anti-TIM-3 monoclonal antibody may for example be selected from the group consisting of Sym023 and sabatolimab.

An anti-TIGIT monoclonal antibody may for example be tiragolumab.

An anti-LAG-3 monoclonal antibody may for example be relatlimab.

In particular, the ICI may be an anti-PD-L1 or an anti-PD-1 monoclonal antibody, and in particular be an anti-PD-1 monoclonal antibody.

In particular, the ICI may be selected in the group consisting of an anti-PD-L1 an anti-NKG2A, an anti-TIM-3 and an anti-PD-1 monoclonal antibody, and more particularly in the group consisting of an anti-NKG2A, an anti-TIM-3 and an anti-PD-1 monoclonal antibody.

The pharmaceutical composition, lentiviral vector, lentiviral vector particle or isolated cell for use according to the invention and the immune checkpoint inhibitor may be administered simultaneously or separately.

By simultaneously, it is understood that (i) the pharmaceutical or vaccine composition, lentiviral vector, lentiviral vector particle or isolated cell and (ii) the immune checkpoint inhibitor, may be administered at the same moment or up to the same day or couple of days. In this case, they can be administered in the same composition or in separate compositions.

By separately, it is understood that (i) the pharmaceutical composition, lentiviral vector, lentiviral vector particle or isolated cell according to the invention and (ii) the immune checkpoint inhibitor may be administered with at least several days, for example at least two days of difference.

In particular, when (i) the pharmaceutical composition, lentiviral vector, lentiviral vector particle or isolated cell and (ii) the immune checkpoint inhibitor are administered separately, the pharmaceutical composition, lentiviral vector, lentiviral vector particle or isolated cell according to the invention may be administered before the immune checkpoint inhibitor.

The same characteristics may be applied to the combinatory therapy according to the invention and described above.

Advantageously, the pharmaceutical composition, lentiviral vector, lentiviral vector particle or isolated cell according to the invention may be administered at least 2 and in particular at least 4 days before the administration of the immune checkpoint inhibitor. Accordingly, the immune checkpoint inhibitor may advantageously be administered at least 2 and in particular at least 4 days after the pharmaceutical composition, lentiviral vector, lentiviral vector particle or isolated cell according to the invention. The immune checkpoint inhibitor may more particularly be administered 4 days to 1 month after the pharmaceutical composition, lentiviral vector, lentiviral vector particle or isolated cell according to the invention, in particular 4 days to 15 days, and more particularly 4 days to 10 days after the pharmaceutical composition, lentiviral vector, lentiviral vector particle or isolated cell according to the invention.

The pharmaceutical composition, lentiviral vector, lentiviral vector particle, isolated cell or pharmaceutical composition for use according to the invention and the immune checkpoint inhibitor may be administered by the same route or through different routes.

The at least one immune checkpoint inhibitor herein is administered in a therapeutically effective dose, i.e. a dose that produces the effects for which it is administered. The exact dose of immune checkpoint inhibitor will depend on the purpose of the treatment and will be ascertainable by one skilled in the art using known techniques.

The characteristics described above for the use of a lentiviral vector, lentiviral vector particle, isolated cell or pharmaceutical composition in combination with a checkpoint inhibitor may be applied to the combinatory treatment according to the invention and described elsewhere in the text.

The specification further relates to a method for the treatment and/or prevention of prostate cancers in an individual in need thereof, comprising the administration to said individual of at least one lentiviral vector of the invention, lentiviral vector particle of the invention, isolated cell of the invention, or pharmaceutical or vaccine composition according to the invention.

The invention further relates to the use of at least one lentiviral vector of the invention, lentiviral vector particle of the invention, isolated cell of the invention, or pharmaceutical composition of the invention, in particular in the form of a vaccine composition according to the invention, for the treatment and/or prevention of prostate cancers in an individual in need thereof.

In particular, the cancers are prostate cancers, in particular metastatic prostate cancers, more particularly metastatic castration-resistant prostate cancers.

The lentiviral vector of the invention, lentiviral vector particle of the invention, isolated cell of the invention, or pharmaceutical composition of the invention, in particular in the form of a vaccine composition according to the invention may be as described above.

The examples and figures which follow are presented by way of illustration and without implied limitation of the invention.

### EXAMPLES

### Materials and methods

### Mice

C57BL/6JRj mice were purchased from Janvier Labs (Le Genest Saint Isle, France). All animals were maintained under specific pathogen-free conditions, and all procedures were performed according to an approved animal protocol and in accordance with recommendations for the proper use and care of laboratory animals. All animal experiments were conducted in accordance with guidelines established by the French and European regulations for the care and use of laboratory animals.

### Lenti-PROST-02 production

Codon-optimized sequence of the poly-epitopic hPAP-hPSA antigen (herein after called PROST-02 antigen) was synthesized by Genecust (Boynes, France) and inserted into the pFlap lentiviral plasmid, between the BamHI and XhoI sites, located between the human β2-microglobulin promoter and the mutated *atg* starting codon of the woodchuck post-transcriptional regulatory element (WPRE) sequence, as previously described (Ku et al. Commun Biol 4:713 (2021), Ku et al. Expert Rev Vaccines 20:1571-1586 (2021), Nemirov et al. Pharmaceutics 15(3):846 (2023), Anna et al. Mucosal Immunol 15:1389-1404 (2022), Cousin et al. Cell Rep 26:1242-1257 (2019), Demidova et al. Expert Rev Vaccines 23(1):674-687 (2024) and Douguet et al. EMBO Mol Med 15(10):e17723 (2023)).

Production of non-integrative Lenti-PROST-02 vector was performed in the human embryonic kidney HEK 293 T cell production system by a tripartite transfection with: (i) the lentiviral plasmid encoding the PROST-02 antigen (SEQ ID NO: 3), (ii) an "envelope" plasmid which encodes the envelope glycoprotein of Vesicular Stomatitis Virus (VSV-G) from Indiana serotype and placed under the transcriptional control of cytomegalovirus CMV promoter, and (iii) a packaging plasmid harboring *gag, pol, tat,* and *rev* genes. The integrase resulting from the packaging plasmid carries a missense amino acid in its catalytic triad, i.e. the D64V mutation, which prevents the integration of viral DNA into the host chromosome (Ku et al. Expert Rev Vaccines 20:1571-1586 (2021)).

Recombinant Lenti-PROST-02 vector particles, were successfully produced and concentrated by ultracentrifugation with titers ranging from 1 to 20 × 10¹⁰ TU/mL.

### Lenti-TROP2-1 and Lenti-TROP2-2 production

For T-cell epitope mapping, 15-mer overlapping peptides (overlapping by 11 amino acids) (purity ≥85%), spanning the complete sequence of the hTROP2 antigen, synthesized by Proteogenix (Strasbourg, France) were used.

The antigen constructs were cloned into a mutated pFlap-β2-microglobulin (B2m)Ag-WPRE backbone. This plasmid includes the cPPT/CTS sequence (SEQ ID NO: 34), essential for transducing non-dividing cells. To prevent vector replication, the U3 promoter was deleted from the 3' long terminal repeat (LTR) region. The β2-microglobulin (β2m) promoter drives antigen expression in transduced cells, with preferential expression in antigen-presenting cells. The plasmid lacks known enhancer sequences, minimizing the risk of mutagenesis or genotoxicity. Additionally, it carries a mutant woodchuck hepatitis virus (WHV) post-transcriptional regulatory element (WPRE) (SEQ ID NO: 35). Unlike the wild-type WPRE, which contains a truncated protein with potential oncogenic activity, the mutant version includes point mutations in the protein start codon, preventing oncogenic effects (Themis et al., Mol Ther. (2005)).

The packaging plasmid (pNDK) contains the gag-pol sequences from HIV-1 subtype NDK (GenBank acc. n°A34828), but does not express nef, vif, vpr, or env proteins. A D64V mutation in the HIV-1 integrase (pol gene) inhibits viral integration while allowing transgene expression *in vitro,* making the lentiviral particles non-integrative.

For the envelope plasmid, the pCMV-VSV-G INDco (Indiana) vector was constructed by subcloning the Vesicular Stomatitis Virus (VSV) G protein from the Indiana (GenBank acc. n°J02428) serotype into the pVAX1 expression vector (Invitrogen). Synthetic mammalian codon-optimized genes (GeneArt) encoding glycoproteins from various Vesiculoviruses were cloned into the pVAX1 plasmid, including VSV Indiana serotype (GenBank acc. n°FW591952), VSV New Jersey serotype (GenBank acc. n°FW591956), and Cocal virus (GenBank acc. n°AF045556.1).

After amplification of HEK 293 T cells (ATCC) in DMEM with 1% penicillin/streptomycin and 10% fetal calf serum, non-integrative lentiviral particles were produced by transient calcium phosphate co-transfection of HEK 293 T cells (ATCC) with 3 plasmids: (i) the transfer plasmid, (ii)VSV-G encoding expression plasmid and (iii) packaging plasmid (culture medium was replaced by serum free medium after 4 hours). Supernatant was harvested at 48 hours after transfection and clarified by 2500 rpm centrifugation. Viral particles were concentrated by ultracentrifugation, during 1 hour at 22000 rpm, and resuspended in 20 mM Pipes, 75 mM NaCl and 2.5% sucrose buffer.

### Vector titration

Lentiviral vector titer was determined by quantitative PCR after transduction of HEK 293 T cells. Aphidicolin (8 µM) was added to HEK 293 T cells 24 hours before transduction and was maintained during the whole titration process. At 72 hours post transduction, cells were incubated 30 minutes with lysis buffer (200 mM Tris, 1% NP40 and 1% Tween20), containing 50 µg/mL RNase A (Sigma). Proteinase K (0.2 mg/mL) was added to the suspension which was incubated 4 hours at 56°C. Inactivation of Proteinase K was realized by heating the suspension 10 minutes à 95°C. Pairs of primers specific to the RRE coding region for the pflap sequence in the lentiviral vector (Forward: SEQ ID NO: 36; Reverse : SEQ ID NO: 37), along with primers for the housekeeping gene Glyceraldehyde-3-Phosphate Dehydrogenase (GAPDH) (Forward: SEQ ID NO: 38; Reverse: SEQ ID NO: 39) in the host cells, were used for quantitative PCR. Titer is expressed as TU/mL.

### Tumor cell line

The EL4 parental cell line (ATCC, TIB-3) was stably transduced, at a multiplicity of infection (MOI) of 10, by an integrative lentiviral vector encoding the full-length human PSA under the transcriptional control of the ubiquitin (UBC) promoter (Christensen et al. Transgenic Res 5:213-218 (1996) and Wiborg et al. EMBO J 4:755-759 (1985)). A positive bulk of transduced EL4 cells were cloned, screen for their potential to secrete human PSA in their culture supernatants, determined by Human kallikrein 3/PSA DuoSet ELISA kit (Cat # DY1344, RD systems, biotechne). The EL4:hPSA 3E9 clone was selected, based on its capacity to secrete high amounts of human PSA. 3E9 cells were cultured in RPMI 1640 supplemented with 10 % FBS, penicillin (100 U/mL), and streptomycin (100 µg/mL) at 37°C in 5 % CO₂ and used at low (< 10) passage number.

### Transplantation and immunotherapy with Lenti-PROST-02

To evaluate the timeline of 3E9 tumor engraftment following Lenti-PROST-02 immunotherapy, mice were engrafted subcutaneously with 8 × 10⁵ 3E9 cells. Mice were immunized intramuscularly with the indicated doses of Lenti-PROST-02 in 50 µL. Tumors were measured 2 to 3 times a week using a digital caliper. The tumor volume was calculated using the formula V = L × W² /2, where V is the volume, L the length (the longest diameter), and W the width (the shortest diameter). Mice were killed when 3E9 tumors reached 1500 mm³ in volume. The 3E9 tumor did not become ulcerated and 3E9-engrafted mice were not moribund.

### T-cell assays

Fifteen-mer peptides spanning the whole hPAP or hPSA segments included in the designed PROST-02 antigen were synthesized by Genscript. Fifteen-mer peptides spanning the complete sequence of the hTROP2 antigen were synthesized by Proteogenix (Strasbourg, France). The peptides were used at final concentration of 1 µg/mL for ELISPOT or 2-5 µg/mL for intracellular cytokine staining (ICS). T-cell assays were performed in RPMI1640 medium supplemented with 10 % FCS, 100 U/mL penicillin, 100 mg/mL streptomycin, 1 × 10⁴ M non-essential amino-acids, 1 % vol/vol HEPES, 1 × 10³ M sodium pyruvate and 5 × 10⁵ M of β-mercapto-ethanol. IFN-γ ELISPOT and IL-2, TNFα and IFN-γICS assays were performed as detailed elsewhere (Ku et al. Cell Host Microbe 29:236-249 (2021) and Lopez et al. Cell Rep 40:111142 (2022)).

### Cytometric analysis of tumor immune infiltrates (Lenti-PROST-02)

3E9 tumors recovered from animals were digested during 30 minutes at 37°C by use of Collagenase and DNAseI, as previously described (Ku et al. Cell Host Microbe 29:236-249 (2021)) and were then homogenized in a GentleMacs system (Miltenyi). Tumor cell suspensions were filtered through 70 µm-pore filters, washed in PBS by centrifugation at 1200 rpm for 8 minutes. The cells were stained as follows.

To study T-cell and their activation status, cells were stained with a mixture of eF450-anti-CD4 (clone RM4-5, eBioscience), APC-anti-CD8 (clone 53-6.7, eBioscience), FITC-anti-CD44 (clone IM7, Biolegend), PerCP-eF710-anti-KLRG1 (clone 2F1, eBioscience) and PE-Cy7-anti-Tim3. The mixture contained FcγII/III receptor blocking anti-CD16/CD32 (clone 2.4G2, BD Biosciences) and Near IR Live/Dead (Invitrogen). Cells were incubated with the antibody mixture for 25 minutes at 4°C and washed in PBS + 3 % head-inactivated FBS and fixed with Cytofix (BD Biosciences).

Samples were acquired in an Attune NxT cytometer (Invitrogen) and the data were analyzed using FlowJo software (Treestar, OR, USA).

### qRT-PCR (Material and Methods)

The 3E9 tumor inflammatory mediators in Ctrl Lenti- or Lenti-PROST-02-treated mice were studied using the primer pairs listed in Table 1 (provided below), following a protocol recently detailed elsewhere (Ku et al., Cell Host Microbe 29(2) 236-249.e6 (2021)) using the comparative ΔΔ*C*_{T} method. Briefly, total RNA from tumors was prepared and genomic DNA was eliminated. The samples were stored at -80°C. The RNA quality was verified in a Bioanalyzer 2100 system (Agilent Technologies) and RNA samples were quantitated using a NanoDrop (Thermo Scientific NanoDrop). The RNA integrity number was 6.0-8.0. The mRNA abundance (*C*_{T} value) of each target genes was normalized to SDHA gene and compared to a calibrator value, corresponding to the mean of *C*_{T} values determined in the tumors from the Ctrl Lenti-treated mice. The fold change in gene expression was then calculated using 2^{-ΔΔ*C*}_{T}.

### Lenti-TROP2-1 and Lenti-TROP2-2 mouse immunization and immunogenicity studies

Six- to eight-week-old naive C57BL/6JRj female mice were vaccinated with 1 × 10⁷, 1 × 10⁸ or 1 × 10⁹ TU of Lenti-TROP2 or Ctrl Lenti via the intramuscular route. 14 days later, splenocyte suspensions were isolated from individual mice and restimulated for at least 18 hours with either 7 distinct hTROP2 peptide pools or individual 15-mer peptides, each at a final concentration of 2 µg/mL. Each peptide pool was composed of overlapping 15-mer peptides, with an 11 amino acid offset, corresponding to the full-length human TROP2.

### Statistical analyses

Unless indicated otherwise, statistical significance was determined using the two-tailed unpaired t test. Statistical significance of the percent survival was determined using the Log-rank Mantel-Cox test. All statistical analyses were performed using Prism GraphPad v9.

**Table 1. Primers used for the qRT-PCR experiments in Example 6**

| **Gene** | **cDNA accession number** | **SEQ ID NO Forward primer** | **SEQ ID NO Reverse primer** | **Amplicon (bp)** |
|---|---|---|---|---|
| IFNα | NM_010502.2 | SEQ ID NO: 40 | SEQ ID NO: 41 | 131 |
| IFNγ | NM_008337.4 | SEQ ID NO: 42 | SEQ ID NO: 43 | 92 |
| TNFα | NM_013693 | SEQ ID NO: 44 | SEQ ID NO: 45 | 175 |
| TGFβ | NM_011577.2 | SEQ ID NO: 46 | SEQ ID NO: 47 | 170 |
| IL-1β | NM_008361.4 | SEQ ID NO: 48 | SEQ ID NO: 49 | 220 |
| IL-2 | NM_008366.3 | SEQ ID NO: 50 | SEQ ID NO: 51 | 141 |
| IL-4 | NM_021283.2 | SEQ ID NO: 52 | SEQ ID NO: 53 | 154 |
| IL-5 | NM_010558.1 | SEQ ID NO: 54 | SEQ ID NO: 55 | 130 |
| IL-6 | NM_031168.2 | SEQ ID NO: 56 | SEQ ID NO: 57 | 116 |
| IL-10 | NM_010548.2 | SEQ ID NO: 58 | SEQ ID NO: 59 | 157 |
| IL-12p40 | NM_001303244.1 | SEQ ID NO: 60 | SEQ ID NO: 61 | 180 |
| IL-18 | NM_008360.2 | SEQ ID NO: 62 | SEQ ID NO: 63 | 159 |
| IL-33 | NM_001164724.2 | SEQ ID NO: 64 | SEQ ID NO: 65 | 136 |
| CCL2 | NM_011333.3 | SEQ ID NO: 66 | SEQ ID NO: 67 | 249 |
| CCL3 | NM_011337.2 | SEQ ID NO: 68 | SEQ ID NO: 69 | 163 |
| CCL4 | NM_013652.2 | SEQ ID NO: 70 | SEQ ID NO: 71 | 242 |
| CCL5 | NM_013653.3 | SEQ ID NO: 72 | SEQ ID NO: 73 | 185 |
| CCL19 | NM_011888.4 | SEQ ID NO: 74 | SEQ ID NO: 75 | 177 |
| CCL21 | NM_002989.4 | SEQ ID NO: 76 | SEQ ID NO: 77 | 136 |
| CXCL5 | NM_009141.3 | SEQ ID NO: 78 | SEQ ID NO: 79 | 242 |
| CXCL9 | NM_008599.4 | SEQ ID NO: 80 | SEQ ID NO: 81 | 207 |
| CXCL10 | NM_021274.2 | SEQ ID NO: 82 | SEQ ID NO: 83 | 150 |

### Example 1: Lenti-PROST-02 antigen and lentiviral vector design

To select the T-cell immunogenic regions of human PAP (Acc# P15309) and human PSA (Acc# P07288) proteins, presentable by molecules of Human Leukocyte Antigen of class-I (HLA-I), the inventors used SYFPEITHI (http://www.syfpeithi.de), IEDB (https://www.iedb.org), and netCTLpan (https://services.healthtech.dtu.dk/services/NetCTLpan-1.1/) epitope prediction tools. The additional advantage of NetCTLpan is to provide a combined prediction score by integrating: (i) strength of peptide-MHC-I binding, (ii) efficacy of proteasomal C-terminal cleavage, and (iii) efficacy of peptide transport by TAP (transporter associated with antigen processing). Initially, 9 amino acid (aa)-long peptides that could be presented by 6 common HLA alleles, i.e. HLA-A*01:01, HLA-A*02:01, HLA-A*03:01, HLA-A*11:01, HLA-A*24:02, HLA-B*35:01, were searched by combining the results generated by the three prediction tools. To narrow down the list of potential epitopes, only those with the prediction score >21 in SYFPEITHI, < 1 in IEDB and netCTLpan were selected. Both predicted and already identified epitopes were mapped to the reference sequences of human PSA and PAP proteins.

To further extend the possibility of presentation of epitopes predicted in global human population, the above-mentioned IEDB predictions were repeated for 9-10 aa-long epitopes to 27 HLA-I alleles that are shared by 97 % of human population. The vast majority of the resulted predicted epitopes was mapped to the immunogenic regions identified above, thus validating the choice of potentially immunogenic regions. All selected regions flanked by three native aa residues from each side were assembled together, creating a single poly-epitopic hPAP-hPSA fusion sequence, called PROST-02 (SEQ ID NO: 3), without generating predictable irrelevant junctional epitopes. No significant homology was found for the PROST-02 clustered antigenic sequences versus complete human protein database (https://www.uniprot.org), as scanned by Blast (https://blast.ncbi.nlm.nih.gov/Blast.cgi), which is reassuring with regard to the risk of non-specific autoimmune responses in case of the use of these sequences in prospective immuno-oncotherapies.

The inventors further designed a non-integrative lentiviral vector, named "Lenti-PROST-02", to encode the PROST-02 fusion antigen, under the transcriptional control of human β2 microglobulin promoter (Ku et al. Commun Biol 4:713 (2021)) (Figure 1). The human β2 microglobulin promoter contains conserved cis-regulatory elements: (i) interferon (IFN)-stimulated response elements (ISREs) that are the binding site for the IFN regulatory factors, and (ii) SXY modules which interact with a multiprotein complex. These elements transactivate and are regulated by pro-inflammatory cytokines, which are upregulated in a spatiotemporal manner in antigen presenting cells and especially DCs (Gobin et al. Blood 101:3058-3064 (2003) and van den Elsen et al. Front Immunol 2:48 (2011)).

Recombinant lentiviral vector particles, pseudotyped with the envelope glycoprotein of vesicular stomatitis virus (VSV-G) from Indiana serotype, were successfully produced in the human embryonic kidney HEK 293 T cell production system and concentrated by ultracentrifugation with titers ranging from 1 to 20 × 10¹⁰ TU/mL.

### Example 2: T-cell immunogenicity of Lenti-PROST-02

The T-cell immunogenicity of Lenti-PROST-02 was first evaluated *in vivo* by immunizing male C57BL/6 (H-2^{b}) mice (n = 5 mice per group) via the intramuscular route at day 0 with 1 × 10⁹ TU of Lenti-PROST-02 or an empty lentiviral vector (Ctrl Lenti). On day 14, splenocytes from individual mice were used in IFN-γ ELISPOT after *in vitro* stimulation with pools of 15-mer peptides spanning the complete sequences of the hPAP-hPSA PROST-02 fusion antigen.

Splenocytes from the Ctrl Lenti-injected mice did not respond to the peptide stimulation, while the splenocytes from the Lenti-PROST-02-immunized mice were responsive and allowed identification of one hPAP-derived and three hPSA-derived positive peptide pools (Figure 2A, top). Subsequent deconvolution of the latter allowed precise epitope mapping of the hPAP-hPSA PROST-02 antigen (Figure 2A, bottom). ICS of splenocytes from Lenti-PROST-02-immunized mice showed that the responding splenocytes were CD8⁺ T cells, containing a majority of IFNγ⁺ TNFα⁺ cells (Figure 2B).

Therefore, these results established that Lenti-PROST-02 immunization induced specific responses against numerous hPAP and/or hPSA T-cell epitopes and that the principal immune effector cells induced by Lenti-PROST-02 immunization were CD8⁺ T cells able to produce simultaneously IFNγ and TNFα.

### Example 3: Lenti-PROST-02-based immunotherapy against a preclinical prostate cancer model

Intramuscular immunization of C57BL/6 mice with escalating doses of Lenti-PROST-02 showed that the induced anti-hPAP and anti-hPSA T-cell immunity was dose dependent and that the highest dose of 1 × 10⁹ TU/mouse generated the strongest responses (Figure 3A), which is in accordance with previous findings in other preclinical murine models (Douguet et al. EMBO Mol Med 15(10):e17723 (2023), Fert et al. NPJ Vaccines 9:102 (2024), Ku et al. EMBO Mol Med 13:e14459 (2021), Ku et al. Cell Host Microbe 29:236-249 (2021), Lopez et al. Cell Rep 40:111142 (2022), Nemirov et al. Front Immunol 14:1208041 (2023), Somaiah et al. Clin Cancer Res 25:5808-5817 (2019), Tada et al. JCI Insight 7:e161598 (2022), Vesin et al. Mol Ther 30:2984-2997 (2022)).

To evaluate the anti-tumor effect of Lenti-PROST-02 immunotherapy, the inventors set up a tumor model based on the C57BL/6 syngeneic EL4 cell line (H-2^{b}), transduced to stably produce the full-length human PSA. The "3E9" clone was selected among others for its highest potential in hPSA secretion in the culture supernatant, and surface H-2K^{b} and H-2D^{b} expression.

The anti-tumor efficacy of Lenti-PROST-02 was assessed in C57BL/6 male mice subcutaneously engrafted with 8 × 10⁵ 3E9 tumor cells *(n* = 7 mice per group). Six days post tumor inoculation, when the 3E9 tumors are well established, mice were randomized and treated by a single intramuscular injection of 1 × 10⁸ or 1 × 10⁹ TU immunogenic doses of Lenti-PROST-02. Tumors-bearing control mice received 1 × 10⁹ TU of Ctrl Lenti. In all Ctrl Lenti-treated mice, tumor volume reached 1500 mm³, defined as humane endpoint, before day 30 (Figure 3B). In net opposite, an anti-tumor effect was detected in Lenti-PROST-02-treated mice in a dose dependent manner (Figure 3B). Notably, the high dose of 1 × 10⁹ TU of Lenti-PROST-02 led to a complete tumor eradication in 7 out of 8 mice. Full tumor eradication was also observed in 3 out of 8 mice with the dose of 1 × 10⁸ TU of Lenti-PROST-02 (Figure 3B). The survival of the Lenti-PROST-02-immunized mice was significantly prolonged with the dose of 1 × 10⁹ TU of Lenti-PROST-02 (Figure 3C). The active secretion of hPSA by 3E9 tumors resulted *in vivo* in the presence of this antigen in the bloodstream of tumor-bearing mice, as measured on day 21 post-tumor transplantation (Figure 3D). Presence of hPSA in the blood was determined by ELISA (Human kallikrein 3/PSA DuoSet ELISA kit). hPSA was not measurable in the bloodstream of the Lenti-PROST-02-treated mice that recovered from their 3E9 tumors. Notably, in the Lenti-PROST-02-treated group, the only hPSA-positive mouse was the one whose tumor had not been eradicated (Figure 3D). Therefore, similar to physiological prostate cancers, PSA blood levels serve as a reliable biomarker in this preclinical model, accurately reflecting the status of the prostate tumor.

These results clearly demonstrated the substantial efficacy of the therapeutic Lenti-PROST-02 vaccine according to the invention, eliciting a robust anti-tumor response in the immunotherapy of the preclinical hPSA-expressing virulent tumor model established by the inventors.

### Example 4: Impact of Lenti-PROST-02-treatment on intra-tumoral immune cells

To get more insights on the Lenti-PROST-02 mode of action, the inventors compared the 3E9 tumors from mice treated with Lenti Ctrl or Lenti-PROST-02. Briefly, C57BL/6 mice were engrafted subcutaneously with 8 × 10⁵ 3E9 cells on day 0 and treated with 1 × 10⁹ TU of Lenti Ctrl or Lenti-PROST-02 on day 6. Tumors were studied on day 15, i.e. day 9 days post-vaccination.

As soon as day 9 after a single intramuscular administration of 1 × 10⁹ TU of Lenti-PROST-02, when the tumor shrinking began, the proportions of CD8⁺ - but not CD4⁺ - TILs displayed a net tendency to increase, compared to those in the growing tumors of Ctrl Lenti-treated mice (Figure 4A). In the shrinking tumors of Lenti-PROST-02-treated mice, the CD8⁺ T subset contained significantly higher proportions of CD44⁺ KLRG1 (killer-cell lectin like receptor)⁺ cells and PD1⁺ TIM3 (T cell immunoglobulin domain and mucin domain)⁺ cells compared to the CD8⁺ T subset in the growing tumors of Ctrl Lenti-treated mice (Figure 4A and Figure 4B). This predominant activated/terminally differentiated CD44⁺ KLRG1⁺ and PD1⁺ TIM3⁺ profile of CD8⁺ T cells in the shrinking tumors seemingly resulted from sustained anti-tumor effector activity.

On day 9 post Lenti-PROST-02 treatment, the mice tumor size (Figure 4D) was inversely proportional to the frequency of IFNγ⁺ TNFα⁺ CD8⁺ T splenocytes specific of immunogenic regions of the PROST-02 fusion antigen (Figure 4C). Principal-component analysis (PCA) applied to the proportions of activated/differentiated TILs, T-splenocyte responses to Lenti-PROST-02 vaccine and tumor size on day 9 post vaccination showed two distinct experimental groups, corresponding to the Ctrl Lenti or the Lenti-PROST-02 treatments (Figure 4E).

Therefore, these results demonstrated that Lenti-PROST-02 induced high-quality T-cell responses against hPAP and hPSA prostate-specific antigens as soon as day 9 post vaccination, leading to tumor shrinkage. This early anti-tumor effect was notably accompanied by increased proportions of CD8⁺ TILs with activated-effector phenotype.

### Example 5: Long-term protective immune memory induced by Lenti-PROST-02 therapy

The inventors determined whether the tumor-cured mice developed a long-term protective immune memory. After initial tumor engraftment with 8 × 10⁵ 3E9 cells *(n* = 12 mice per group), C57BL/6 mice received a single intramuscular injection of 1 × 10⁹ TU of Lenti-PROST-02. To mimic tumor relapse, cured mice (n = 12) were re-challenged subcutaneously with 8 × 10⁵ of 3E9 cells in the opposite flank at day 68 after the first tumor engraftment, and maintained without any additional treatment. A group of naive mice received the same 3E9 tumor cell suspension, as a positive control of tumor growth (n = 6). All naive control mice developed tumors, while all initially cured mice were protected from the tumor re-challenge (Figure 5).

Therefore, these results established that the Lenti-PROST-02 therapy induced long-term protective immune memory against prostate-specific antigens, in a preclinical murine model of prostate tumor with immunosuppressive characteristics.

### Example 6: Inflammatory status of tumors in Lenti-PROST-02-treated mice

On day 9 post Lenti-PROST-02 administration, the inflammatory status of the 3E9 tumors in Ctrl Lenti- or Lenti-PROST-02-treated mice was analyzed by qRT-PCR on RNA extracted from the total tumors.

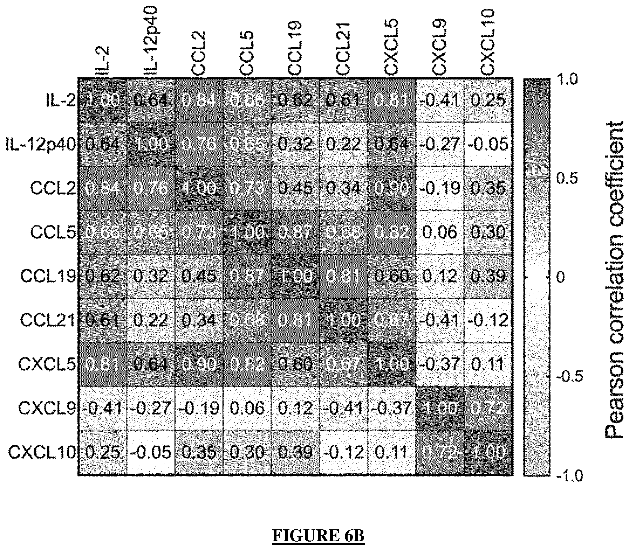

In Lenti-PROST-02-treated mice, the tumors globally displayed a more inflammatory profile than in Ctrl Lenti-treated mice (Figure 6A). The expression of IL-2 and IL-12p40 was higher in the tumors of Lenti-PROST-02-treated mice (Figure 6A), and in a correlative manner (Figure 6B). The expression of, among others, CCL2 and CCL5 chemokines, as well as CCL19 and CCL21 chemokines, was also increased (Figure 6A) in a correlative manner (Figure 6B) in the tumors of Lenti-PROST-02 treated mice.

Therefore, a single intramuscular administration of Lenti-PROST-02 resulted in a "cold-to-hot" inflammatory switch of the PSA-expressing tumor microenvironment, in correlation with increased proportions of activated/differentiated CD8⁺ TILs and tumor regression. This inflammatory switch can also notably contribute to MHC-I upregulation on the tumor cells, rendering them better targets of effector CD8⁺ T cells.

### Example 7: T-cell immunogenicity of Lenti-TROP2-1 and Lenti-TROP2-2 in mice

The inventors designed two distinct non-integrative lentiviral vector-based vaccine candidates encoding: (i) the full-length human TROP-2 (Lenti-TROP2-1) and (ii) the full-length human TROP-2 deleted from the signal peptide (1-26 amino acid segment) and the transmembrane domain (275-297 amino acid segment) (Lenti-TROP2-2). In these two lentiviral vectors, the antigens are under the transcriptional control of the human β-2-microglobulin promoter (Ku et al., Communications biology, 4(1):713 (2021)).

The T-cell immunogenicity of the Lenti-TROP2 vaccine candidates described above was evaluated *in vivo* by immunizing C57BL/6 mice via intramuscular route on day 0 with 1 × 10⁹ TU of a control (Ctrl) Lenti, of Lenti-TROP2-1 or of Lenti-TROP2-2. On day 14, splenocytes from individual mice were used in IFN-γ ELISPOT after *in vitro* stimulation with pooled peptides derived from the full-length sequence of human TROP2.

Lenti-TROP2-1 and Lenti-TROP2-2 displayed a strong capacity to induce IFNγ-producing T-cell immunity against at least four of the tested peptide pools (Figure 7A). Lenti-TROP2-1 induced an additional T-cell immunity against the first hTROP2 peptide pool (Pool #1-10) (Figure 7A). To further epitope-map the hTROP2 antigen, the inventors analyzed T-cell immunity after *in vitro* stimulation with individual peptides from the positive peptide pools. The inventors found the same 9 positive 15-mer peptides in C57BL/6 mice immunized with Lenti-TROP2-1 or Lenti-TROP2-2. In accordance with the results shown in Figure 7A, vaccination with Lenti-TROP2-1 induced T-cell responses against 3 additional 15-mer peptides (Figure 7B).

Next, to determine the effective dose range of the Lenti-TROP2-2 vaccine candidate, the inventors assessed T-cell responses following stimulation with 11 of the positive 15-mer peptides identified above. Groups of four mice each received intramuscular injections of escalating doses of the vectors, i.e. 1 × 10⁷, 1 × 10⁸ or 1 × 10⁹ TU. IFNγ-producing T-cell responses were observed starting at 1 × 10⁸ TU, with no further increase in response at 1 × 10⁹ TU, indicating that the optimal effective dose for Lenti-TROP2-2 in C57BL/6 mice is 1 × 10⁸ TU (Figure 8).

Therefore, these results demonstrated that both Lenti-TROP2-1 and Lenti-TROP2-2 therapy induced specific T-cell immunity against numerous hTROP2 immunogenic regions, and that immune effector cells induced by Lenti-TROP2-1 and Lenti-TROP2-2 immunization were IFNγ-producing T cells. Altogether, these results demonstrated the preclinical proof of concept of the T-cell immunogenicity of the developed lentiviral vectors Lenti-TROP2-1 and Lenti-TROP2-2.

### SEQUENCES

SEQ ID NO: 1: amino acid sequence of the hPAP portion of the PROST-02 antigen sequence 
MRAAPLLLARAASLSLGFLFLLFFWLDRSVLAKELKFVTLVFRHGHYELGEYIRKR
YRKFLNESYKHEQVYIRSTDVDRTLMSAMTNLAALFPPEGVSIWNPILLWQPIPVH
TVPLSEDQLLYLPFRNCPRFQELRLHPYKDFIATLGKLSGLHGQDLFGIWSKVYDP
LYCESVHNFTLPSWATEDTMTKLRELSELSLLSLYGIHKQKEKSHMKRATQIPSYK
KLIMYSAHDTTVSGLQMALDVYNGLLPPYASCHLTELYFEKGEYFVEMYYRNET
QHEPYPLM
SEQ ID NO: 2: amino acid sequence of the hPSA portion of the PROST-02 antigen sequence 
WVPVVFLTLSVTWIGAAPLILSRIVGGWECEKHSQRGRAVCGGVLVHPQWVLTA
AHCIRNKSVILLGRHSLFHPEDTGQVFQVSHSFPHPLYDMSLLKNRFLRPGDDSSH
DLMLLRLGPDDMDLPTQEPALGTTCYASGWGSIEPEEFLTPKKLQCVDLHVISND
VCAQVHPQKVTKFMLCAGRWTGGSEPCALPERPSLYTKVVHYRKWIKD
SEQ ID NO: 3: amino acid sequence of the PROST-02 antigen sequence 
MRAAPLLLARAASLSLGFLFLLFFWLDRSVLAKELKFVTLVFRHGHYELGEYIRKR
YRKFLNESYKHEQVYIRSTDVDRTLMSAMTNLAALFPPEGVSIWNPILLWQPIPVH
TVPLSEDQLLYLPFRNCPRFQELRLHPYKDFIATLGKLSGLHGQDLFGIWSKVYDP
LYCESVHNFTLPSWATEDTMTKLRELSELSLLSLYGIHKQKEKSHMKRATQIPSYK
KLIMYSAHDTTVSGLQMALDVYNGLLPPYASCHLTELYFEKGEYFVEMYYRNET
QHEPYPLMLPGPDDWVPVVFLTLSVTWIGAAPLILSRIVGGWECEKHSQRGRAVC
GGVLVHPQWVLTAAHCIRNKSVILLGRHSLFHPEDTGQVFQVSHSFPHPLYDMSL
LKNRFLRPGDDSSHDLMLLRLGPDDMDLPTQEPALGTTCYASGWGSIEPEEFLTPK
KLQCVDLHVISNDVCAQVHPQKVTKFMLCAGRWTGGSEPCALPERPSLYTKVVH
YRKWIKD
SEQ ID NO: 4: amino acid sequence of the hTROP2-1 antigen sequence 
MARGPGLAPPPLRLPLLLLVLAAVTGHTAAQDNCTCPTNKMTVCSPDGPGGRCQ
CRALGSGMAVDCSTLTSKCLLLKARMSAPKNARTLVRPSEHALVDNDGLYDPDC
DPEGRFKARQCNQTSVCWCVNSVGVRRTDKGDLSLRCDELVRTHHILIDLRHRPT
AGAFNHSDLDAELRRLFRERYRLHPKFVAAVHYEQPTIQIELRQNTSQKAAGDVDI
GDAAYYFERDIKGESLFQGRGGLDLRVRGEPLQVERTLIYYLDEIPPKFSMKRLTA
GLIAVIVVVVVALVAGMAVLVITNRRKSGKYKKVEIKELGELRKEPSL
SEQ ID NO: 5: amino acid sequence of the hTROP2-2 antigen sequence 
MHTAAQDNCTCPTNKMTVCSPDGPGGRCQCRALGSGMAVDCSTLTSKCLLLKAR
MSAPKNARTLVRPSEHALVDNDGLYDPDCDPEGRFKARQCNQTSVCWCVNSVG
VRRTDKGDLSLRCDELVRTHHILIDLRHRPTAGAFNHSDLDAELRRLFRERYRLHP
KFVAAVHYEQPTIQIELRQNTSQKAAGDVDIGDAAYYFERDIKGESLFQGRGGLDL
RVRGEPLQVERTLIYYLDEIPPKFSMKRLTTNRRKSGKYKKVEIKELGELRKEPSL
SEQ ID NO: 6: amino acid sequence of the hTROP2-3 antigen sequence 
MAQIFVKTLTGKTITLEVEPSDTIENVKAKIQDKEGIPPDQQRLIFAGKQLEDGRTL
SDYNIQKESTLHLVLRLRGVAARSHTAAQDNCTCPTNKMTVCSPDGPGGRCQCR
ALGSGMAVDCSTLTSKCLLLKARMSAPKNARTLVRPSEHALVDNDGLYDPDCDP
 
EGRFKARQCNQTSVCWCVNSVGVRRTDKGDLSLRCDELVRTHHILIDLRHRPTAG
AFNHSDLDAELRRLFRERYRLHPKFVAAVHYEQPTIQIELRQNTSQKAAGDVDIGD
AAYYFERDIKGESLFQGRGGLDLRVRGEPLQVERTLIYYLDEIPPKFSMKRLTTNR
RKSGKYKKVEIKELGELRKEPSL
SEQ ID NO: 7: amino acid sequence of the hTROP2-4 antigen sequence 
MHTAAQDNCTCPTNKMTVCSPDGPGGRCQCRALGSGMAVDCSTLTSKCLLLKAR
MSAPKNARTLVRPSEHALVDNDGLYDPDCDPEGRFKARQCNQTSVCWCVNSVG
VRRTDKGDLSLRCDELVRTHHILIDLRHRPTAGAFNHSDLDAELRRLFRERYRLHP
KFVAAVHYEQPTIQIELRQNTSQKAAGDVDIGDAAYYFERDIKGESLFQGRGGLDL
RVRGEPLQVERTLIYYLDEIPPKFSMKRLTTNRRKGKYKKVEIKELGELRKEPL
SEQ ID NO: 8: amino acid sequence of the hTROP2-5 antigen sequence 
MHTAAQDNCTCPTNKMTVCSPDGPGGRCQCRALGSGMAVDCSTLTSKCLLLKAR
MSAPKNARTLVRPSEHALVDNDGLYDPDCDPEGRFKARQCNQTSVCWCVNSVG
VRRTDKGDLSLRCDELVRTHHILIDLRHRPTAGAFNHSDLDAELRRLFRERYRLHP
KFVAAVHYEQPTIQIELRQNTSQKAAGDVDIGDAAYYFERDIKGESLFQGRGGLDL
RVRGEPLQVERTLIYYLDEIPPKFSMKRLT
SEQ ID NO: 9: amino acid sequence of the hTARP-1 antigen sequence 
QMFLPSPLFFFLQLLKQSSRRLEHTFVFLANFSLMLLRYIGKKRRATRFWDPRRGT
P
SEQ ID NO: 10: amino acid sequence of the hTARP-2 antigen sequence 
QMFLPSPLFFFLQLLKQSSRRLEHTFVFLRNFSLMVLRYIGKKRRATRFWDPRRGT
P
SEQ ID NO: 11: amino acid sequence of a possible linker sequence
   LPGPDD
SEQ ID NO: 12: amino acid sequence of segment 6-80 of the mutated human ubiquitin C^{G77V} 
MAQIFVKTLTGKTITLEVEPSDTIENVKAKIQDKEGIPPDQQRLIFAGKQLEDGRTL
SDYNIQKESTLHLVLRLRGV
SEQ ID NO: 13: amino acid sequence of the immunological tag
   SIINFEKL
SEQ ID NO: 14: amino acid sequence of AAAA linker
   AAAA
SEQ ID NO: 15: amino acid sequence of the full hTARP-1 construction 
MAQIFVKTLTGKTITLEVEPSDTIENVKAKIQDKEGIPPDQQRLIFAGKQLEDGRTL
SDYNIQKESTLHLVLRLRGVAAAAQMFPPSLLFFFLQLLKQSSRRLEHTFVFLANFS
LMLLRYIGKKRRATRFWDPRRGTPAAAASIINFEKL
SEQ ID NO: 16: amino acid sequence of the full hTARP-2 construction 
MAQIFVKTLTGKTITLEVEPSDTIENVKAKIQDKEGIPPDQQRLIFAGKQLEDGRTL
SDYNIQKESTLHLVLRLRGVAAAAQMFPPSLLFFFLQLLKQSSRRLEHTFVFLRNFS
LMVLRYIGKKRRATRFWDPRRGTPAAAASIINFEKL
SEQ ID NO: 17: nucleic acid sequence of the hPAP portion of the PROST-02 antigen sequence 
atgagggccgcccctttgttgttggctagggccgcctccctgtccctgggcttcctgttcctgctgttcttctggctggaccggtccgt
gttggccaaggagctgaagttcgtgaccctggtgtttcggcatggccactacgagttgggagaatatatccgcaagaggtaccgca
agttcctgaacgagtcttacaagcacgagcaggtttacatcaggtccaccgacgtggacaggactctcatgtccgctatgaccaac
ctggccgcactgttcccccccgaaggcgtgtccatctggaaccccatcctcctgtggcagcccatccccgtgcacaccgtgcccct
gagcgaggaccagctgctgtacctgcccttccgcaattgcccccgtttccaggagctgagactccacccctacaaggacttcattg
ccaccctcggcaagttgtccggcctgcacggccaggatttgttcggcatttggtccaaagtctatgaccccctctactgcgaatccgt
gcacaacttcaccctcccctcctgggccaccgaagacaccatgaccaagctgagggagctgtccgagctgagtttgctgtccctgt
acggcatccacaaacagaaggagaaatcccatatgaaaagggccacccagatcccctcctacaagaaactgatcatgtactccgc
ccacgacaccacagtctccggcctccagatggccctcgacgtctacaacggactgctgcccccctacgcctcctgtcacctcacc
gaactctacttcgaaaagggcgaatacttcgtggaaatgtactacaggaacgaaacccagcacgaaccctatcccctcatg
SEQ ID NO: 18: nucleic acid sequence of the hPSA portion of the PROST-02 antigen sequence 
tgggtgcctgttgtcttcctgactttgtccgtgacctggatcggcgctgcccctctgatcttgtcccggattgtgggcggctgggagtg
cgaaaaacactcccagaggggccgggctgtgtgtggcggtgtgctggtgcatcctcagtgggtgctgactgctgctcattgcatta
ggaacaagagtgtgatcttgctggggaggcactccttgttccaccccgaggatactggtcaggtgtttcaggtgtcccattccttccc
ccaccccctctacgacatgtccctgctgaagaacagattcttgcgccccggcgacgactcctcccacgacttgatgctgctgcggc
tcggccccgacgatatggacctccccacccaggagcccgccctgggaacaacctgctacgcctccggctggggctccatcgag
cccgaagagttcctcacccccaagaaactgcagtgcgttgacctgcacgtgatttccaacgacgtgtgcgcccaggtgcaccccc
agaaggtgaccaagttcatgctgtgcgccggcaggtggacaggcggctctgaaccctgcgccctccccgaaagaccctccctgt
acaccaaagtggtgcactataggaagtggatcaaggac
SEQ ID NO: 19: nucleic acid sequence of the PROST-02 antigen sequence 
atgagggccgcccctttgttgttggctagggccgcctccctgtccctgggcttcctgttcctgctgttcttctggctggaccggtccgt
gttggccaaggagctgaagttcgtgaccctggtgtttcggcatggccactacgagttgggagaatatatccgcaagaggtaccgca
agttcctgaacgagtcttacaagcacgagcaggtttacatcaggtccaccgacgtggacaggactctcatgtccgctatgaccaac
ctggccgcactgttcccccccgaaggcgtgtccatctggaaccccatcctcctgtggcagcccatccccgtgcacaccgtgcccct
gagcgaggaccagctgctgtacctgcccttccgcaattgcccccgtttccaggagctgagactccacccctacaaggacttcattg
ccaccctcggcaagttgtccggcctgcacggccaggatttgttcggcatttggtccaaagtctatgaccccctctactgcgaatccgt
gcacaacttcaccctcccctcctgggccaccgaagacaccatgaccaagctgagggagctgtccgagctgagtttgctgtccctgt
acggcatccacaaacagaaggagaaatcccatatgaaaagggccacccagatcccctcctacaagaaactgatcatgtactccgc
ccacgacaccacagtctccggcctccagatggccctcgacgtctacaacggactgctgcccccctacgcctcctgtcacctcacc
gaactctacttcgaaaagggcgaatacttcgtggaaatgtactacaggaacgaaacccagcacgaaccctatcccctcatgctccc
cggccccgacgactgggtgcctgttgtcttcctgactttgtccgtgacctggatcggcgctgcccctctgatcttgtcccggattgtg
ggcggctgggagtgcgaaaaacactcccagaggggccgggctgtgtgtggcggtgtgctggtgcatcctcagtgggtgctgact
gctgctcattgcattaggaacaagagtgtgatcttgctggggaggcactccttgttccaccccgaggatactggtcaggtgtttcagg
tgtcccattccttcccccaccccctctacgacatgtccctgctgaagaacagattcttgcgccccggcgacgactcctcccacgactt
gatgctgctgcggctcggccccgacgatatggacctccccacccaggagcccgccctgggaacaacctgctacgcctccggct
ggggctccatcgagcccgaagagttcctcacccccaagaaactgcagtgcgttgacctgcacgtgatttccaacgacgtgtgcgc
ccaggtgcacccccagaaggtgaccaagttcatgctgtgcgccggcaggtggacaggcggctctgaaccctgcgccctccccg
aaagaccctccctgtacaccaaagtggtgcactataggaagtggatcaaggactgataa
SEQ ID NO: 20: nucleic acid sequence of the hTROP2-1 antigen sequence 
atggctaggggacctgggctggctcctcctctgcgactgcctctgctgctgctggtgctggctgctgtgactggacataccgct
gcccaggataactgcacctgtcccacaaataagatgaccgtgtgcagcccagacggaccaggaggcagatgccagtgtagggc
cctgggctccggaatggcagtggattgcagcaccctgacatccaagtgtctgctgctgaaggccagaatgtccgccccaaagaac
gcacgcacactggtgcggccttctgagcacgccctggtggacaatgatggcctgtacgaccctgattgcgacccagagggcaga
ttcaaggccaggcagtgtaaccagaccagcgtgtgctggtgcgtgaatagcgtgggcgtgcggagaacagataagggcgacct
gtccctgcgctgtgatgagctggtgcggacccaccacatcctgatcgacctgaggcacaggcctacagcaggagcctttaaccac
tctgatctggacgcagagctgaggcgcctgttccgggagagatacaggctgcacccaaagtttgtggccgccgtgcactatgagc
agcccaccatccagatcgagctgaggcagaatacatctcagaaggcagcaggcgatgtggacatcggcgatgccgcctactattt
cgagagagacatcaagggagagagcctgtttcagggaaggggaggcctggatctgcgcgtgcggggagagccactgcaggtg
gagcgcaccctgatctactatctggacgagatcccccctaagttcagcatgaagcggctgacagccggcctgatcgccgtgatcg
tggtggtggtggtggccctggtggcaggcatggccgtgctggtcatcaccaaccggagaaagtccggcaaatacaaaaaggtgg
aaatcaaagaactgggcgaactgcgaaaggaaccaagtctgtga
SEQ ID NO: 21: nucleic acid sequence of the hTROP2-2 antigen sequence 
atgcataccgctgcccaggataactgcacctgtcccacaaataagatgaccgtgtgcagcccagacggaccaggaggcagatgc
cagtgtagggccctgggctccggaatggcagtggattgcagcaccctgacatccaagtgtctgctgaaggccagaatgtccg
ccccaaagaacgcacgcacactggtgcggccttctgagcacgccctggtggacaatgatggcctgtacgaccctgattgcgacc
cagagggcagattcaaggccaggcagtgtaaccagaccagcgtgtgctggtgcgtgaatagcgtgggcgtgcggagaacagat
aagggcgacctgtccctgcgctgtgatgagctggtgcggacccaccacatcctgatcgacctgaggcacaggcctacagcagga
gcctttaaccactctgatctggacgcagagctgaggcgcctgttccgggagagatacaggctgcacccaaagtttgtggccgccg
tgcactatgagcagcccaccatccagatcgagctgaggcagaatacatctcagaaggcagcaggcgatgtggacatcggcgatg
ccgcctactatttcgagagagacatcaagggagagagcctgtttcagggaaggggaggcctggatctgcgcgtgcggggagag
ccactgcaggtggagcgcaccctgatctactatctggacgagatcccccctaagttcagcatgaagcggctgacaaccaaccgga
gaaagtccggcaaatacaaaaaggtggaaatcaaagaactgggcgaactgcgaaaggaaccaagtctgtga
SEQ ID NO: 22: nucleic acid sequence of the hTROP2-3 antigen sequence 
atggcccagatcttcgtgaagaccctgactggtaagaccatcactctcgaagtggagccgagtgacaccattgagaatgtcaagg
caaagatccaagacaaggaaggcatccctcctgaccagcagaggttgatctttgctgggaaacagctggaagatggacgcaccc
tgtctgactacaacatccagaaagagtccaccctgcacctggtcctccgtctcagaggtgtggcagcccgatcccataccgctgcc
caggataactgcacctgtcccacaaataagatgaccgtgtgcagcccagacggaccaggaggcagatgccagtgtagggccct
gggctccggaatggcagtggattgcagcaccctgacatccaagtgtctgctgctgaaggccagaatgtccgccccaaagaacgc
acgcacactggtgcggccttctgagcacgccctggtggacaatgatggcctgtacgaccctgattgcgacccagagggcagattc
aaggccaggcagtgtaaccagaccagcgtgtgctggtgcgtgaatagcgtgggcgtgcggagaacagataagggcgacctgtc
cctgcgctgtgatgagctggtgcggacccaccacatcctgatcgacctgaggcacaggcctacagcaggagcctttaaccactct
gatctggacgcagagctgaggcgcctgttccgggagagatacaggctgcacccaaagtttgtggccgccgtgcactatgagcag
cccaccatccagatcgagctgaggcagaatacatctcagaaggcagcaggcgatgtggacatcggcgatgccgcctactatttcg
agagagacatcaagggagagagcctgtttcagggaaggggaggcctggatctgcgcgtgcggggagagccactgcaggtgga
gcgcaccctgatctactatctggacgagatcccccctaagttcagcatgaagcggctgacaaccaaccggagaaagtccggcaa
atacaaaaaggtggaaatcaaagaactgggcgaactgcgaaaggaaccaagtctgtga
SEQ ID NO: 23: nucleic acid sequence of the hTROP2-4 antigen sequence 
atgcataccgctgcccaggataactgcacctgtcccacaaataagatgaccgtgtgcagcccagacggaccaggaggcagatgc
cagtgtagggccctgggctccggaatggcagtggattgcagcaccctgacatccaagtgtctgctgaaggccagaatgtccg
ccccaaagaacgcacgcacactggtgcggccttctgagcacgccctggtggacaatgatggcctgtacgaccctgattgcgacc
cagagggcagattcaaggccaggcagtgtaaccagaccagcgtgtgctggtgcgtgaatagcgtgggcgtgcggagaacagat
aagggcgacctgtccctgcgctgtgatgagctggtgcggacccaccacatcctgatcgacctgaggcacaggcctacagcagga
gcctttaaccactctgatctggacgcagagctgaggcgcctgttccgggagagatacaggctgcacccaaagtttgtggccgccg
 
tgcactatgagcagcccaccatccagatcgagctgaggcagaatacatctcagaaggcagcaggcgatgtggacatcggcgatg
ccgcctactatttcgagagagacatcaagggagagagcctgtttcagggaaggggaggcctggatctgcgcgtgcggggagag
ccactgcaggtggagcgcaccctgatctactatctggacgagatcccccctaagttcagcatgaagcggctgacaaccaaccgga
gaaagggcaaatacaaaaaggtggaaatcaaagaactgggcgaactgcgaaaggaaccactgtga
SEQ ID NO: 24: nucleic acid sequence of the hTROP2-5 antigen sequence 
atgcataccgctgcccaggataactgcacctgtcccacaaataagatgaccgtgtgcagcccagacggaccaggaggcagatgc
cagtgtagggccctgggctccggaatggcagtggattgcagcaccctgacatccaagtgtctgctgctgaaggccagaatgtccg
ccccaaagaacgcacgcacactggtgcggccttctgagcacgccctggtggacaatgatggcctgtacgaccctgattgcgacc
cagagggcagattcaaggccaggcagtgtaaccagaccagcgtgtgctggtgcgtgaatagcgtgggcgtgcggagaacagat
aagggcgacctgtccctgcgctgtgatgagctggtgcggacccaccacatcctgatcgacctgaggcacaggcctacagcagga
gcctttaaccactctgatctggacgcagagctgaggcgcctgttccgggagagatacaggctgcacccaaagtttgtggccgccg
tgcactatgagcagcccaccatccagatcgagctgaggcagaatacatctcagaaggcagcaggcgatgtggacatcggcgatg
ccgcctactatttcgagagagacatcaagggagagagcctgtttcagggaaggggaggcctggatctgcgcgtgcggggagag
ccactgcaggtggagcgcaccctgatctactatctggacgagatcccccctaagttcagcatgaagcggctgacatga
SEQ ID NO: 25: nucleic acid sequence of the hTARP-1 antigen sequence 
caaatgtttctgccctcccccctgttttttttcctccagttgcttaagcagtccagccgcagactggagcacacattcgtgttcctggca
aacttctcactcatgctgctgcgctacataggcaagaagaggagagccaccagattttgggacccaagacggggaacccca
SEQ ID NO: 26: nucleic acid sequence of the hTARP-2 antigen sequence 
caaatgtttctgccctcccccctgttttttttcctccagttgcttaagcagtccagccgcagactggagcacacattcgtgttcctgagg
aacttctcactcatggtgctgcgctacataggcaagaagaggagagccaccagattttgggacccaagacggggaacccca
SEQ ID NO: 27: nucleic acid sequence of the LPGPDD linker sequence
   ctccccggccccgacgac
SEQ ID NO: 28: nucleic acid sequence of segment 6-80 of the mutated human ubiquitin C^{G77V} 
atggcccagatcttcgtgaaaaccctgaccggcaagaccatcacactggaagtggaacccagcgacaccatcgagaacgtgaa
ggccaagatccaggacaaagagggcatccctcctgaccagcagagactgatcttcgccggaaagcagctggaagatggcagaa
ccctgagcgactacaacatccagaaagagtctaccctgcacctggtgctgagactgagaggcgtt
SEQ ID NO: 29: nucleic acid sequence of the immunological tag
   agcatcatcaacttcgagaagctgtag
SEQ ID NO: 30: nucleic acid sequence of AAAA linker placed between the segment 6-80 of the mutated human ubiquitin C^{G77V} and hTARP-1 or hTARP-2
   gcagccgccgct
SEQ ID NO: 31: nucleic acid sequence of AAAA linker placed between the immunological tag and hTARP-1 or hTARP-2
   gctgccgctgcc
SEQ ID NO: 32: nucleic acid sequence of the hTARP-1 construction 
atggcccagatcttcgtgaaaaccctgaccggcaagaccatcacactggaagtggaacccagcgacaccatcgagaacgtgaa
ggccaagatccaggacaaagagggcatccctcctgaccagcagagactgatcttcgccggaaagcagctggaagatggcagaa
ccctgagcgactacaacatccagaaagagtctaccctgcacctggtgctgagactgagaggcgttgcagccgccgctcaaatgttt
 
ctgccctcccccctgttttttttcctccagttgcttaagcagtccagccgcagactggagcacacattcgtgttcctggcaaacttctca
ctcatgctgctgcgctacataggcaagaagaggagagccaccagattttgggacccaagacggggaaccccagctgccgctgc
cagcatcatcaacttcgagaagctgtag
SEQ ID NO: 33: nucleic acid sequence of the hTARP-2 construction 
atggcccagatcttcgtgaaaaccctgaccggcaagaccatcacactggaagtggaacccagcgacaccatcgagaacgtgaa
ggccaagatccaggacaaagagggcatccctcctgaccagcagagactgatcttcgccggaaagcagctggaagatggcagaa
ccctgagcgactacaacatccagaaagagtctaccctgcacctggtgctgagactgagaggcgttgcagccgccgctcaaatgttt
ctgccctcccccctgttttttttcctccagttgcttaagcagtccagccgcagactggagcacacattcgtgttcctgaggaacttctca
ctcatggtgctgcgctacataggcaagaagaggagagccaccagattttgggacccaagacggggaaccccagctgccgctgc
cagcatcatcaacttcgagaagctgtag
SEQ ID NO: 34: nucleic acid sequence of the cPPT/CTS sequence 
aattttaaaagaaaaggggggattggggggtacagtgcaggggaaagaatagtagacataatagcaacagacatacaaactaaa
gaattacaaaaacaaattacaaaaattcaaaatttt
SEQ ID NO: 35: nucleic acid sequence of woodchuck hepatitis B virus (WHV) post-transcriptional regulatory element (WPRE) 
ttcccgataatcaacctctggattacaaaatttgtgaaagattgactggtattcttaactatgttgctccttttacgctatgtggatacgctg
ctttaatgcctttgtatcatgctattgcttcccgtatggctttcattttctcctccttgtataaatcctggttgctgtctctttatgaggagttgt
ggcccgttgtcaggcaacgtggcgtggtgtgcactgtgtttgctgacgcaacccccactggttggggcattgccaccacctgtcag
ctcctttccgggactttcgctttccccctccctattgccacggcggaactcatcgccgcctgccttgcccgctgctggacaggggctc
ggctgttgggcactgacaattccgtggtgttgtcggggaagctgacgtcctttccgcggctgctcgcctgtgttgccacctggattct
gcgcgggacgtccttctgctacgtcccttcggccctcaatccagcggaccttccttcccgcggcctgctgccggctctgcggcctct
tccgcgtcttcgccttcgccctcagacgagtcggatctccctttgggccgcctccccgc
SEQ ID NO: 36: Forward primer RRE coding region
   tggaggaggagatatgaggg
SEQ ID NO: 37: Reverse primer RRE coding region
   ctgctgcactataccagaca
SEQ ID NO: 38: Forward primer GAPDH
   tctcctctgacttcaacagc
SEQ ID NO: 39: Reverse primer GAPDH
   ccctgc actttttaagagcc
SEQ ID NO: 40: Forward primer IFNα
   ggatgtgaccttcctcagactc
SEQ ID NO: 41: Reverse primer IFNα
   accttctcctgcgggaatccaa
SEQ ID NO: 42: Forward primer IFNγ
   tcaagtggcatagatgtggaagaa
SEQ ID NO: 43: Reverse primer IFNγ
   tggctctgcaggattttcatg
SEQ ID NO: 44: Forward primer TNFα
   catcttctcaaaattcgagtgacaa
SEQ ID NO: 45: Reverse primer TNFα
   tgggagtagacaaggtacaaccc
SEQ ID NO: 46: Forward primer TGFβ
   tgacgtcactggagttgtacgg
SEQ ID NO: 47: Reverse primer TGFβ
   ggttcatgtcatggatggtgc
SEQ ID NO: 48: Forward primer IL-1β
   tgccaccttttgacagtgatg
SEQ ID NO: 49: Reverse primer IL-1β
   aaggtccacgggaaagacac
SEQ ID NO: 50: Forward primer IL-2
   cctgagcaggatggagaattaca
SEQ ID NO: 51: Reverse primer IL-2
   tccagaacatgccgcagag
SEQ ID NO: 52: Forward primer IL-4
   gtaccaggagccatatccacg
SEQ ID NO: 53: Reverse primer IL-4
   acgagctcactctctgtggt
SEQ ID NO: 54: Forward primer IL-5
   gatgaggcttcctgtccctact
SEQ ID NO: 55: Reverse primer IL-5
   tgacaggttttggaatagcatttcc
SEQ ID NO: 56: Forward primer IL-6
   ctgcaagtgcatcatcgttgttc
SEQ ID NO: 57: Reverse primer IL-6
   taccacttcacaagtcggaggc
SEQ ID NO: 58: Forward primer IL-10
   cagtggagcaggtgaagagt
SEQ ID NO: 59: Reverse primer IL-10
   gtccagcagactcaatacacact
SEQ ID NO: 60: Forward primer IL-12p40
   ggaagcacggcagcagaata
SEQ ID NO: 61: Reverse primer IL-12p40
   aacttgagggagaagtaggaatgg
SEQ ID NO: 62: Forward primer IL-18
   gacagcctgtgttcgaggatatg
SEQ ID NO: 63: Reverse primer IL-18
   tgttcttacaggagagggtagac
SEQ ID NO: 64: Forward primer IL-33
   ctactgcatgagactccgttctg
SEQ ID NO: 65: Reverse primer IL-33
   agaatcccgtggataggcagag
SEQ ID NO: 66: Forward primer CCL2
   aggtccctgtcatgcttctg
SEQ ID NO: 67: Reverse primer CCL2
   tctggacccattccttcttg
SEQ ID NO: 68: Forward primer CCL3
   cctctgtcacctgctcaaca
SEQ ID NO: 69: Reverse primer CCL3
   gatgaattggcgtggaatct
SEQ ID NO: 70: Forward primer CCL4
   tgcaaacctaaccccgagc
SEQ ID NO: 71: Reverse primer CCL4
   tctgtctgcctcttttggtca
SEQ ID NO: 72: Forward primer CCL5
   gtgcccacgtcaaggagtat
SEQ ID NO: 73: Reverse primer CCL5
   gggaagctatacagggtca
SEQ ID NO: 74: Forward primer CCL19
   ctgcctcagattatctgccat
SEQ ID NO: 75: Reverse primer CCL19
   aggtagcggaaggctttcac
SEQ ID NO: 76: Forward primer CCL21
   aaggcagtgatggagggg
SEQ ID NO: 77: Reverse primer CCL21
   cggggtaagaacaggattg
SEQ ID NO: 78: Forward primer CXCL5
   gcgttgtgtttgcttaaccg
SEQ ID NO: 79: Reverse primer CXCL5
   gaacactggccgttctttcc
SEQ ID NO: 80: Forward primer CXCL9
   aaaatttcatcacgcccttg
SEQ ID NO: 81: Reverse primer CXCL9
   tctccagcttggtgaggtct
SEQ ID NO: 82: Forward primer CXCL10
   tgccgtcattttctgcctca
SEQ ID NO: 83: Reverse primer CXCL10
   aggctcgcagggatgatttc

## Claims

1. A lentiviral vector, in particular an integrative or a non-integrative lentiviral vector, comprising a nucleic acid sequence encoding at least one antigen sequence selected from the group consisting of human prostatic acid phosphatase (hPAP), human prostate specific antigen (hPSA), human prostate-specific membrane antigen (hPSMA), human prostate stem cell antigen (hPSCA), human six-transmembrane epithelial antigen of the prostate-1 (hSTEAP1), human trophoblast cell surface antigen 2 (hTROP2), human TCR gamma alternate reading frame protein (hTARP), and fragments thereof.

2. The lentiviral vector according to claim 1, comprising a nucleic acid sequence encoding at least two antigen sequences independently selected from the group consisting of human prostatic acid phosphatase (hPAP), human prostate specific antigen (hPSA), human prostate-specific membrane antigen (hPSMA), human prostate stem cell antigen (hPSCA), human six-transmembrane epithelial antigen of the prostate-1 (hSTEAP1), human trophoblast cell surface antigen 2 (hTROP2), human TCR gamma alternate reading frame protein (hTARP), and fragments thereof.

3. The lentiviral vector according to claim 1 or 2, wherein the at least one or at least two antigen sequence(s) comprise:
(i) human prostatic acid phosphatase (hPAP) or at least a fragment thereof; and/or
(ii) human prostate specific antigen (hPSA) or at least a fragment thereof; and/or
(iii) human trophoblast cell surface antigen 2 (hTROP2) or at least a fragment thereof; and/or
(iv) human TCR gamma alternate reading frame protein (hTARP) or at least a fragment thereof.

4. The lentiviral vector according to any one of the preceding claims, wherein the at least one or at least two antigen sequence(s) comprise:
- human prostatic acid phosphatase (hPAP) or at least a fragment thereof, and human prostate specific antigen (hPSA) or at least a fragment thereof; or
- human trophoblast cell surface antigen 2 (hTROP2) or at least a fragment thereof.

5. The lentiviral vector according to any one of the preceding claims, wherein the nucleic acid sequence encodes at least:
- an amino acid sequence having at least 60% sequence identity, in particular at least 80% sequence identity, more particularly at least 90% sequence identity, with the sequence set forth as SEQ ID NO: 1; and/or
- an amino acid sequence having at least 60% sequence identity, in particular at least 80% sequence identity, more particularly at least 90% sequence identity, with the sequence set forth as SEQ ID NO: 2.

6. The lentiviral vector according to any one of the preceding claims, wherein the nucleic acid sequence encodes at least an amino acid sequence having at least 60% sequence identity, in particular at least 80% sequence identity, more particularly at least 90% sequence identity, with the sequence set forth as SEQ ID NO: 1 and an amino acid sequence having at least 60% sequence identity, in particular at least 80% sequence identity, more particularly at least 90% sequence identity, with the sequence set forth as SEQ ID NO: 2;
in particular wherein the amino acid sequence having at least 60% sequence identity, in particular at least 80% sequence identity, more particularly at least 90% sequence identity, with the sequence set forth as SEQ ID NO: 1 is fused, directly or by the intermediate of a linker sequence, at the N-terminal end of the amino acid sequence having at least 60% sequence identity, in particular at least 80% sequence identity, more particularly at least 90% sequence identity, with the sequence set forth as SEQ ID NO: 2.

7. The lentiviral vector according to any one of the preceding claims, wherein the nucleic acid sequence encodes at least an amino acid sequence having 60% sequence identity, in particular at least 80% sequence identity, more particularly at least 90% sequence identity, with the sequence set forth as SEQ ID NO: 3.

8. The lentiviral vector according to any one of claims 1 to 4, wherein the human trophoblast cell surface antigen 2 (hTROP2) antigen sequence or at least a fragment thereof is selected from the group consisting of:
(a) an amino acid sequence having at least 60% sequence identity, in particular at least 80% sequence identity, more particularly at least 90% sequence identity, with the sequence set forth as SEQ ID NO: 4; and/or
(b) an amino acid sequence having at least 60% sequence identity, in particular at least 80% sequence identity, more particularly at least 90% sequence identity, with the sequence set forth as SEQ ID NO: 5; and/or
(c) an amino acid sequence having at least 60% sequence identity, in particular at least 80% sequence identity, more particularly at least 90% sequence identity, with the sequence set forth as SEQ ID NO: 6; and/or
(d) an amino acid sequence having at least 60% sequence identity, in particular at least 80% sequence identity, more particularly at least 90% sequence identity, with the sequence set forth as SEQ ID NO: 7; and/or
(e) an amino acid sequence having at least 60% sequence identity, in particular at least 80% sequence identity, more particularly at least 90% sequence identity, with the sequence set forth as SEQ ID NO: 8.

9. The lentiviral vector according to any one of claims 1 to 3, wherein the human TCR gamma alternate reading frame protein (hTARP) antigen sequence or at least a fragment thereof is selected from the group consisting of:
- an amino acid sequence having at least 60% sequence identity, in particular at least 80% sequence identity, more particularly at least 90% sequence identity, with the sequence set forth as SEQ ID NO: 9; and/or
- an amino acid sequence having at least 60% sequence identity, in particular at least 80% sequence identity, more particularly at least 90% sequence identity, with the sequence set forth as SEQ ID NO: 10.

10. The lentiviral vector according to any one of the preceding claims, wherein the lentiviral vector comprises:
(i) a MHC Class I promoter, and in particular a human β2-microglobulin promoter; and/or
(ii) a cPPT/CTS sequence, in particular the cPPT/CTS sequence set forth as sequence SEQID NO: 33; and/or
(iii) a 3' long terminal repeat (LTR) which is devoid of its U3 promoter sequence; and/or
(iv) a mutant form of the woodchuck hepatitis B virus (WHV) post-transcriptional regulatory element (WPRE), and in particular having the sequence set forth as sequence SEQ ID NO: 35.

11. A lentiviral vector particle comprising at least a lentiviral vector as defined in any one of claims 1 to 10.

12. The lentiviral vector particle according to claim 11, further comprising:
- a vesicular stomatitis virus glycoprotein (VSV-G), in particular a VSV-G Indiana serotype or a VSV-G New Jersey serotype; and/or
- HIV-1 subtype D Gag and Pol proteins.

13. A pharmaceutical composition, in particular a vaccine composition, comprising, in a physiologically acceptable medium, at least one lentiviral vector according to any one of claims 1 to 10 or a lentiviral vector particle according to any one of claims 11 to 12.

14. A combinatory treatment comprising:
(i) at least one lentiviral vector according to any one of claims 1 to 10, or lentiviral vector particle according to any one of claims 11 to 12, or pharmaceutical composition according to claim 13; and
(ii) at least one anticancer agent or treatment and/or at least one immunotherapy agent or treatment.

15. The combinatory treatment according to claim 14, wherein the at least one anticancer agent or treatment is selected from the group consisting of DNA methyltransferase inhibitors; histone deacetylase inhibitors; and adjuvants, in particular Toll-Like Receptor (TLR) agonists, poly I:C (polyinosinic:polycytidylic acid), the stabilized form of poly I:C (polyICLC), CpG oligodeoxynucleotide, and cGAMP (Cyclic GMP-AMP); and mixtures thereof; and/or
wherein the at least one immunotherapy agent or treatment is selected from the group consisting of immune checkpoint inhibitors, in particular anti-PD-1, anti-PD-L1 (PD-1 Ligand), anti-CTLA-4 (Cytotoxic T-Lymphocyte-Associated protein 4), anti-TIM-3 (T-cell immunoglobulin and mucin-domain containing-3), anti-LAG3 (Lymphocyte-activation gene 3), anti-TIGIT (T cell immunoreceptor with Ig and ITIM domains), and anti-NKG2A (Natural killer group 2 member ) antibodies; and mixtures thereof.

16. A lentiviral vector according to any one of claims 1 to 10, or a lentiviral vector particle according to any one of claims 11 to 12, or a pharmaceutical composition according to claim 13, or a combinatory treatment according to any one of claims 14 to 15, for use in the treatment and/or prevention of prostate cancers.
